(19) Europäisches Patentamt
European Patent Office
Office européen des brevets

(11) EP 1 506 010 B1

(12) **EUROPEAN PATENT SPECIFICATION**

(45) Date of publication and mention of the grant of the patent:
**22.10.2008 Bulletin 2008/43**

(21) Application number: **03722133.0**

(22) Date of filing: **12.05.2003**

(51) Int Cl.:
***A61K 39/39*** *(2006.01)* ***A61P 37/00*** *(2006.01)*
***A61P 35/00*** *(2006.01)* ***A61P 31/00*** *(2006.01)*

(86) International application number:
**PCT/CA2003/000678**

(87) International publication number:
**WO 2003/094963 (20.11.2003 Gazette 2003/47)**

(54) **METHYLATED IMMUNOSTIMULATORY OLIGONUCLEOTIDES AND METHODS OF USING THE SAME**

METHYLIERTE IMMUNSTIMULIERENDE OLIGODEOXYNUKLEOTIDE UND VERFAHREN ZU DEREN VERWENDUNG

OLIGONUCLEOTIDES METHYLES IMMUNOSTIMULATEURS ET METHODES D'UTILISATION DE CES DERNIERS

(84) Designated Contracting States:
**AT BE BG CH CY CZ DE DK EE ES FI FR GB GR HU IE IT LI LU MC NL PT RO SE SI SK TR**

(30) Priority: **10.05.2002 US 379343 P**
**07.11.2002 US 290545**
**04.04.2003 US 460646 P**

(43) Date of publication of application:
**16.02.2005 Bulletin 2005/07**

(73) Proprietor: **Tekmira Pharmaceuticals Corporation**
**Burnaby BC V5J 5J8 (CA)**

(72) Inventors:
- **TAM, Ying, K.**
  **Vancouver, British Columbia V6S 1C3 (CA)**
- **SEMPLE, Sean**
  **Vancouver, British Columbia V6G 3E3 (CA)**
- **KLIMUK, Sandra**
  **Vancouver, British Columbia V6K 4K7 (CA)**
- **CHIKH, Ghania**
  **Vancouver, British Columbia, V6G 1M9 (CA)**

(74) Representative: **Lee, Nicholas John et al**
**Kilburn & Strode**
**20 Red Lion Street**
**London WC1R 4PJ (GB)**

(56) References cited:
**WO-A-01/15726** **WO-A-96/02555**

- **MCCLUSKIE M J ET AL: "The potential of oligodeoxynucleotides as mucosal and parenteral adjuvants" VACCINE, BUTTERWORTH SCIENTIFIC. GUILDFORD, GB, vol. 19, no. 17-19, 21 March 2001 (2001-03-21), pages 2657-2660, XP004231093 ISSN: 0264-410X**
- **LEONETTI CARLO ET AL: "Encapsulation of c-myc antisense oligodeoxynucleotides in lipid particles improves antitumoral efficacy in vivo in a human melanoma line" CANCER GENE THERAPY, vol. 8, no. 6, June 2001 (2001-06), pages 459-468, XP002260435 & ISSN: 0929-1903**
- **LOISEL ET AL: "Contribution of plasmid DNA to hepatotoxicity after systemic administration of lipoplexes." HUMAN GENE THERAPY UNITED STATES, vol. 12, no. 6, 9 April 2001 (2001-04-09), pages 685-696, UNITED STATES ISSN: 1043-0342**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

ANNEXE

SEQUENCE LISTING

<110> Tam, Ying K.
Semple, Sean
Klimuk, Sandra
Chikh, Ghania

<120> METHYLATED IMMUNOSTIMULATORY OLIGONUCLEOTIDES AND METHODS OF USING THE SAME

<130> 9220-PCT

<140> PCT/CA03/00678
<141> 2003-12-05

<150> 60/379,343
<151> 2002-05-10

<150> 60/460,646
<151> 2003-04-04

<150> 10/290,545
<151> 2002-11-07

<150> 10/437,275
<151> 2003-05-12

<160> 32

<170> PatentIn version 3.2

<210> 1
<211> 20
<212> DNA
<213> Mus sp.

<400> 1
tccatgacgt tcctgacgtt 20

<210> 2
<211> 16
<212> DNA
<213> Homo sapiens

<400> 2
taacgttgag gggcat 16

<210> 3
<211> 16
<212> DNA
<213> Homo sapiens

<400> 3
taagcatacg gggtgt 16

<210> 4
<211> 16
<212> DNA
<213> Homo sapiens

<220>
<221> modified_base
<222> (4)..(4)
<223> methylated cytosine residue

<400> 4
taacgttgag gggcat 16

<210> 5
<211> 6
<212> DNA
<213> Homo sapiens

<400> 5
aacgtt 6

<210> 6
<211> 24
<212> DNA
<213> Homo sapiens

<400> 6
gatgctgtgt cggggtctcc gggc 24

<210> 7
<211> 24
<212> DNA
<213> Homo sapiens

<400> 7
tcgtcgtttt gtcgttttgt cgtt 24

<210> 8
<211> 20

<212> DNA
<213> Homo sapiens

<400> 8
tccaggactt ctctcaggtt 20

<210> 9
<211> 18
<212> DNA
<213> Homo sapiens

<400> 9
tctcccagcg tgcgccat 18

<210> 10
<211> 20
<212> DNA
<213> Mus sp.

<400> 10
tgcatccccc aggccaccat 20

<210> 11
<211> 20
<212> DNA
<213> Homo sapiens

<400> 11
gcccaagctg gcatccgtca 20

<210> 12
<211> 20
<212> DNA
<213> Homo sapiens

<400> 12
gcccaagctg gcatccgtca 20

<210> 13
<211> 15
<212> DNA
<213> Homo sapiens

<400> 13
ggtgctcact gcggc 15

<210> 14
<211> 16
<212> DNA
<213> Homo sapiens

<400> 14
aaccgttgag gggcat 16

<210> 15
<211> 24
<212> DNA
<213> Homo sapiens

<400> 15
tatgctgtgc cggggtcttc gggc 24

<210> 16
<211> 18
<212> DNA
<213> Homo sapiens

<400> 16
gtgccggggt cttcgggc 18

<210> 17
<211> 18
<212> DNA
<213> Homo sapiens

<400> 17
ggaccctcct ccggagcc 18

<210> 18
<211> 18
<212> DNA
<213> Homo sapiens

<400> 18
tcctccggag ccagactt 18

<210> 19
<211> 15
<212> DNA
<213> Homo sapiens

<400> 19
aacgttgagg ggcat 15

<210> 20
<211> 15
<212> DNA
<213> Homo sapiens

<400> 20
ccgtggtcat gctcc 15

<210> 21
<211> 21
<212> DNA
<213> Homo sapiens

<400> 21
cagcctggct caccgccttg g 21

<210> 22
<211> 20
<212> DNA
<213> Mus sp.

<400> 22
cagccatggt tccccccaac 20

<210> 23
<211> 20
<212> DNA
<213> Homo sapiens

<400> 23
gttctcgctg gtgagtttca 20

<210> 24
<211> 18
<212> DNA
<213> Homo sapiens

<400> 24
tctcccagcg tgcgccat 18

<210> 25
<211> 15
<212> DNA
<213> Homo sapiens

<400> 25
gtgctccatt gatgc 15

<210> 26
<211> 33
<212> RNA
<213> Homo sapiens

<400> 26
gaguucugau gaggccgaaa ggccgaaagu cug 33

<210> 27
<211> 6
<212> DNA
<213> Artificial

<220>
<223> synthetic

<400> 27
rrcgyy 6

<210> 28
<211> 15
<212> DNA
<213> Homo sapiens

<400> 28
aacgttgagg ggcat 15

<210> 29
<211> 16
<212> DNA
<213> Homo sapiens

<400> 29
caacgttatg gggaga 16

<210> 30
<211> 16
<212> DNA
<213> Homo sapiens

<400> 30
taacgttgag gggcat 16

<210> 31
<211> 20
<212> DNA
<213> Homo sapiens

<220>
<221> modified_base
<222> (8)..(17)
<223> methylated cytosine residue at positions 8 and 17

<400> 31
tccatgacgt tcctgacgtt 20

<210> 32
<211> 24
<212> DNA
<213> Homo sapiens

<220>
<221> modified_base
<222> (2)..(21)
<223> methylated cytosine residue at positions 2, 5, 13, and 21

<400> 32
tcgtcgtttt gtcgttttgt cgtt 24

## Description

TECHNICAL FIELD

The invention relates to lipid-nucleic acid formulations useful for stimulating an immune response *in vivo,* and in particular, to adjuvants and vaccines comprising liposomal formulations of nucleic acid sequences comprising at least one methylated cytosine so as to synergistically enhance their immunostimulatory activity, wherein the methylated cytosine forms part of a CpG motif.

BACKGROUND OF THE INVENTION

Methylation of cytosine is the only known endogenous modification of DNA in eukaryotes, and occurs by the enzymatic addition of a methyl or hydroxymethyl group to the carbon-4 or carbon-5 position of cytosine. Costello and Plass, 2001, J. Med. Genet. 38: 285. The lower frequency of methylated cytosine residues found in bacterial DNA was suggested by many investigators as the reason why bacterial DNA could elicit an immune response *in vitro* and *in vivo,* in contrast to vertebrate DNA having a higher frequency of methylated cytosine residues which failed to stimulate any response. Messina et al, 1991, J. Immunol 147: 1759. Later studies using a peripheral blood mononuclear cell assay (PBMC) to measure mitogenicity of oligonucleotides *in vitro* showed that unmethylated DNA could be mitogenic but not methylated DNA. Of the many oligonucleotide sequences tested, those bearing a CpG dinucleotide motif were shown to be particularly mitogenic. Krieg et al, 1995, Nature 374: 546-9. Oligonucleotides bearing a CpG dinucleotide motif were also shown to be immunostimulatory *in vivo,* provided again, that the cytosine of the dinucleotide was not methylated. Parronchi P. et al 1999, J. Immunol 163: 5946-53; Kreig A.M, 1999, Biochim Biophys Acta 1489: 107-16. Similarly, WO01/15726 describes lipid-nucleic acid particles and their use in enhancing the immune response in mammals and WO96/02555 describes oligonucleotides containing unmethylated CpG dinucleotides and therapeutic uses based on their ability to stimulate an immune response in a subject.

Similarly, U.S. Patent No 6,194,388 disclosed that B cell mitogenicity in T-cell depleted spleen cells was abolished when cytosines of the CpG motif were methylated but not when other cytosines were methylated, measuring mitogenicity based on *in vitro* thymidine incorporation into PBMC. Subsequently, based on screening over 300 oligonucleotide sequences for their ability to induce B cell activation in T-cell depleted spleen cells measured by uridine uptake *in vitro,* related U.S. Patent No. 6,207,646 disclosed that oligonucleotides having unmethylated CpG dinucleotide motifs were more effective in stimulating mitogenicity than oligonucleotides lacking the CpG motif and that methylated counterparts of the same CpG oligonucleotides were not as effective. The site of methylation that most negatively impacted B-cell mitogenicity was again shown to be the cytosine of the CpG dinucleotide, which when methylated reduced *in vitro* stimulation in comparison to the methylation of other cytosines.

Similar conclusions were reached by the same group of applicants in U.S. Patent No. 6,239,116 (reduced NK cell lytic activity in methylated sequences), U.S. Patent No. 6,406,705 (non-CpG oligonucleotides lack adjuvant affect when combined with HBsAg), and U.S. Patent No. 6,429,199 (methylation of CpG motif caused a loss of stimulatory effect in combinations with GM-CSF). In general, therefore, methylated CpG oligonucleotides have been consistently shown to be either non-effective or much less effective than methylated oligonucleotides in stimulating mitogenic effects *in vitro* or in eliciting immunostimulatory effects *in vivo.*

Recently, Schetter *et al.* in International Publication No. WO 02/069369 suggested that certain types of methylated CpG oligonucleotides may possess immunostimulatory activity based on an *in vitro* PBMC assay measuring production of certain leukocyte surface markers, including CD86, CD80, CD25 and CD69. The nucleic acid sequences tested, however, were heavily methylated at 4-9 sites in oligonucleotides having 1-4 CpG dinucleotides, and in all exemplified cases the methylated oligonucleotides remained less effective than their unmethylated counterparts. A variety of additional oligonucleotide structures were also investigated, including inosine substituted for guanosine, a cytosine adjacent to an inosine, a "dSpacer" having a sugar devoid of base substituted for a base adjacent to an inosine, a ZpG dinucleotide where Z is replaced by 2-deoxyuridine, 5-fluoro-2'deoxy uridine, and a CpY dinucleotide where Y is a 2-aminopurine, xanthosine, N7-methyl-xanthosine, nebularine or a dspacer. Unfortunately, no useful control sequence, such as a randomized sequence or mixture of sequences of the same length, was provided to demonstrate the predictive value of the *in vitro* assays and/or the accuracy of the applicants' conclusions with respect to the proposed immunostimulatory activity that might occur *in vivo.* Moreover, no immunostimulatory activity was seen with dendritic cells, a key subset of antigen-presenting cells.

There remains a continuing need in the art for improved adjuvant compositions having enhanced immunostimulatory activity. The compositions must be capable of stimulating a wider range of antigen-presenting cells, including in particular dendritic cells. Further, the compositions must be capable, both alone and in combination with tumor, pathogen or other antigens, to stimulate effective immune responses *in vivo.*

There is also an interest in increasing the breadth of nucleic acid sequence motifs that can be used as immunostimulatory adjuvants. What is needed, in particular, are compositions and methods capable of eliciting consistent immunostimulatory activity from CpG dinucleotide sequences having methylated cytosines, as well as other oligonucleotide structures demonstrating significant variability in their immunostimulatory activity *in vitro.*

## SUMMARY OF THE INVENTION

In a first aspect, the present invention provides an adjuvant comprising a lipid-nucleic acid (LNA) formulation, wherein said LNA formulation comprises a lipid component comprising at least one cationic lipid and a nucleic acid component comprising at least one methylated oligonucleotide, wherein said at least one methylated oligonucleotide comprises at least one CpG dinucleotide having a methylated cytosine, wherein said adjuvant is capable of stimulating dendritic cells *in vivo* in response to antigenic stimulation. In one embodiment of the first aspect of the invention said adjuvant is capable of stimulating dendritic cell expansion *in vivo,* characterized by an increase in the number of antigen-presenting cells expressing at least one of a CD11c and a DEC205 marker. In a second embodiment said adjuvant is capable of stimulating dendritic cell activation *in vivo,* characterized by an increase in the number of antigen-presenting cells co-expressing at least one of a CD11c and a DEC205 marker in conjunction with a CD86 marker. In a further embodiment said at least one methylated oligonucleotide comprises two CpG dinucleotides, and wherein at least one of the cytosines in said CpG dinucleotides is methylated. In a still further embodiment said oligonucleotide comprises a modified phosphate backbone.

In a second aspect, the present invention provides a vaccine comprising an adjuvant according to the present invention in combination with at least one target antigen, wherein said at least one target antigen is mixed with or associated with said LNA formulation, and wherein said vaccine is capable of stimulating dendritic cells *in vivo* in response to presentation of said at least one target antigen by said formulation to antigen-presenting cells. In one embodiment said at least one target antigen comprises a plurality of epitopes from the same antigen. In an alternative embodiment said at least one target antigen comprises a plurality of epitopes from different antigens.

In a third aspect, the present invention provides the use of an adjuvant according to the first aspect as a vaccine according to the second aspect in the manufacture of a medicament for stimulating an enhanced host immune response to antigenic stimulation.

In a forth aspect, the present invention provides an *in vitro* method for stimulating dendritic cells, comprising contacting at least one dendritic cell with a lipid-methylated nucleic acid formulation for stimulating an immune response in an animal, said formulation comprising a lipid component and a nucleic acid component comprising a methylated nucleic acid sequence, wherein said methylated nucleic acid sequence comprises at least one CpG dinucleotide having a methylated cytosine, and wherein said lipid-methylated nucleic acid formulation is capable of stimulating dendritic cells *in vivo* in response to antigenic stimulation.

In a fifth aspect, the present invention provides the use of a lipid-methylated nucleic acid formulation for stimulating an immune response in an animal, said formulation comprising a lipid component and a nucleic acid component comprising a methylated nucleic acid sequence, wherein said methylated nucleic acid sequence comprises at least one CpG dinucleotide having a methylated cytosine, and wherein said lipid-methylated nucleic acid formulation is capable of stimulating dendritic cells *in vivo* in response to antigenic stimulation, in the manufacture of a medicament for stimulating host dendritic cells.

In a sixth aspect, the present invention provides the use of a lipid-nucleic (LNA) formulation associated with a target antigen in the manufacture of a medicament for simultaneously delivering antigenic and adjuvant immune stimulation to antigen presenting cells, said LNA formulation comprising a lipid component comprising at least one cationic lipid and a nucleic acid component comprising at least one methylated oligonucleotide comprising at least one CpG dinucleotide having a methylated cytosine, wherein said LNA formulation is capable of stimulating dendritic cells *in vivo.*

In a seventh aspect, the present invention provides products containing an adjuvant of the present invention and at least one target antigen mixed or associated with said LNA formulation for simultaneous, sequential or separate use in stimulating an enhanced host immune response to antigen stimulation.. Further preferred features of each aspect are set out in the claims and are further described in the specification.

The present inventors have discovered that the incorporation of methylated nucleic acid sequences into the lipid-nucleic acid (LNA) formulations described herein solves the aforementioned problems in the prior art and provides synergistic benefits. In particular, when used in accordance with the present invention the immunostimulatory activity of methylated nucleic acid sequences can be significantly enhanced and improved immune stimulation consistently achieved *in vivo,* in marked contrast to the widely variable results reported in the prior art. Surprisingly, these lipid-methylated nucleic acid formulations also demonstrate therapeutic efficacy that is as good as, and in many cases better than, similar lipid-nucleic acid formulations employing the corresponding unmethylated sequences. Further, immunostimulatory activity is obtained in a broader class of antigen-presenting cells, and in particular in dendritic cells. Moreover, as demonstrated herein, and unlike the prior art, effective immune stimulation can be achieved with encapsulated nucleic acids having only a single methylated CpG dinucleotide.

Such lipid-methylated nucleic acid formulations for stimulating an immune response in an animal, may comprise a lipid component and a nucleic acid component comprising at least one methylated nucleic acid sequence. In certain preferred embodiments, the methylated nucleic acid sequence comprises a methyl or hydroxymethyl group attached to the carbon-4 position (4-mC) or carbon-5 position (5-mC) of at least one cytosine, wherein the methylated cytosine residue will generally be part of a CpG dinucleotide motif located in said sequence. In alternative preferred embodiments, the methylated nucleic acid is fully encapsulated by the lipid component to form a liposomal particle, as further described herein.

In certain embodiments, the methylated nucleic acid sequence lacks immunostimulatory activity *in vivo* when administered to an animal as a free nucleic acid. The lipid-methylated nucleic acid formulation may be either equivalent to or more immunostimulatory *in vivo* than a corresponding lipid-nucleic acid formulation having the same sequence but lacking methylation of one or more cytosine residues.

The nucleic acid sequence comprises at least one CpG dinucleotide having a methylated cytosine. In a preferred embodiment, the nucleic acid sequence comprises a single CpG dinucleotide, wherein the cytosine in said CpG dinucleotide is methylated. In a specific embodiment, the nucleic acid sequence comprises the sequence 5' TAACGTTGAGGGGCAT 3' (ODN1m). In an alternative embodiment, the nucleic acid sequence comprises at least two CpG dinucleotides, wherein at least one cytosine in the CpG dinucleotides is methylated. In a further embodiment, each cytosine in the CpG dinucleotides present in the sequence is methylated. In another specific embodiment, the nucleic acid sequence comprises the sequence 5'TTCCATGACGTTCCTGACGTT 3' (ODN2m). In another embodiment, the nucleic acid sequence comprises a plurality of CpG dinucleotides, wherein at least one of said CpG dinucleotides comprises a methylated cytosine. Significantly however, and unlike the prior art teachings, effective immune stimulation may be obtained as described herein utilizing nucleic acid sequences having only a single CpG dinucleotide with a methylated cytosine, or a plurality of CpG dinucleotides wherein only one or a couple of the cytosines of said CpG dinucleotides are methylated.

The lipid-methylated nucleic acid formulation may be capable of activating and/or expanding dendritic cells when administered to an animal *in vivo.* Dendritic cells bearing at least one of a CD11c and DEC205 marker may be expanded *in vivo* upon administration of the above formulations, preferably in conjunction with antigenic stimulation. Alternatively, dendritic cells bearing the CD11c or DEC205 marker may be activated *in vivo* to express a CD86 marker after administration of the above formulations, again preferably in conjunction with antigenic stimulation.

The lipid-nucleic acid formulation may further comprise a pharmaceutically acceptable carrier, buffer or diluent.

In certain embodiments, the nucleic acid is comprised of a phosphodiester backbone. In other embodiments, the nucleic acid is comprised of a non-phosphodiester backbone. In more particular embodiments, the non-phosphodiester backbone is a phosphorothioate backbone.

In various embodiments of the composition the liposomal particle comprises a cationic lipid. Example cationic lipids are selected from a group of cationic lipids consisting of DDAB, DODAC, DOTAP, DMRIE, DOSPA, DMDMA, DC-Chol, DODMA, DODAP and mixtures thereof. In further embodiments, the liposomal particle further comprises a neutral lipid selected from the group consisting of DOPE, DSPC, POPC, diacylphosphatidylcholine, diacylphosphatidylethanolamine, ceramide, sphingomyelin, cephalin, cerebrosides, and mixtures thereof. In other embodiments, the liposomal particle alone comprises a neutral lipid selected from the group consisting of DOPE, DSPC, POPC,diacylphosphatidylcholine, diacylphosphatidylethanolamine, ceramide, sphingomyelin, cephalin, cerebrosides and mixtures thereof. In other embodiments, the lipid particle comprises a lipid selected from the group consisting of but not limited to DODAP, DODMA, DSPC, POPC, and mixtures of thereof. In certain other embodiments, the lipid particle is comprised of a mixture of sphingomyelin and a lipid selected from the group consisting of DODAP, DODMA, DSPC, POPC, and mixtures of thereof. In still other embodiments, the lipid component comprises DSPC, DODMA, Chol, and PEG-DMG and the ratio of said DSPC to said DODMA to said Chol to said PEG-DMG is about 20: 25: 45: 10 mol/mol. In a further more specific embodiment, the ratio of said lipid component to said nucleic component is about 0.01-0. 25 wt/wt.

In other embodiments the lipid particle further includes a steric barrier lipid component on the surface of the lipid particle. In certain embodiments, the steric barrier lipid component is selected from the group consisting of PEG-DMG, PEG-PE, and a PEG ceramide. In one embodiment, the PEG ceramide is PEG-ceramide C-14. In another embodiment the PEG ceramide is PEG-ceramide C-20.

A composition may comprise an antigen of interest in combination with the aforementioned lipid-methylated nucleic acid formulations. The antigen may be either mixed with or associated with the lipid-methylated nucleic acid formulation. Preferably, the antigen is associated with the formulation, as described herein. The antigen may be a tumor antigen. In preferred embodiments, the methylated nucleic acid sequence comprises a 4-mC or 5-mC located within at least one CpG dinucleotide motif. In particularly preferred embodiments, the methylated nucleic acid sequence is encapsulated in aliposomal particle. In further embodiments, the antigen is also encapsulated in the liposomal particle.

Methods of stimulating enhanced immune activity in an animal comprise administering any of the foregoing compositions to the animal in order to induce an improved immune response. The LNA formulations may be used directly as adjuvants, or may advantageously be combined with one or more target antigens in vaccine formulations. Preferably, administration of the subject compositions is capable of stimulating one or more dendritic cells present in the animal's

immune system. The target antigen may be administered in association with the lipid-nucleic acid formulations described herein, and more preferably with a liposomal particle. The antigen may be encapsulated in the liposomal particle. The antigen may comprise one or more epitopes from one or more tumor antigens or microbial antigens.

In a further aspect, *in vitro* methods for stimulating antigen-presenting cells are provided, comprising the step of contacting at least one antigen-presenting cell *in vitro,* with an immunostimulatory composition as described herein. In preferred embodiments, the antigen-presenting cell comprises a dendritic cell.

Methods for expanding dendritic cells *in vivo* are described, comprising administering to a host the subject lipid-nucleic acid formulations comprising a nucleic acid sequence having at least one 4mC or 5mC located within a CpG dinucleotide, wherein said administration is effective to expand dendritic cells in said host. Preferably, expansion of said dendritic cells is characterized by an increase in the number of host antigen- presenting cells expressing at least one of a CD11c and DEC205 marker.

Methods for activating dendritic cells *in vivo* are described, comprising administering to a host the subject lipid-nucleic acid formulations comprising a nucleic acid sequence having at least one 4mC or 5mC located within a CpG dinucleotide, wherein said administration is effective to expand dendritic cells in said host in response to antigenic stimulation. Preferably, activation of said dendritic cells is characterized by an increase in the number of host antigen-presenting cells co-expressing at least one of aCD11c or DEC205 marker in conjunction with a CD86 marker. Antigenic stimulation may be achieved by administration of the subject formulations in combination with one or more target antigens of interest, either mixed with or associated with the lipid- methylated nucleic acid formulation.

## BRIEF DESCRIPTION OF THE DRAWINGS

Figure 1 illustrates in vitro stimulation of leukocytes bearing the activation marker CD69 results from treating whole blood with free oligonucleotides. Mouse whole blood was treated in vitro with either the free oligonucleotide herein designated ODN1 or with the oligonucleotide designated ODN2.

Figure 2 illustrates in vivo treatment of mice by injection with encapsulated or free ODN1 and ODN2 oligonucleotides produces results that are contrary to those obtained in vitro.

Figure 3 shows that when encapsulated in a lipid vesicle the methylatedODN1 m was more active than the unmethylated counterpart ODN1 in stimulating activation of dendritic cells *in vivo.*

Figure 4A shows that both the methylated ODN1m and the unmethylated ODN1 stimulated the expansion of CD11c positive cells in spleen and whole blood.

Figure 4B shows that both ODN1 and ODN1m stimulate the expansion of DEC205 positive cells in spleen, whole blood and lymph node.

Figure 5 shows that the methylated ODN1m was more active than the unmethylated counterpart ODN1, in stimulating CD86 expression when either ODN was lipid encapsulated.

Figure 6 shows that *in vivo* administration of free oligonucleotide had no affect on stimulation of IL-6, IL-12 IFN-gamma or MCP-1. In contrast, *in vivo* administration of lipid encapsulated oligonucleotides stimulated production of each of these cytokines.

Figure 7A illustrates increased IL-12 induction by treatment of mice with either encapsulated PO or PS oligonucleotide ODN1 in comparison to free oligonucleotide ODN1 measured over an oligonucleotide dosage scale. Figure 7B shows that treatment with encapsulated PO oligonucleotides stimulates a strong early induction of IFN-gamma while treatment with encapsulated PS oligonucleotides stimulates a smaller but still effective induction of IFN-gamma.

Figure 8 shows a comparison of IgM titres indicative of a Th-1 response upon administration of free PS or PO oligonucleotides.

Figure 9 shows a comparison of IgG production indicative of a Th-2 response upon administration of free PS or PO oligonucleotide, including methylated oligonucleotides

Figure 10 shows that over a series of screenings of animals treated with methylated or unmethylated lipid encapsulated oligonucleotides, the methylated oligonucleotides are about the same or better than the unmethylated oligonucleotide in stimulating proliferation of dendritic cells, NK cells and CD8+ T-cells .

Figures 11A and B show that over a series of screenings of animals treated with methylated or unmethylated lipid encapsulated oligonucleotides, the methylated oligonucleotides are better than the unmethylated oligonucleotide in stimulating proliferation of cytotoxic T lymphocytes and Ag-specific lymphocytes.

Figure 11C illustrates data from a representative tetramer study that was included in the overall screenings described in Figures 11A and 11B.

Figure 12 illustrates that when administered to an animal as free oligonucleotides, methylated versions have less therapeutic efficacy than methylated nucleotides in reducing tumor growth.

Figure 13 illustrates that encapsulation of oligonucleotides provides improved efficacy of methylated and unmethylated oligonucleotides over free ODN, particularly when the oligonucleotides contain a natural phosphorothioate (PS) backbone.

Figure 14 shows that encapsulation of oligonucleotides provides improved efficacy of methylated and unmethylated oligonucleotides over free ODN, when the oligonucleotides contain a phosphodiester (PO) backbone.

Figure 15 shows that lipid encapsulated PS oligonucleotides ODN2and ODN2m each exhibit therapeutic efficacy.

Figure 16 illustrates an adjuvant effect and therapeutic efficacy of administering the methylated ODN1m to an animal inoculated with a B16 melanoma tumor. Encapsulation of the ODN1m oligonucleotide in a lipid particle increased its efficacy in reducing tumor volume.

Figure 17 shows that for a series of mice inoculated with the B16 melanoma and subsequently treated by administration of a 20mg/kg dose of oligonucleotide, the average tumor size of tumours in mice treated with encapsulated free oligonucleotides ODN1 and ODN1m.

Figure 18 shows the reduction in tumor volume when mice were treated with encapsulated methylated ODN1m and the unmethylated counterpart ODN1.

Figure 19 shows survival rates of mice treated with the encapsulated methylated ODN1 m in comparison to treatment with the unmethylated ODN1 in two different studies.

Figure 20 illustrates the efficacy in terms of tumor volume when methylated ODN1m and the unmethylated counterpart ODN1are encapsulated in a lipid particle.

Figure 21 shows the survival rate of mice treated with encapsulated methylated ODN1m relative to treatment with the unmethylated encapsulated ODN1.

Figure 22 illustrates that encapsulated PS oligonucleotides ODN1 and ODN2produced an IFN-gamma peak that is not produced by encapsulated PO oligonucleotides 6 days after treatment.

Figure 23 shows the effect on blood clearance in mice methylated or unmethylated oligonucleotides encapsulated in lipid particles having different PEG-ceramide steric coatings.

Figure 24 illustrates therapeutic efficacy of liposomal particles encapsulating methylated or methylated CpG oligonucleotide in treating a tumor by administering the composition to an animal having the tumor.

Figure 25 illustrates that lipid encapsulation of methylatedPS-ODN5m provided a more effective therapeutic benefit than encapsulation of the equivalent unmethylated PS-ODN5 at reducing tumor growth over time.

Figure 26 shows the survival rate of the mice treated with free and encapsulated methylatedODN5m relative to treatment with the unmethylated encapsulated and free ODN5.

Figure 27 illustrates efficacy in terms of tumor volume when treated with free unmethylated and methylated PS and PO ODN 7 and free and encapsulated PO-ODN7m.

Figure 28 shows survival rates of mice treated with the free unmethylated and methylated PS and PO ODN7 in comparison to treatment with the encapsulated PO ODN7m.

Figure 29 shows the CTL response to a B16 cell target after immunization with a multiple epitope cancer vaccine using encapsulated ODN1 m.

Figure 30 shows the CTL response to a B16 cell target after immunization with a multiple epitope cancer vaccine using peptide-pulsed dendritic cells.

Figure 31 shows the CTL response to a B16 cell target after immunization with tumor cell lysate in combination with encapsulated ODN 1 m or dendritic cells.

## DETAILED DESCRIPTION OF THE INVENTION

The invention provides adjuvants, vaccines, the use thereof, and the use of formulations based on the discovery that methylated nucleic acids, particularly methylated oligonucleotides, and more particularly methylated oligonucleotides bearing a methylated cytosine of a CpG dinucleotide motif can enhance stimulation of immune responses either *in vitro, ex vivo* and *in vivo,* by encapsulation of the nucleic acid in a lipid particle. It is further disclosed that lipid-encapsulated methylated nucleic acids, which are ordinarily not immunostimulatory in their free form *in vivo,* can in fact be just as effective and in some cases more effective at stimulating immune responses when encapsulated in the subject formulations in comparison with their unmethylated counterparts.

The invention is exemplified by testing methylated and unmethylated counterparts of various oligonucleotides, configured with various backbones and encapsulated in various formulations of lipid particles. The lipid encapsulated methylated oligonucleotides are immunostimulatory with ordinary phosphodiester (PO) backbones as well as phosphorothioate (PS) backbones. It is further disclosed that, in some cases, the PO backbones may enhance a Th-1 mediated cellular immune response, while the PS backbones may stimulate a Th-1 mediated humoral immune response. In certain aspects of the invention, the lipid encapsulated methylated oligonucleotides are further combined with target antigens, particularly microbial antigens and/or tumor-associated antigens.

In particular, when used in accordance with the present invention the immunostimulatory activity of methylated nucleic acid sequences is significantly enhanced and improved immune stimulation is consistently achieved *in vivo,* in marked contrast to the widely variable results reported in the prior art. Further, immunostimulatory activity is obtained

in a broader class of antigen-presenting cells, and in particular in dendritic cells. The invention demonstrates for the first time, activation and expansion of dendritic cells by treatment with methylated oligonucleotides when they are lipid encapsulated. Accordingly *in vitro* methods of enhancing activation and/or expansion of dendritic cells is another aspect of the invention. Detailed methods of making, using and testing the various formulations of the invention are described hereafter and in the references cited herein.

Abbreviations and Definitions

The following abbreviations are used herein: RBC, red blood cells ; DDAB, N, N- distearyl-N,N-dimethylammonium bromide; DODAC,N, N-dioleyl-N, N-dimethylammonium chloride ; DOPE, 1, 2-sn-dioleoylphoshatidylethanolamine ; DOSPA, 2,3-dioleyloxy-N- (2(sperminecarboxamido) ethyl)-N, N-dimethyl-1-propanaminiu m trifluoroacetate ; DOTAP, 1,2-dioleoyloxy-3- (N, N,N-trimethylamino) propane chloride ; DOTMA, 1,2-dioleyloxy-3- (N, N, N- trimethylamino) propanechloride ; OSDAC, N-oleyl-N-stearyl-N, N-dimethylammonium chloride ; RT, room temperature; HEPES,4-(2-hydroxyethyl)-1-piperazineethanesulfonic acid; FBS, fetal bovine serum; DMEM, Dulbecco's modified Eagle's medium; PEG-Cer-C.sub.14, 1-O-(2'-(.omega.-methoxypolyethyleneglycol)succinoyl)-2-N-myristoyl-sphing osine; PEG-Cer-C.sub.20, 1-0- (2'-(.omega.-methoxypolyethyleneglycol)succinoyl)-2-N-arachidoyl-sphin gosine; PBS, phosphate-buffered saline; THF, tetrahydrofuran; EGTA, ethylenebis(oxyethylenenitrilo)- tetraacetic acid; SF-DMEM, serum-free DMEM; NP40, nonylphenoxypolyethoxyethanol, 1,2 dioleoyl-3 dimethylaminopropane (DODAP), palmitoyl oleoyl phsphatidylcholine (POPC) anddistearoylphosphatidylcholine (DSPC).

The technical and scientific terms used herein have the meanings commonly understood by one of ordinary skill in the art to which the present invention pertains, unless otherwise defined. Reference is made herein to various methodologies known to those of skill in the art. Standard reference works setting forth the general principles of recombinant DNA technology include Sambrook, J., et al., Molecular Cloning,: A Laboratory Manual, 2d Ed., Cold Spring Harbor Laboratory Press, Planview, N. Y. (1989); McPherson, M. J. , Ed., Directed Mutagenesis: A Practical Approach, IRL Press, Oxford (1991); Jones, J., Amino Acid and Peptide Synthesis, Oxford Science Publications, Oxford (1992); Austen, B. M. and Westwood, O.M.R., Protein Targeting and Secretion, IRL Press, Oxford (1991). Any suitable materials and/or methods known to those of skill can be utilized in carrying out the present invention; however, preferred materials and/or methods are described. Materials, reagents and the like to which reference is made in the following description and examples are obtainable from commercial sources, unless otherwise noted. It is believed that one skilled in the art can, based on the description herein, utilize the present invention to its fullest extent.

The immunostimulatory compositions used in the methods of the present invention will generally be referred to as lipid-therapeutic agent ("LTA") formulations comprising at least one lipid component and at least one therapeutic agent, and having greater immunostimulatory activity than thee therapeutic agent alone, *in vivo.* "Therapeutic agent" or "therapeutic compound" or "drug" as used herein can be used interchangeably and refer to any synthetic, recombinant, or naturally occurring molecule that provides a beneficial effect in medical treatment of a subject. Examples of therapeutic agents include, by are not limited to nucleic acids, peptides, and chemicals.

The therapeutic agent may comprise at least one methylated oligonucleotide, and most preferably at least one methylated oligodeoxynucleotide ("ODN"). The methylated cytosine residue is part of a CpG dinucleotide motif located in said sequence. The CpG comprises a methyl or hydroxymethyl group attached to the carbon-4 position (4-mC) or carbon-5 postion (5-mC) of at least one cytosine. In further embodiments, the methylated nucleic acid sequence may alternatively or additionally comprise methyl modifications of the deoxribose or ribose sugar moiety as described in Henry et al. 2000 J. Pharmacol. Exp. Ther. 292:468, Zhao et al. 1999 Bioorg. Med. Chem Lett. 9:3453, Zhao et al. 2000 Biorg Med. Chem Lett. 10:1051. In a particularly preferred embodiment, the ODN comprises a methylated nucleic acid sequence that has immunostimulatory activity and is designated an immunostimulatory sequence ("ISS") in non-methylated form.

"Subject" or"host" as used herein refers to an organism, male or female, having an immune system, preferably an animal, more preferably a vertebrate, even more preferably a mammal, still even more preferably a rodent, and most preferably a human. Further examples of a subject include, but are not limited to, dogs, cats, cows, horses, pigs, sheep, goats, mice, rabbits, and rats. "Patient" as used herein refers to a subject in need of treatment for a medical condition (e.g., disease or disorder).

*"In vivo"* as used herein refers to an organism, preferably in a mammal, more preferably in a rodent, and most preferably in a human.

"Immunostimulatory," "immunostimulatory activity" or "stimulating an immune response," and grammatical equivalents thereof, as used herein refers to inducing, increasing, enhancing, or modulating an immune response, or otherwise providing a beneficial effect with respect to an immune response. As used herein "immune response" refers to systemic and/or mucosal immune responses. Preferably, and in view of the wide variation in *in vitro* experimental results reported in the prior art, the immunostimulatory activity of a given formulation and nucleic acid sequence is determined in a suitable *in vivo* assay as described herein.

"A target antigen" as used herein refers to an antigen of interest to which a immune response can be directed or stimulated. The target antigen used in the compositions of the present invention for stimulating an immune response directed to that target antigen may be a synthetic, naturally-occurring or isolated molecule or a fragment thereof, and may comprise single or multiple epitopes. Thus, the compositions of the present invention may stimulate immune responses directed to single or multiple epitopes of an antigen. In preferred embodiments, the target antigen is associated with the lipid particles of the present invention. "In association with", "associated with", or grammatical equivalents thereof, as used herein with reference to an antigen (or target antigens), refers to antigens that are attached to or encapsulated by another component. With reference to the lipid particles or liposomes of the present invention, the antigen may be, for example, encapsulated in the lumen or intralamellar spaces of the lipid particles; disposed or attached within or partially within the lipid membrane, or attached (*e.g.*, covalently or ionically) to the lipid particle. The antigen may be attached to the interior of the lipid particle or, more preferably, the antigen is attached to the exterior of the lipid particle. In preferred embodiments the antigen is encapsulated within the lipid particle.

Examples of antigens useful in the compositions and methods of the present invention include, but are not limited to, peptides or proteins, cells, cell extracts, polysaccharides, polysaccharide conjugates, lipids, glycolipids, glycopeptides, and carbohydrates. In one embodiment, the antigen is in the form of a peptide or protein antigen. In another embodiment, the antigen is a nucleic acid encoding a peptide or protein in a form suitable for expression in a subject and presentation to the immune system of that subject. In a preferred embodiment, the compositions used in the methods of the present invention comprise a peptide or protein target antigen that stimulates an immune response to that target antigen in a mammal. Preferably, the target antigen is a pathogen ("target pathogen") capable of infecting a mammal including, for example, bacteria, viruses, fungi, yeast, parasites and other microorganisms capable of infecting mammalian species. Alternatively, the target antigen may be a tumor-associated antigen.

A "tumor-associated antigen" as used herein is a molecule or compound (e.g., a protein, peptide, polypeptide, lipid, glycolipid, carbohydrate and/or DNA) associated with a tumor or cancer cell and which is capable of provoking an immune response when expressed on the surface of an antigen presenting cell in the context of an MHC molecule. Tumor-associated antigens include self antigens, as well as other antigens that may not be specifically associated with a cancer but nonetheless enhance an immune response to and/or reduce the growth of a cancer when administered to an animal. In view of the potential risk of autoimmune reactions, the use of self antigens in the subject vaccines may be limited to non-critical tissues such as breast, prostate, testis, melanocytes, etc. More specific embodiments are provided herein.

A "microbial antigen" as used herein is an antigen of a microorganism and includes but is not limited to, infectious virus, infectious bacteria, infectious parasites and infectious fungi. Microbial antigens may be intact microorganisms, and natural isolates, fragments, or derivatives thereof, synthetic compounds which are identical to or similar to naturally-occurring microbial antigens and, preferably, induce an immune response specific for the corresponding microorganism (from which the naturally-occurring microbial antigen originated). In a preferred embodiment, a compound is similar to a naturally-occurring microorganism antigen if it induces an immune response (humoral and/or cellular) to a naturally-occurring microorganism antigen. Compounds or antigens that are similar to a naturally-occurring microorganism antigen are well known to those of ordinary skill in the art. A non-limiting example of a compound that is similar to a naturally-occurring microorganism antigen is a peptide mimic of a polysaccharide antigen. More specific embodiments are provided herein.

The term "antigen" is further intended to encompass peptide or protein analogs of known or wild-type antigens such as those described above. The analogs may be more soluble or more stable than wild type antigen, and may also contain mutations or modifications rendering the antigen more immunologically active. Also useful in the compositions and methods of the present invention are peptides or proteins which have amino acid sequences homologous with a desired antigen's amino acid sequence, where the homologous antigen induces an immune response to the respective pathogen.

"Homologous" as used herein refers to the subunit sequence similarity between two polymeric molecules, e.g., between two nucleic acid molecules (e.g., two DNA molecules or two RNA molecules) or two polypeptide molecules. When a subunit position in both molecules is occupied by the same monomeric subunit, e. g., if a position in each of two DNA molecules is occupied by adenine, then they are homologous at that position. The homology between two sequences is a direct function of the number of matching or homologous positions, e. g., if half (e.g. five positions in a polymer ten subunits in length) of the positions in two compound sequences are homologous then the two sequences are 50% homologous, if 90% of the positions, e. g., 9 of 10, are matched or homologous, the two sequences share 90% homology. By way of example, the DNA sequences 5'-CCGTTA-3'and 5'-GCGTAT-3' share 50% homology. By the term "substantially homologous" as used herein, is meant DNA or RNA which is about 50% homologous, more preferably about 70% homologous, even more preferably about 80% homologous and most preferably about 90% homologous to the desired nucleic acid. Genes which are homologous to the desired antigen-encoding sequence should be construed to be included in the invention provided they encode a protein or polypeptide having a biological activity substantially similar to that of the desired antigen. Where in this text, protein and/or DNA sequences are defined by their percent

homologies or identities to identified sequences, the algorithms used to calculate the percent homologies or percent identities include the following: the Smith-Waterman algorithm (J. F. Collins et al, Comput. Appl. Biosci. , (1988) 4: 67-72; J. F. Collins et al, Molecular Sequence Comparison and Alignment, (M. J. Bishop et al, eds.) In Practical Approach Series: Nucleic Acid and Protein Sequence Analysis XVIII, IRL Press: Oxford, England, UK (1987) 417), and the BLAST and FASTA programs (E.G. Shpaer et al, 1996, Genomics, 38: 179-191).

Analogs of the antigens described herein can differ from naturally occurring proteins or peptides by conservative amino acid sequence differences or by modifications which do not affect sequence, or by both. For example, conservative amino acid changes may be made, which although they alter the primary sequence of the protein or peptide, do not normally alter its function. Modifications (which do not normally alter primary sequence) include *in vivo,* or *in vitro* chemical derivatization of polypeptides, e.g., acetylation, or carboxylation. Also contemplated as antigens are proteins modified by glycosylation, e.g., those made by modifying the glycosylation patterns of a polypeptide during its synthesis and processing or in further processing steps; e.g., by exposing the polypeptide to enzymes which affect glycosylation, e.g., mammalian glycosylating or deglycosylating enzymes. Also contemplated as antigens are amino acid sequences which have phosphorylated amino acid residues, e.g., phosphotyrosine, phosphoserine, or phosphothreonine. Also contemplated as antigens are polypeptides which have been modified using ordinary molecular biological techniques so as to improve their resistance to proteolytic degradation or to optimize solubility properties. Analogs of such polypeptides include those containing residues other than naturally occurring L-amino acids, e.g., D-amino acids or non-naturally occurring synthetic amino acids.

The antigens of the present invention are not limited to products of any of the specific exemplary processes listed herein. In addition to substantially full length polypeptides, the antigens useful in the present invention include immunologically active fragments of the polypeptides. For example, the antigen may be a fragment of a complete antigen including at least one epitope. "Epitope" as used herein refers to any antigenic determinant on an antigen to which the paratope of an antibody can bind. Epitopic determinants usually consist of chemically active surface groupings of molecules such as, e.g., amino acids or sugar side chains and usually have specific three-dimensional structural characteristics. Particularly preferred embodiments of the compositions and methods of the present invention include combination antigens which include multiple epitopes from the same target antigen, or epitopes from two or more different target antigens (i.e., polytope vaccines). For example, the combination antigens can be the same or different type such as, e.g., a peptide-peptide antigen, glycolipid-peptide antigen, or glycolipid-glycolipid antigen.

A polypeptide or antigen is "immunologically active" if it induces an immune response to a target antigen or pathogen. "Vaccine" as used herein refers to a composition comprising a target antigen that stimulates a specific immune response to that target antigen.

"Adjuvant" as used herein refers to any substance which can stimulate or enhance the stimulation of an immune responses. Some adjuvants can cause activation of a cell of the immune system, for example, an adjuvant can cause an immune cell to produce and secrete cytokines. Examples of adjuvants that can cause activation of a cell of the immune system include, but are not limited to, saponins purified from the bark of the Q. saponaria tree, such as QS21 (a glycolipid that elutes in the 21 st peak with HPLC fractionation; Aquila Biopharmaceuticals, Inc., Worcester, Mass.); poly(di(carboxylatophenoxy)phosphazene (PCPP polymer; Virus Research Institute, USA); derivatives of lipopolysaccharides such as monophosphoryl lipid A (MPL; Ribi ImmunoChem Research, Inc., Hamilton, Mont.), muramyl dipeptide (MDP; Ribi) and threonyl-muramyl dipeptide (t-MDP; Ribi); OM-174 (a glucosamine disaccharide related to lipid A; OM Pharma SA, Meyrin, Switzerland) ; and Leishmaniaelongation factor (a purified Leishmania protein; Corixa Corporation, Settle, Wash.). Traditional adjuvants are well known in the art and include, for example, aluminum phosphate or hydroxide salts ("alum").

As compared to known adjuvants, the present invention provides improved adjuvants comprising combinations of lipids and nucleic acids that act synergistically to stimulate enhanced, Th-1 biased immune responses. Such compositions of the present invention comprise a nucleic acid component and a lipid component. The nucleic acid component comprises at least one methylated oligonucleotide, wherein said at least one methylated oligonucleotide comprises at least one CpG dinucleotide having a methylated cytosine.

In preferred embodiments the immunostimulatory compositions used in the methods of the present invention comprise a lipid component comprising a lipid membrane that encapsulates a therapeutic agent. As used herein "liposomal particle," "liposome," "lipidvesicle," and "liposomal vesicle, "or grammatical equivalents thereof, may be used interchangeably and refer to structures, particles, complexes, or formulations comprising lipid- containing membranes which enclose or encapsulate an aqueous interior. In preferred embodiments, the liposomes enclose or encapsulate therapeutic agents, e.g., nucleic acids. The liposomes may have one or more lipid membranes. Liposomes having one lipid-containing membrane are referred to herein as "unilamellar." Liposomes having multiple lipid-containing membranes are referred to herein as "multilamellar." "Lipid bilayer" as used herein refers to a lipid-containing membrane having two layers. In preferred embodiments, the liposomes are multilamellar.

The immunostimulatory compositions used in the methods of the present invention generally comprise lipid particles encapsulating at least one methylated nucleic acid. Nucleic Acids

Nucleic acids suitable for use in the compositions of the present invention include, for example, DNA or RNA. Preferably the nucleic acids are oligonucleotides, more preferably ODNs, and most preferably an ODN comprising an ISS ("ISS ODN") and at least one methylated cytosine.

"Nucleic acids" as used herein refer to multiple nucleotides (i.e., molecules comprising a sugar (e.g. ribose or deoxyribose) linked to a phosphate group and to an exchangeable organic base, which is either a substituted pyrimidine (e.g. cytosine (C), thymine (T) or uracis (U)) or a substituted purine (e.g. adenine (A) or guanine (G)). Nucleic acids may be, for example DNA or RNA. Preferably the nucleic acids are oligoribonucleotides and more preferably ODNs. Nucleic acids may also be polynucleosides, i.e., a polynucleotide minus the phosphate and any other organic base containing polymer. The immunostimulatory compositions of the present invention comprise a nucleic acid component. "Nucleic acid component" as used herein with reference to compositions of the present invention refers to a component comprising nucleic acids.

In a preferred embodiment, the oligonucleotides are single stranded and in the range of 5 - 50 nucleotides ("nt") in length. However, any oligonucleotides may be used including, for example, large double stranded plasmid DNA in the range of 500 - 50,000 base pairs ("bp").

Nucleic acids useful in the compositions and methods of the present invention can be obtained from known sources or isolated using methods well known in the art. The nucleic acids can also be prepared by recombinant or synthetic methods which are equally well known in the art. Such nucleic acids can then be encapsulated in lipid particles and the resulting compositions tested for immunostimulatory activity using the methods of the present invention as described herein.

For use *in vivo,* nucleic acids may be resistant to degradation (e.g., via endo-and exonucleases). Secondary structures, such as stem loops, can stabilize nucleic acids against degradation. Alternatively, nucleic acid stabilization can be accomplished via phosphate backbone modifications. A preferred stabilized nucleic acid has at least a partial phosphorothioate modified backbone. Phosphorothioates may be synthesized using automated techniques employing either phosphoramidate or H-phosphonate chemistries. Aryl-and alkylphosphonates can be made, e.g., as described in U.S. Patent No. 4,469,863; and alkylphosphotriesters (in which the charged oxygen moiety is alkylated as described in U.S. Patent No. 5,023,243 and European Patent No. 092,574) can be prepared by automated solid phase synthesis using commercially available reagents. Methods for making other DNA backbone modifications and substitutions have been described (Uhlmann and Peyman, Chem. Rev. 90:544, 1990; Goodchild, Bioconjugate Chem. 1:165, 1990). As described herein, however, the methods and compositions of the present invention alleviate the need to include such modifications to the subject nucleic acids.

Thus, oligonucleotides useful in the compositions and methods of the present invention may have a modified phosphate backbone such as, e.g., phosphorothioate, methylphosphonate, methylphosphorothioate, phosphorodithioate, and combinations thereof with each other and/or with phosphodiester oligonucleotide. In addition, other modified oligonucleotides include: nonionic DNA analogs, such as alkyl- and aryl-phosphates (in which the charged phosphonate oxygen is replaced by an alkyl or aryl group), phosphodiester and alkylphosphotriesters, in which the charged oxygen moiety is alkylated. As demonstrated herein, PO ODN may be preferred where cellular immune responses are desired, while modified ODN such as, e. g. , PS ODN may be preferred where humoral responses are desired.

Numerous other chemical modifications to the base, sugar or linkage moieties are also useful. Bases may be methylated or unmethylated. In the preferred embodiments, methyl or hydroxymethyl groups are attached to the carbon-4 position (4-mC) or carbon-5 position (5-mC) of at least one cytosine. The methylated cytosine is preferably located within a CpG motif in the nucleic acid sequence. Alternatively or additionally, the sugar moiety may be modified with a methyl group as described in the art.

Nucleic acid sequences useful in the compositions and methods of the present invention may be complementary to patient/subject mRNA, such as antisense oligonucleotides, or they may be foreign or non-complementary (e.g., the nucleotide sequences do not specifically hybridize to the patient/subject genome). The nucleotide sequences may be expressed and the resulting expression products may be RNA and/or protein. In addition, such nucleotide sequences may be linked to appropriate promoters and expression elements, and may be contained in an expression vector. Nucleotide sequences useful in the composition and methods of the present invention may be ISS, such as certain palindromes leading to hairpin secondary structures (see Yamamoto S., et al. (1992) J. Immunol. 148: 4072-4076), or CpG motifs, or other known ISS features (such as multi-G domains, see WO 96/11266). In a particularly preferred embodiment, the nucleotide sequence comprises at least one CpG motif having a methylated cytosine.

The nucleic acids useful in the present invention can be synthesized de novo using any of a number of procedures well known in the art. For example, the b-cyanoethyl phosphoramidite method (Beaucage, S. L., and Caruthers, M. H., Tet. Let. 22: 1859,1981); nucleoside H- phosphonate method (Garegg et al., Tet. Let. 27:4051-4054, 1986; Froehler et al., Nucl. Acid. Res. 14: 5399-5407,1986,; Garegg et al., Tet. Let. 27:4055-4058, 1986, Gaffney et al, Tet. Let. 29: 2619-2622, 1988). These chemistries can be performed by a variety of automated oligonucleotide synthesizers available in the market. Also, CpG dinucleotides can be produced on a large scale in plasmids, (see Sambrook, T., et al., Molecular Cloning: A Laboratory Manual, Cold Spring Harbor laboratory Press, New York, 1989). Such plasmids may also encode

other genes to be expressed such as an antigen-encoding gene in the case of a DNA vaccine. Oligonucleotides can be prepared from existing nucleic acid sequences (e.g., genomic or cDNA) using known techniques, such as those employing restriction enzymes, exonucleases or endonucleases.

For administration in vivo, compositions of the present invention, including components of the compositions, e.g., a lipid component or a nucleic acid component, may be associated with a molecule that results in higher affinity binding to target cell (e.g., B-cell, monocytic cell and natural killer (NK) cell) surfaces and/or increased cellular uptake by target cells. The compositions of the present invention, including components of the compositions, can be ionically orcovalently associated with desired molecules using techniques which are well known in the art. A variety of coupling or cross-linking agents can be used, e.g., protein A, carbodiimide, andN-succinimidyl-3- (2-pyridyldithio) propionate (SPDP).

The immune stimulating activity of a nucleic acid sequence in an organism can be determined by simple experimentation, for example, by comparing the sequence in question with other immunostimulatory agents, e. g., other adjuvants, or ISS; or by detecting or measuring the immunostimulatory activity of the sequence in question, e.g., by detecting or measuring the activation of host defense mechanisms or the activation of immune system components. Such assays are well known in the art. Also, one of skill in the art would know how to identify the optimal oligonucleotides useful for a particular mammalian species of interest using routine assays described herein and/or known in the art.

Specific nucleic acid sequences of ODNs suitable for use in the compositions and methods of the invention are described in U. S. Patent Appln. 60/379,343, U.S. Patent Appln. No. 09/649,52, Int. Publ. WO 02/069369, Int. Publ. No. WO 01/15726, U. S. Patent No. 6,406,705, and Raney et al., Journal of Pharmacology and Experimental Therapeutics, 298:1185-1192 (2001). Exemplary sequences of the ODNs are shown in Table 1. In preferred embodiments, ODNs used in the compositions and methods of the present invention have a phosphodiester ("PO") backbone or a phosphorothioate ("PS") backbone. Only those ODNs comprising at least one methylated cytosine residue in at least one CpG motif form part of the present invention.

Table 1

| ODN NAME | ODN SEQ ID NO | ODN SEQUENCE (5'-3') |
|---|---|---|
| ODN 1 (INX-6295) 3 human c-myc | SEQ ID NO: 2 | 5'-TAACGTTGAGGGGCAT- |
| ODN 1m (INX-6303) | SEQ ID NO: 4 | 5'-TAAZGTTGAGGGGCAT-3 |
| ODN 2 (INX-1826) | SEQ ID NO: 1 | 5'-TCCATGACGTTCCTGACGTT-3 |
| ODN 2m (INX-1826m) | SEQ 1D NO: 31 | 5'-TCCATGAZGTTCCTGAZGTT-3 |
| ODN 3 (INX-6300) | SEQ ID NO: 3 | 5'-TAAGCATACGGGGTGT-3 |
| ODN 5 (INX-5001) | SEQ ID NO: 5 | 5'-AACGTT-3 |
| ODN 6 (INX-3002) | SEQ ID NO: 6 | 5'-GATGCTGTGTCGGGGTCTCCGGGC-3' |
| ODN 7 (INX-2006) | SEQ ID NO: 7 | 5'-TCGTCGTTTTGTCGTTTTGTCGTT-3' |
| ODN 7m (INX-2006m)- | SEQ ID NO: 32 | 5'-TZGTZGTTTTGTZGTTTTGTZGTT-3' |
| ODN 8 (INX-1982) | SEQ ID NO: 8 | 5'-TCCAGGACTTCTCTCAGGTT-3' |
| ODN 9 (INX-G3139) | SEQ ID NO: 9 | 5'-TCTCCCAGCGTGCGCCAT-3' |
| ODN 10 (PS-3082) murine Intracellular Adhesion Molecule-1 | SEQ ID NO: 10 | 5'-TGCATCCCCCAGGCCACCAT-3 |
| ODN 11 (PS-2302) human Intracellular Adhesion Molecule-1 | SEQ ID NO: 11 | 5'-GCCCAAGCTGGCATCCGTCA-3' |
| ODN 12 (PS-8997) human Intracellular Adhesion Molecule-1 | SEO ID NO: 12 | 5'-GCCCAAGCTGGCATCCGTCA-3' |
| ODN 13 (US3) human erb-B-2 | SEQ ID NO: 13 | 5'-GGT GCTCACTGC GGC-3' |
| ODN 14 (LR-3280) human c-myc | SEQ ID NO: 14 | 5'-AACC GTT GAG GGG CAT-3' |
| ODN 15 (LR-3001) human c-myc | SEQ ID NO: 15 | 5'-TAT GCT GTG CCG GGG TCT TCG GGC-3' |
| ODN 16 (Inx-6298) | SEQ ID NO: 16 | 5'-GTGCCG GGGTCTTCGGGC-3' |

(continued)

| ODN NAME | ODN SEQ ID NO | ODN SEQUENCE (5'-3') |
|---|---|---|
| ODN 17 (hIGF-1 R) human Insulin Growth Factor 1 - Receptor | SEQ ID NO: 17 | 5'-GGACCCTCCTCCGGAGCC-3' |
| ODN 18 (LR-52) human Insulin Growth Factor 1 - Receptor | SEQ ID NO: 18 | 5'-TCC TCC GGA GCC AGA CTT-3' |
| ODN 19 (hEGFR) human Epidermal Growth Factor - Receptor | SEQ ID NO: 19 | 5'-AAC GTT GAG GGG CAT-3' |
| ODN 20 (EGFR) Epidermal Growth Factor - Receptor | SEQ ID NO: 20 | 5'-CCGTGGTCA TGCTCC-3' |
| ODN-21 (hVEGF) human Vascular Endothelial Growth Factor | SEQ ID NO: 21 | 5'-CAG CCTGGCTCACCG CCTTGG-3' |
| ODN 22 (PS-4189) murine Phosphokinase C - alpha | SEQ ID NO: 22 | 5'-CAG CCA TGG TTC CCC CCA AC-3' |
| ODN 23 (PS-3521) | SEQ ID NO: 23 | 5'-GTT CTC GCT GGT GAG TTT CA-3' |
| ODN 24 (hBcl-2) human Bcl-2 | SEQ ID NO: 24 | 5'-TCT CCCAGCGTGCGCCAT-3' |
| ODN 25 (hC-Raf-1) human C-Raf-s | SEQ ID NO: 25 | 5'-GTG CTC CAT TGA TGC-3' |
| ODN #26 (hVEGF-R1) human Vascular Endothelial Growth Factor Receptor-1 | SEQ ID NO: 26 | 5'-GAGUUCUGAUGAGGCCGAAAGGCCG AAAGUCUG-3' |
| ODN #27 | SEQ ID NO: 27 | 5'-RRCGYY-3' |
| ODN # 28 (INX-3280) | SEQ ID NO: 28 | 5'-AACGTTGAGGGGCAT-3' |
| ODN #29 (INX-6302) | SEQ ID NO: 29 | 5'-CAACGTTATGGGGAGA-3' |
| ODN #30 (INX-6298) human c-myc | SEQ ID NO: 30 | 5'-TAACGTTGAGGGGCAT-3' |

"Z" represents a methylated cytosine residue.
Note: ODN 14 is a 15-mer oligonucleotide and ODN 1 is the same oligonucleotide having a thymidine added onto the 5' end making ODN 1 into a 16-mer. No difference in biological activity between ODN 14 and ODN 1 has been detected and both exhibit similar immunostimulatory activity (Mui *et al.,* 2001)

**Lipids and other components**

Lipid formulations and methods of preparing liposomes as delivery vehicles are known in the art, and any of number of such formulations may find advantageous use herein, including those described in U.S. Patent No. 6,465,439, U.S. Patent No. 6,379,698, U.S. Patent No. 6,365,611, and U.S. Patent No. 6,093,816.
Preferred lipid formulations are the lipid particle formulations described herein and more fully described in, for example, U.S. Patent No. 5,785,992, U.S. Patent No. 6,287,591, U.S. Patent No. 6,287,591 B1, co-pending U.S. Patent Appln. Ser. No. 60/379,343, and co-pending U.S. Patent Appln. Ser. No. 09/649,527.

In one preferred embodiment, the preferred lipid formulation is DSPC, DODMA, Chol, and PEG-DMG having a ratio of 20:25:45:10 mol/mol. As used herein, the molar amount of each lipid in a lipid formulation is given in the same order that the lipid is listed (e.g., the ratio of DSPC to DODMA to Chol to PEG-DMG is 20 DSPC: 25 DODMA: 45 Chol; 10 PEG-DMG or "20:25:45:10"). In alternate embodiments the DSPC may be replaced with POPC, the DODMA replaced with DODAP and the PEG-DMG replaced with PEGCer14 or PEGCer20.

The term "lipid" refers to a group of organic compounds that are esters of fatty acids and are characterized by being insoluble in water but soluble in many organic solvents. They are usually divided in at least three classes: (1) "simple lipids" which include fats and oils as well as waxes; (2) "compound lipids" which include phospholipids and glycolipids; and (3) "derived lipids" such as steroids and compounds derived from lipid manipulations. A wide variety of lipids may be used with the invention, some of which are described below.

The term "charged lipid" refers to a lipid species having either a cationic charge or negative charge or which is a zwitterion which is not net neutrally charged, and generally requires reference to the pH of the solution in which the lipid is found.

Cationic charged lipids at physiological pH include, but are not limited to, N,N-dioleyl-N,N-dimethylammonium chloride ("DODAC"); N-(2,3-dioleyloxy)propyl)-N,N,N-trimethylammonium chloride ("DOTMA"); N,N-distearyl-N,N-dimethylammonium bromide ("DDAB"); N-(2,3-dioleytoxy)propyl)-N,N,N-trimethylammonium chloride ("DOTAP"); 3b-(N-(N',N'-dimethylaminoethane)-carbamoyl)cholesterol ("DC-Chol") and N-(1,2-dimyristyloxyprop-3-yl)-N,N-dimethyl-N-hydroxyethyl ammonium bromide ("DMRIE"). Additionally, a number of commercial preparations of catioinic lipids are available which can be used in the present invention. These include, for example, Lipofectin™ (commercially available cationic liposomes comprising DOTMA and 1,2-dioleoyl-sn-3-phosphoethanolamine ("DOPE"), from GIBCO/BRL, Grand Island, New York, U.S.A); and Lipofectamine™ (commercially available cationic liposomes comprising N-(1-(2,3-dioleyloxy)propyl)-N-(2-(sperminecarboxamido)ethyl)-N,N-dimethylammonium trifluoroacetate ("DOSPA").

Some cationic charged lipids are titratable, that is to say they have a pKa at or near physiological pH, with the significant consequence for this invention that they are strongly cationic in mild acid conditions and weakly (or not) cationic at physiological pH. Such cationic charged lipids include, but are not limited to, N-(2,3-dioleyloxy)propyl)-N,N-dimethylammonium chloride ("DODMA") and 1,2-Dioleoyl-3-dimethylammonium-propane ("DODAP"). DMDMA is also a useful titratable cationic lipid.

Anionic charged lipids at physiological pH include, but are not limited to, phosphatidyl inositol, phosphatidyl serine, phosphatidyl glycerol, phosphatidic acid, diphosphatidyl glycerol, poly(ethylene glycol)-phosphatidyl ethanolamine, dimyristoylphosphatidyl glycerol, dioleoylphosphatidyl glycerol, dilauryloylphosphatidyl glycerol, dipalmitoylphosphatidyl glycerol, distearyloylphosphatidyl glycerol, dimyristoyl phosphatic acid, dipalmitoyl phosphatic acid, dimyristoyl phosphatidyl serine, dipalmitoyl phosphatidyl serine, brain phosphatidyl serine, and the like.

Some anionic charged lipids may be titrateable, that is to say they would have a pKa at or near physiological pH, with the significant consequence for this invention that they are strongly anionic in mild base conditions and weakly (or not) anionic at physiological pH. Such anionic charged lipids can be identified by one skilled in the art based on the principles disclosed herein.

The term "neutral lipid" refers to any of a number of lipid species which exist either in an uncharged or neutral zwitterionic form at physiological pH. Such lipids include, for example, diacylphosphatidylcholine, diacylphosphatidylethanolamine, ceramide, sphingomyelin, cephalin, cholesterol, cerebrosides and diacylglycerols.

Certain preferred lipid formulations used in the invention include aggregation preventing compounds such as PEG-lipids or polyamide oligomer-lipids (such as an ATTA-lipid), and other steric-barrier or "stealth"-lipids, detergents, and the like. Such lipids are described in U.S. Patent No. 4,320,121, U.S. Patent No. 5,820,873, U.S. Patent No. 5,885,613, Int. Publ. No. WO 98/51278, and U.S. Pat. Appln. Serial No. 09/218,988 relating to polyamide oligomers. These lipids and detergent compounds prevent precipitation and aggregation of formulations containing oppositely charged lipids and therapeutic agents. These lipids may also be employed to improve circulation lifetime *in vivo* (see Klibanov et al. (1990) FEBS Letters, 268 (1): 235-237), or they may be selected to rapidly exchange out of the formulation *in vivo* (see U.S. Patent No. 5,885,613,

A preferred embodiment of the invention employs exchangeable steric-barrier lipids (as described in U.S. Patent No. 5,820,873, U.S. Patent No. 5,885,613, and U.S. Pat. Appln. Ser. No. 09/094540 and U.S. Pat. No. 6,320,017. Exchangeable steric-barrier lipids such as PEG2000-CerC14 and ATTAB-CerC14 are steric-barrier lipids which rapidly exchange out of the outer monolayer of a lipid particle upon administration to a subject/patient. Each such lipid has a characteristic rate at which it will exchange out of a particle depending on a variety of factors including acyl chain length, saturation, size of steric barrier moiety, membrane composition and serum composition, etc. Such lipids are useful in preventing aggregation during particle formation, and their accelerated departure from the particle upon administration provides benefits, such as programmable fusogenicity and particle destabilizing activity, as described in the above noted patent submissions.

Some lipid particle formulations may employ targeting moieties designed to encourage localization of liposomes at certain target cells or target tissues. Targeting moieties may be associated with the outer bilayer of the lipid particle (i.e., by direct conjugation, hydrophobic interaction or otherwise) during formulation or post-formulation. These methods are well known in the art. In addition, some lipid particle formulations may employ fusogenic polymers such as PEAA, hemagluttinin, other lipo-peptides (see U.S. Pat. No. 6,417,326, and U.S. Pat. Appln. Ser. No. 09/674,191, all incorporated herein by reference) and other features useful for *in vivo* and/or intracellular delivery.

In another preferred embodiment, the lipid component lipid particles of the present invention comprises sphingomyelin and cholesterol ("sphingosomes"). In a preferred embodiment, the lipid particles used in the compositions and methods of the present invention are comprised of sphingomyelin and cholesterol and have an acidic intraliposomal pH. The lipid particles comprising sphingomyelin and cholesterol have several advantages when compared to other formulations. The sphingomyelin/cholesterol combination produces liposomes which have extended circulation lifetimes, are much more stable to acid hydrolysis, have significantly better drug retention characteristics, have better loading characteristics into tumors and the like, and show significantly better anti-tumor efficacy than other liposomal formulations tested.

In a preferred embodiment, the lipid particles of the present invention comprise a cationic compound of Formula

I and at least one neutral lipid as follows (and fully described in U.S. Pat. Serial No. 5,785,992, incorporated herein by reference).In a preferred embodiment, the LNA formulations of the present invention comprise a cationic compound of Formula I and at least one neutral lipid as follows (and fully described in U.S. Pat. Serial No. 5,785,992, incorporated herein by reference).

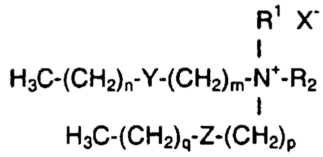

In Formula I, $R^1$ and $R^2$ are each independently $C_1$ to $C_3$; alkyl. Y and Z are akyl or alkenyl chains and are each independently: $-CH_2CH_2CH_2CH_2CH_2$--, --CH=$CHCH_2CH_2CH_2$--, --$CH_2$ CH=$CHCH_2CH_2$--, --$CH_2CH_2$CH=$CHCH_2$--, --$CH_2CH_2CH_2$CH=CH--,-CH=CHCH=$CHCH_2$--, --CH=$CHCH_2$CH=CH--, or --$CH_2$CH=CH-CH=CH--, with the proviso that Y and Z are not both --$CH_2CH_2CH_2CH_2CH_2$--. The letters n and q denote integers of from 3 to 7, while the letters m and p denote integers of from 4 to 9, with the proviso that the sums n+m and q+p are each integers of from 10 to 14. The symbol $X^-$ represents a pharmaceutically acceptable anion. In the above formula, the orientation of the double bond can be either cis or trans, however the cis isomers are generally preferred.

In another preferred embodiment, the cationic compounds are of Formula I, wherein $R^1$ and $R^2$ are methyl and Y and Z are each independently: --CH=$CHCH_2CH_2CH_2$--,-$CH_2$CH=$CHCH_2CH_2$--, --$CH_2CH_2$CH=$CHCH_2$-- or --$CH_2CH_2CH_2$CH=CH--. In preferred embodiments, $R^1$ and $R^2$ are methyl; Y and Z are each -CH=$CHCH_2CH_2CH_2$--; n and q are both 7; and m and p are both 5. In another preferred embodiment, the cationic compound is DODAC (N,N-dioleyl-N,N-dimethylammonium chloride). DODAC is a known in the art and is a compound used extensively as an additive in detergents and shampoos. DODA is also used as a co-lipid in liposomal compositions with other detergents (see, Takahashi, et al., GB 2147243).

The neutral lipids in the LNA formulations of the present invention can be any of a variety of neutral lipids which are typically used in detergents, or for the formation of micelles or liposomes. Examples of neutral lipids which are useful in the present compositions are, but are not limited to, diacylphosphatidylcholine, diacylphosphatidylethanolamine, ceramide, sphingomyelin, cephalin, cardiolipin, and cerebrosides. In a preferred embodiment, the present compositions will include one or more neutral lipids which are diacylphosphatidylcholine, diacylphosphatidylethanolamine, ceramide or sphingomyelin. The acyl groups in these neutral lipids are preferably acyl groups derived from fatty acids having $C_{10}$-$C_{24}$ carbon chains. More preferably the acyl groups are lauroyl, myristoyl, palmitoyl, stearoyl or oleoyl. In particularly preferred embodiments, the neutral lipid will be 1,2-sn-dioleoylphosphatidylethanolamine.

The anion, X-, can similarly be any of a variety a pharmaceutically acceptable anions. These anions can be organic or inorganic, including for example, $Br^-$, $Cl^-$, $F^-$, $I^-$, sulfate, phosphate, acetate, nitrate, benzoate, citrate, glutamate, and lactate. In preferred embodiments, $X^-$ is $Cl^-$ or $AcO^-$.

In addition to the other components described herein, the compositions of the present invention may contain a pharmaceutically acceptable carrier. Pharmaceutically acceptable carriers are well-known in the art. The choice of carrier is determined in part by the particular composition to be administered as well as by the particular method used to administer the composition. Preferably, the pharmaceutical carrier is in solution, in water or saline.

In the compositions of the present invention, the ratio of cationic compound to neutral lipid is preferably within a range of from about 25:75 (cationic compound:neutral lipid), or preferably to 75:25 (cationic compound:neutral lipid), or preferably about 50:50.

The cationic compounds which are used in the compositions of the present invention can be prepared by methods known to those of skill in the art using standard synthetic reactions (see March, Advanced Organic Chemistry, 4th Ed., Wiley-Interscience, NY, N.Y. (1992), incorporated herein by reference). For example, the synthesis of OSDAC can be carried out by first treating oleylamine with formaldehyde and sodium cyanoborohydride under conditions which result in the reductive alklation of the amine. This approach provides dimethyl oleylamine, which can then be alkylated with stearyl bromide to form the corresponding ammonium salt. Anion exchange results in the formation of OSDAC. Dimethyloleylamine can also be synthesized by treatment of oleyl bromide with a large excess of dimethylamine, and further derivatized as described above.

For cationic compounds in which both fatty acid chains are unsaturated (i.e., DODAC), the following general procedure can be used. An unsaturated acid (i.e., oleic acid) can be converted to its corresponding acyl chloride with such reagents as oxalyl chloride, thionyl chloride, PCl3 or PCl5. The acyl chloride can be treated with an unsaturated amine (i.e., oleylamine) to provide the corresponding amide. Reduction of the amide with, for example, lithium aluminum hydride provides a secondary amine wherein both alkyl groups are unsaturated long chain alkyl groups. The secondary

amine can then be treated with alkyl halides such as methyl iodide to provide a quaternary ammonium compound. Anion exchange can then be carried out to provide cationic compounds having the desired pharmaceutically acceptable anion. The alkylamine precursor can be synthesized in a similar manner. For example, treatment of an alkyl halide with a methanolic solution of ammonia in large excess will produce the required amine after purification. Alternatively, an acyl chloride, produced by treatment of the appropriate carboxylic acid with oxalyl chloride, can be reacted with ammonia to produce an amide. Reduction of the amide with LiAlH4 will provide the required alkylamine.

In preferred embodiments, the pharmaceutical compositions of the present invention are formulated as micelles or liposomes. Micelles containing the cationic compounds and neutral lipids of the present invention can be prepared by methods well known in the art. In addition to the micellar formulations of the present compositions, the present invention also provides micellar formulations which include other species such as lysophosphatidylcholine, lysophosphatidylethanolamine, lysophosphatidylserine, lysophosphatidylglycerol, phosphatidylethanolamine-polyoxyethylene conjugate, ceramide-polyoxyethylene conjugate or phosphatidic acid-polyoxyethylene conjugate.

The polyoxyethylene conjugates which are used in the compositions of the present invention can be prepared by combining the conjugating group (i.e. phosphatidic acid or phosphatidylethanolamine) with an appropriately functionalized polyoxyethylene derivative. For example, phosphatidylethanolamine can be combined with omega-methoxypolyethyleneglycol succinate to provide a phosphatidylethanolamine-polyoxyethylene conjugate (see, e.g., Parr, et al., Biochim. Biophys. Acta 1195:21-30 (1994), incorporated herein by reference).

The selection of neutral lipids for use in the compositions and methods of the present invention is generally guided by consideration of, e.g., liposome size and stability of the liposomes in the bloodstream. As described above, the neutral lipid component in the liposomes is a lipid having two acyl groups, (i.e., diacylphosphatidylcholine and diacylphosphatidyl-ethanolamine). Lipids having a variety of acyl chain groups of varying chain length and degree of saturation are available or may be isolated or synthesized by well-known techniques. In general, less saturated lipids are more easily sized, particularly when the liposomes must be sized below about 0.3 microns, for purposes of filter sterilization. In one group of embodiments, lipids containing saturated fatty acids with carbon chain lengths in the range of C14 to C22 are preferred. In another group of embodiments, lipids with mono or diunsaturated fatty acids with carbon chain lengths in the range of C14 to C22 are used. Additionally, lipids having mixtures of saturated and unsaturated fatty acid chains can be used.

Liposomes useful in the compositions and methods of the present invention may also be composed of sphingomyelin or phospholipids with other head groups, such as serine and inositol. Still other liposomes useful in the present invention will include cholesterol, diglycerides, ceramides, phosphatidylethanolamine-polyoxyethylene conjugates, phosphatidic acid-polyoxyethylene conjugates, or polyethylene glycol-ceramide conjugates (e.g., PEG-Cer-C14 or PEG-Cer-C20). Methods used in sizing and filter-sterilizing liposomes are discussed below.

A variety of methods are known in the art for preparing liposomes (see e.g., Szoka et al., Ann. Rev. Biophys. Bioeng. 9:467 (1980), U.S. Pat. Nos. 4,235,871, 4,501,728, 4,837,028, the text Liposomes, Marc J. Ostro, ed., Marcel Dekker, Inc., New York, 1983, Chapter 1, and Hope, et al., Chem. Phys. Lip. 40:89 (1986), all of which are incorporated herein by reference). One known method produces multilamellar vesicles of heterogeneous sizes. In this method, the vesicle-forming lipids are dissolved in a suitable organic solvent or solvent system and dried under vacuum or an inert gas to form a thin lipid film. If desired, the film may be redissolved in a suitable solvent, such as tertiary butanol, and then lyophilized to form a more homogeneous lipid mixture which is in a more easily hydrated powder-like form. This film is covered with an aqueous buffered solution and allowed to hydrate, typically over a 15-60 minute period with agitation. The size distribution of the resulting multilamellar vesicles can be shifted toward smaller sizes by hydrating the lipids under more vigorous agitation conditions or by adding solubilizing detergents such as deoxycholate.

Following liposome preparation, the liposomes may be sized to achieve a desired size range and relatively narrow distribution of liposome sizes. A size range of about 0.2-0.4 microns allows the liposome suspension to be sterilized by filtration through a conventional filter, typically a 0.22 micron filter. The filter sterilization method can be carried out on a high through-put basis if the liposomes have been sized down to about 0.2-0.4 microns.

Several techniques are available for sizing liposomes to a desired size. One sizing method is described in U.S. Patent No. 4,737,323.

Sonicating a liposome suspension either by bath or probe sonication produces a progressive size reduction down to small unilamellar vesicles less than about 0.05 microns in size. Homogenization is another method which relies on shearing energy to fragment large liposomes into smaller ones. In a typical homogenization procedure, multitamellar vesicles are recirculated through a standard emulsion homogenizer until selected liposome sizes, typically between about 0.1 and 0.5 microns, are observed. In both methods, the particle size distribution can be monitored by conventional laser-beam particle size discrimination.

Extrusion of liposomes through a small-pore polycarbonate membrane or an asymmetric ceramic membrane is also an effective method for reducing liposome sizes to a relatively well-defined size distribution. Typically, the suspension is cycled through the membrane one or more times until the desired liposome size distribution is achieved. The liposomes may be extruded through successively smaller-pore membranes, to achieve a gradual reduction in liposome

size. For use in the present inventions, liposomes having a size of from about 0.05 microns to about 0.15 microns are preferred.

As further described below, the compositions of the present invention can be administered to a subject by any known route of administration. Once adsorbed by cells, the liposomes (including the complexes previously described) can be endocytosed by a portion of the cells, exchange lipids with cell membranes, or fuse with the cells. Transfer or incorporation of the polyanionic portion of the complex can take place via any one of these pathways. In particular, when fusion takes place, the liposomal membrane can be integrated into the cell membrane and the contents of the liposome can combine with the intracellular fluid.

As described below in detail, additional components, which may also be therapeutic compounds, may be added to the lipid particles of the present invention to target them to specific cell types. For example, the liposomes can be conjugated to monoclonal antibodies or binding fragments thereof that bind to epitopes present only on specific cell types, such as cancer-related antigens, providing a means for targeting the liposomes following systemic administration. Alternatively, ligands that bind surface receptors of the target cell types may also be bound to the liposomes. Other means for targeting liposomes may also be employed in the present invention.

Following a separation step as may be necessary to remove free drug from the medium containing the liposome, the liposome suspension is brought to a desired concentration in a pharmaceutically acceptable carrier for administration to the patient or host cells. Many pharmaceutically acceptable carriers may be employed in the compositions and methods of the present invention. A variety of aqueous carriers may be used, e.g., water, buffered water, 0.4% saline, 0.3% glycine, and the like, and may include glycoproteins for enhanced stability, such as albumin, lipoprotein, globulin. Generally, normal buffered saline (135-150mM NaCl) will be employed as the pharmaceutically acceptable carrier, but other suitable carriers will suffice. These compositions may be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride. These compositions may be sterilized techniques referred to above or produced under sterile conditions. The resulting aqueous solutions may be packaged for use or filtered under aseptic conditions and lyophilized, the lyophilized preparation being combined with a sterile aqueous solution prior to administration.

The concentration of liposomes in the carrier may vary. In preferred embodiments, the concentration of liposomes is about 0.1-200 mg/ml. Persons of skill would know how to vary these concentrations to optimize treatment with different liposome components or for particular patients. For example, the concentration may be increased to lower the fluid load associated with treatment.

The cells of a subject are usually exposed to the compositions of the present invention by in vivo or ex vivo administration. The compositions of the present invention may be administered systemically, e.g., intravenously, with intramuscular, subcutaneous and topical administration also contemplated. Alternatively, intranasal or intratracheal administration may be used. Intratracheal administration may be provided as a liquid, preferably as an aerosol. For example, nebulizers may be used to create aerosols of droplets of between 70-100 μm in diameter. It will be understood that droplet size should generally be of greater size than the liposomes.

Multiple administrations to a patient are contemplated. The dosage schedule of the treatments will be determined by the disease and the patient's condition. Standard treatments with therapeutic compounds, including immunostimulatory compositions (*e.g.*, vaccines), that are well known in the art may serve as a guide to treatment with liposomes containing the therapeutic compounds. The duration and schedule of treatments may be varied by methods well known to those of skill, but the increased circulation time and decreased in liposome leakage will generally allow the dosages to be adjusted downward from those previously employed. The dose of liposomes of the present invention may vary depending on the clinical condition and size of the animal or patient receiving treatment. The standard dose of the therapeutic compound when not encapsulated may serve as a guide to the dose of the liposome-encapsulated compound. The dose will typically be constant over the course of treatment, although in some cases the dose may vary. Standard physiological parameters may be assessed during treatment that may be used to alter the dose of the liposomes of the invention.

## Antigens

As described herein, the liposomal encapsulated methylated nucleic acids may be associated with at least one target antigen. Antigens useful in the compositions and methods of the present invention may be inherently immunogenic, or non-immunogenic, or slightly immunogenic. Examples of antigens include, but are not limited to, synthetic, recombinant, foreign, or homologous antigens. Further examples of antigens include, but are not limited to, HBA - hepatitis B antigen (recombinant or otherwise); other hepatitis peptides; HIV proteins GP120 and GP160; Mycoplasma cell wall lipids; any tumor associated antigen; Carcinoembryonic Antigen (CEA); other embryonic peptides expressed as tumor specific antigens; bacterial cell wall glycolipids; Gangliosides (GM2, GM3); Mycobacterium glycolipids; PGL-1; Ag85B;

TBGL; Gonococcl lip-oligosaccharide epitope 2C7 from Neisseria gonorrhoeae; Lewis(y); and Globo-H; Tn; TF; STn; PorA; TspA or Viral glycolipids/glycoproteins and surface proteins.

The antigen may be in the form of a peptide antigen or it may be a nucleic acid encoding an antigenic peptide in a form suitable for expression in a subject and presentation to the immune system of the immunized subject. The antigen may also be a glycolipid or a glycopeptide. Further, the antigen may be a complete antigen, or it may be a fragment of a complete antigen including at least one therapeutically relevant epitope. "Combination antigens" as herein refer to antigens having multiple epitopes from the same target antigen, or multiple epitopes from two or more different target antigens (polytope vaccines) originating from the same type of target antigens (e.g., both antigens are peptides or both antigens are glycolipids), or different types of target antigens (e.g., glycolipid antigen and peptide antigen).

Antigens may be used in the compositions and methods of the present invention in a crude, purified, synthetic, isolated, or recombinant form. Polypeptide or peptide antigens, (including, for example, antigens that are peptide mimics of polysaccharides) encoded by nucleic acids may also be used in the compositions and methods of the present invention. The term antigen broadly includes any type of molecule which is recognized by a host immune system as being foreign. Antigens include but are not limited to cancer antigens, microbial antigens, and allergens.

A wide variety of antigens are suitable for use in the formulations of the present invention. Generally, an antigen is a material that is administered to a vertebrate host to immunize the host against the same material. Typically, an antigen comprises material associated with a disease state, such as viral infection, bacterial infection, and various malignancies. These materials may include but are not limited to proteins, peptides, polypeptides, lipids, glycolipids, carbohydrates and DNA.

The antigen of the lipid formulation may be encapsulated, associated, or mixed with the liposome or lipid particle. In certain embodiments of the present invention, the antigen is encapsulated in the liposome or lipid particle. In other embodiments, the antigen is mixed with the liposome or lipid particle. In other embodiments, the antigen is associated with the liposome or lipid particle. In one aspect, the antigen is adsorbed to the liposome or lipid particle. In other aspects, the antigen is covalently attached to the liposome or lipid particle. Methods used to covalently attach the antigen to the liposome or lipid particle are those standard methods known to those of skill in the art.

Examples of antigens suitable for use in the present invention include, but are not limited to, polypeptide antigens and DNA antigens. Specific examples of antigens are Hepatitis A, Hepatitis B, small pox, polio, anthrax, influenza, typhus, tetanus, measles, rotavirus, diphtheria, pertussis, tuberculosis, and rubella antigens. In a preferred embodiment, the antigen is a Hepatitis B recombinant antigen. In other aspects, the antigen is a Hepatitis A recombinant antigen. The antigen may be a tumor antigen. Examples of such tumor-associated antigens are MUC-1, EBV antigen and antigens associated with Burkitt's lymphom. In a further aspect, the antigen is a tyrosinase-related protein tumor antigen recombinant antigen. Those of skill in the art will know of other antigens suitable for use in the present invention.

Tumor-associated antigens include both mutated and non-mutated molecules which may be indicative of single tumor type, shared among several types of tumors, and/or exclusively expressed or over expressed in tumor cells in comparison with normal cells. In addition to proteins and glycoproteins, tumor-specific patterns of expression of carbohydrates, gangliosides, glycolipids and mucins have also been documented. Moingeon, *supra.* Exemplary tumor-associated antigens include protein products of oncogenes, tumor suppressor genes and other genes with mutations or rearrangements unique to tumor cells, reactivated embryonic gene products, oncofetal antigens, tissue-specific (but not tumor-specific) differentiation antigens, growth factor receptors, cell surface carbohydrate residues, foreign viral proteins and a number of other self proteins.

Tumor-associated antigens include, e.g., mutated antigens such as the protein products of the Ras p21 protooncogenes, tumor suppressor p53 and HER- 2/neu and BCR-abl oncogenes, as well as CDK4, MUM1, Caspase 8, and Beta catenin; overexpressed antigens such as galectin 4, galectin 9, carbonic anhydrase, Aldolase A, PRAME, Her2/neu, ErbB-2 and KSA, oncofetal antigens such as alpha fetoprotein (AFP), human chorionic gonadotropin (hCG); self antigens such as carcinoembryonic antigen (CEA) and melanocyte differentiation antigens such as Mart 1/Melan A, gp100, gp75, Tyrosinase, TRP1 and TRP2; prostate associated antigens such as PSA, PAP, PSMA, PSM-P1 and PSM- P2; reactivated embryonic gene products such as MAGE 1, MAGE 3, MAGE 4, GAGE I, GAGE 2, BAGE, RAGE, and other cancer testis antigens such as NY-ESO1, SSX2 and SCP1; mucins such as Muc-1 and Muc-2; gangliosides such as GM2, GD2 and GD3, neutral glycolipids and glycoproteins such as Lewis (y) and globo-H; and glycoproteins such as Tn, Thompson- Freidenreich antigen (TF) and sTn. Also included as tumor-associated antigens herein are whole cell and tumor cell lysates as well as immunogenic portions thereof, as well as immunoglobulin idiotypes expressed on monoclonal proliferations of B lymphocytes for use against B cell lymphomas.

Tumor-associated antigens can be prepared by methods known in the art. For example, these antigens can be prepared from cancer cells either by preparing crude extracts of cancer cells (e.g., as described in Cohen et al., Cancer Res., 54: 1055 (1994)), by partially purifying the antigens, by recombinant technology, or by de novo synthesis of known antigens. The antigen may also be in the form of a nucleic acid encoding an antigenic peptide in a form suitable for expression in a subject and presentation to the immune system of the immunized subject. Further, the antigen may be a complete antigen, or it may be a fragment of a complete antigen comprising at least one epitope.

Pathogens include, but are not limited to, infectious virus that infect mammals, and more particularly humans. Examples of infectious virus include, but are not limited to: Retroviridae (e.g. human immunodeficiency viruses, such as HIV-1 (also referred to as HTLV- III, LAV or HTLV-III/LAV, or HIV-III; and other isolates, such as HIV-LP; Picornaviridae (e.g. polio viruses, hepatitis A virus; enteroviruses, human Coxsackie viruses, rhinoviruses, echoviruses); Calciviridae (e.g. strains that cause gastroenteritis); Togaviridae (e.g. equine encephalitis viruses, rubella viruses); Flaviridae (e.g. dengue viruses, encephalitis viruses, yellow fever viruses); Coronoviridae (e.g. coronaviruses); Rhabdoviradae (e.g. vesicular stomatitis viruses, rabies viruses); Coronaviridae (e.g. coronaviruses); Rhabdoviridae (e.g. vesicular stomatitis viruses, rabies viruses); Filoviridae (e.g. ebola viruses); Paramyxoviridae (e.g. parainfluenza viruses, mumps virus, measles virus, respiratory syncytial virus); Orthomyxoviridae (e.g. influenza viruses); Bungaviridae (e.g. Hantaan viruses, bunga viruses, phleboviruses and Nairo viruses); Arena viridae (hemorrhagic fever viruses); Reoviridae (e.g. reoviruses, orbiviurses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae herpes simplex virus (HSV) 1 and 2, varicella zoster virus, cytomegalovirus (CMV), herpes virus; Poxviridae (variola viruses, vaccinia viruses, pox viruses); and Iridoviridae (e.g. African swine fever virus); and unclassified viruses (e.g. the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e. Hepatitis C); Norwalk and related viruses, and astroviruses).

Also, gram negative and gram positive bacteria serve as antigens in vertebrate animals. Such gram positive bacteria include, but are not limited to Pasteurella species, Staphylococci species, and Streptococcus species. Gram negative bacteria include, but are not limited to, Escherichia coli, Pseudomonas species, and Salmonella species. Specific examples of infectious bacteria include but are not limited to: Helicobacterpyloris, Borelia burgdorferi, Legionella pneumophilia, Mycobacteria sps (e.g. M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcusfaecalis, Streptococcus bovis, Streptococcus (anaerobic sps.), Streptococcus pneumoniae, pathogenic Campylobacter sp., Enterococcus sp., Haemophilus infuenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, Rickettsia, and Actinomyces israelli.

Examples of pathogens include, but are not limited to, infectious fungi that infect mammals, and more particularly humans. Examples of infectious fingi include, but are not limited to: Cryptococcus neoformans, Histoplasma capsulatum, Coccidioides immitis, Blastomyces dermatitidis, Chlamydia trachomatis, Candida albicans. Examples of infectious parasites include Plasmodium such as Plasmodium falciparum, Plasmodium malariae, Plasmodium ovale, and Plasmodium vivax. Other infectious organisms (i.e. protists) include Toxoplasma gondii.

Other medically relevant microorganisms that serve as antigens in mammals and more particularly humans are described extensively in the literature, e.g., see C. G. A Thomas, Medical Microbiology, Bailliere Tindall, Great Britain 1983, the entire contents of which is hereby incorporated by reference. In addition to the treatment of infectious human diseases, the compositions and methods of the present invention are useful for treating infections of nonhuman mammals. Specific examples of pathogens and antigens

In preferred embodiments, "treatment", "treat", "treating" as used herein with reference to infectious pathogens, refers to a prophylactic treatment which increases the resistance of a subject to infection with a pathogen or decreases the likelihood that the subject will become infected with the pathogen; and/or treatment after the subject has become infected in order to fight the infection, e.g., reduce or eliminate the infection or prevent it from becoming worse. Many vaccines for the treatment of non-human mammals are disclosed in Bennett, K. Compendium of Veterinary Products, 3rd ed. North American Compendiums, Inc., 1995. As discussed above, antigens include infectious microbes such as virus, bacteria, parasites and fungi and fragments thereof, derived from natural sources or synthetically. Infectious virus of both human and non-human mammals, include retroviruses, RNA viruses, and DNA viruses. This group of retroviruses includes both simple retroviruses and complex retroviruses. The simple retroviruses include the subgroups of B-type retroviruses, C-type retroviruses and D-type retroviruses. An example of a B-type retrovirus is mouse mammary tumor virus ("MMTV"). The C-type retroviruses include subgroups C-type group A (including Rous sarcoma virus ("RSV"), avian leukemia virus ("ALV"), and avian myeloblastosis virus ("AMV")) and C-type group B (including murine leukemia virus ("MLV"), feline leukemia virus ("FeLV"), murine sarcoma virus ("MSV"), gibbon ape leukemia virus ("GALV"), spleen necrosis virus ("SNV"), reticuloendotheliosis virus ("RV") and simian sarcoma virus ("SSV"). The D-type retroviruses include Mason-Pfizer monkey virus ("MPMV") and simian retrovirus type 1 ("SRV-1"). The complex retroviruses include the subgroups of lentiviruses, T-cell leukemia viruses and the foamy viruses. Lentiviruses include HIV-1, but also include HIV-2, SIV, Visna virus, feline immunodeficiency virus ("FIV"), and equine infectious anemia virus ("EIAV'). The T-cell leukemia viruses include HTLV-1, HTLV-II, simian T-cell leukemia virus ("STLV"), and bovine leukemia virus ("BLV"). The foamy viruses include human foamy virus ("HFV"), simian foamy virus ("SFV") and bovine foamy virus ("BFV").

Polypeptides of bacterial pathogens include but are not limited to an iron-regulated outer membrane protein, ("IROMP"), an outer membrane protein ("OMP"), and an A-protein of Aeromonis salmonicida which causes furunculosis, p57 protein of Renibacterium salmoninarum which causes bacterial kidney disease ("BKD"), major surface associated antigen ("msa"), a surface expressed cytotoxin ("mpr"), a surface expressed hemolysin ("ish"), and a flagellar antigen of Yersiniosis; an extracellular protein ("ECP"), an iron-regulated outer membrane protein ("IROMP"), and a structural protein of Pasteurellosis; an OMP and a flagellar protein of Vibrosis anguillarum and V. ordalii; a flagellar protein, an OMP protein, aroA, and purA of Edwardsiellosis ictaluri and E. tarda; and surface antigen of Ichthyophthirius; and a structural and regulatory protein of Cytophaga columnari; and a structural and regulatory protein of Rickettsia.

Polypeptides of a parasitic pathogen include but are not limited to the surface antigens of Ichthyophthirius. An "allergen" refers to a substance (antigen) that can induce an allergic or asthmatic response in a susceptible subject. The list of allergens is enormous and can include pollens, insect venoms, animal dander dust, fungal spores and drugs (e.g. penicillin). Examples of natural, animal and plant allergens include but are not limited to proteins specific to the following genuses: Canine (Canis familiaris); Dermatophagoides (e.g. Dermatophagoides farinae); Felis (Felis domesticus); Ambrosia (Ambrosia artemiisfolia; Lolium (e.g. Lolium perenne or Lolium multiflorum); Cryptomeria (Cryptomeria japonica); Alternaria (Alternaria alternata); Alder; Alnus (Alnus gultinoasa); Betula (Betula verrucosa); Quercus (Quercus alba); Olea (Olea europa); Artemisia (Artemisia vulgaris); Plantago (e.g. Plantago lanceolata); Parietaria (e.g. Parietaria officinalis or Parietaria judaica); Blattella (e.g. Blattella germanica); Apis (e.g. Apis multiflorum); Cupressus (e.g. Cupressus sempervirens, Cupressus arizonica and Cupressus macrocarpa); Juniperus (e.g. Juniperus sabinoides, Juniperus virginiana, Juniperus communis and Juniperus ashei); Thuya (e.g. Thuya orientalis); Chamaecyparis (e.g. Chamaecyparis obtusa); Periplaneta (e.g. Periplaneta americana); Agropyron (e.g. Agropyron repens); Secale (e.g. Secale cereale); Triticum (e.g. Triticum aestivum); Dactylis (e.g. Dactylis glomerata); Festuca (e.g. Festuca elatior); Poa (e.g. Poa pratensis or Poa compressa); Avena (e.g. Avena sativa); Holcus (e.g. Holcus lanatus); Anthoxanthum (e.g. Anthoxanthum odoratum); Arrhenatherum (e.g. PArrhenatherum elatius); Agrostis (e.g. Agrostis alba); Phleum (e.g. Phleum pratense); Phalaris (e.g. Phalaris arundinacea); Paspalum (e.g. Paspalum notatum); Sorghum (e.g. Sorghum halepensis); and Bromus (e.g. Bromus inermis).

**Other Drug Components**

Some preferred embodiments of the invention further comprise other therapeutic agents, e.g., drugs or bioactive agents. These additional components may provide direct additional therapeutic benefit or additional immune-stimulating benefits. A wide variety of therapeutic compounds may be delivered by the compositions and methods of the present invention. Examples of therapeutic compounds include, but are not limited to, nucleic acids, proteins, peptides, oncolytics, anti-infectives, anxiolytics, psychotropics, immunomodulators, ionotropes, toxins such as gelonin and inhibitors of eucaryotic protein synthesis, and the like. Preferred therapeutic compounds for entrapment in the liposomes of the present invention are those which are lipophilic cations. Among these are therapeutic agents of the class of lipophilic molecules which are able to partition into a lipid bilayer phase of a liposome, and which therefore are able to associate with the liposomes in a membrane form. Further examples of therapeutic compounds include, but are not limited to, prostaglandins, amphotericin B, methotrexate, cisplatin and derivatives, progesterone, testosterone, estradiol, doxorubicin, epirubicin, beclomethasone and esters, vitamin E, cortisone, dexamethasone and esters, betamethasone valerete and other steroids, the fluorinated quinolone antibacterial ciprofloxacin and its derivatives, and alkaloid compounds and their derivatives. Among the alkaloid derivatives are swainsonine and members of the vinca alkaloids and their semisynthetic derivatives, such as, e.g., vinblastine, vincristine, vindesin, etoposide, etoposide phosphate, and teniposide. Among this group, vinblastine and vincristine, and swainsonine are particularly preferred. Swainsonine (Creaven and Mihich, Semin. Oncol. 4:147 (1977) has the capacity to stimulate bone marrow proliferation (White and Olden, Cancer Commun. 3:83 (1991)). Swainsonine also stimulates the production of multiple cytokines including IL-1, IL-2. TNF, GM-CSF and interferons (Newton, Cancer Commun. 1:373 (1989); Olden, K., J. Natl. Cancer Inst., 83:1149 (1991)). Further Swainsonine reportedly induces B- and T-cell immunity, natural killer T-cell and macrophage-induced destruction of tumor cells *in vitro,* and when combined with interferon, has direct anti-tumor activity against colon cancer and melanoma cancers *in vivo* (Dennis, J., Cancer Res., 50:1867 (1990); Olden, K., Pharm. Ther. 44:85 (1989); White and Olden, Anticancer Res., 10:1515 (1990)). Other alkaloids useful in the compositions and methods of the present invention include, but are not limited to, paclitaxel (taxol) and synthetic derivatives thereof. Additional drug components, include but are not limited to, any bioactive agents known in the art which can be incorporated into lipid particles.

These additional drug components may be encapsulated or otherwise associated the lipid particles described herein. Alternatively, the compositions of the invention may include drugs or bioactive agents that are not associated with the lipid-nucleic acid particle. Such drugs or bioactive agents may be in separate lipid carriers or co-administered.

**Manufacturing of Compositions**

Manufacturing the compositions of the invention may be accomplished by any technique, but most preferred are the ethanol dialysis or detergent dialysis methods detailed in the following publications, patents, and applications. U.S. Pat. Ser. No. 5,705,385; U.S. Pat. No. 5,976,567; U.S. Pat. Appln. No. 09/140,476; U.S. Pat. No. 5,981,501; U.S. Pat. No. 6,287,591; Int. Publ. No. WO 96/40964; and Int. Publ. No. WO 98/51278. These manufacturing methods provide for small and large scale manufacturing of immunostimulatory compositions comprising therapeutic agents encapsulated in a lipid particle, preferably lipid-nucleic acid particles. The methods also generate such particles with excellent pharmaceutical characteristics.

Vaccine compositions of the present invention may be prepared by adding a target antigen (to which the immune response is desired). Means of incorporating antigens are well known in the art and include, for example: 1) passive encapsulation of the antigen during the formulation process (e.g., the antigen can be added to the solution containing the ODN); 2) addition of glycolipids and other antigenic lipids to an ethanol lipid mixture and formulated using the ethanol-based protocols described herein; 3) insertion into the lipid vesicle (e.g., antigen- lipid can be added into formed lipid vesicles by incubating the vesicles with antigen-lipid micelles); and 4) the antigen can be added post-formulation (e.g., coupling in which a lipid with a linker moiety is included into formulated particle, and the linker is activated post formulation to couple a desired antigen). Standard coupling and cross-linking methodologies are well known in the art. An alternative preparation incorporates the antigen into a lipid-particle which does not contain a nucleic acid, and these particles are mixed with lipid-nucleic acid particles prior to administration to the subject.

**Characterization of Compositions Used in the Present Invention**

Preferred characteristics of the compositions used in the present invention are as follows.

The preferred lipid-nucleic acid particles useful in the invention comprise a lipid membrane (generally a phospholipid bilayer) exterior which fully encapsulates an interior space. These particles, also sometimes herein called lipid membrane vesicles, are small particles with mean diameter 50-200 nm, preferably 60-130 nm. Most preferred for intravenous administrations are particles of a relatively uniform size wherein 95% of particles are within 30 nm of the mean. The nucleic acid and other bioactive agents are contained in the interior space, or associated with an interior surface of the encapsulating membrane.

"Fully encapsulated" as used herein indicates that the nucleic acid in the particles is not significantly degraded after exposure to serum or a nuclease assay that would significantly degrade free DNA. In a fully encapsulated system, preferably less than 25% of particle nucleic acid is degraded in a treatment that would normally degrade 100% of free nucleic acid, more preferably less than 10% and most preferably less than 5% of the particle nucleic acid is degraded. Alternatively, full encapsulation may be determined by an Oligreen™ assay. Fully encapsulated also suggests that the particles are serum stable, that is, that they do not rapidly decompose into their component parts upon *in vivo* administration.

These characteristics of the compositions of the present invention distinguish the preferred particles of the invention from lipid-nucleic acid aggregates (also known as cationic complexes or lipoplexes) such as DOTMA/DOPE (LIPOFECTIN™) formulations. These aggregates are generally much larger (>250 nm) diameter, they do not competently withstand nuclease digestion. They generally decompose upon *in vivo* administration. Lipid-nucleic acid formulations comprising cationic lipid-nucleic acid aggregates with weak antigens, as described above, may provide suitable vaccines for local and regional applications, such as intramuscular, intra-peritoneal and intrathecal administrations, and more preferably intranasal administration.

The liposomal particles of the invention can be formulated at a wide range of drug:lipid ratios. "Drug to lipid ratio" as used herein refers to the amount of therapeutic nucleic acid (*i.e.*, the amount of nucleic acid which is encapsulated and which will not be rapidly degraded upon exposure to the blood) in a defined volume of preparation divided by the amount of lipid in the same volume. This may be determined on a mole per mole basis or on a weight per weight basis, or on a weight per mole basis. Drug to lipid ratio may determine the lipid dose that is associated with a given dose of nucleic acid. In a preferred embodiment, the compositions of the present invention have a drug:lipid ratio in the range of about 0.01 to 0.25 (wt/wt).

**Uses of the Compositions and Methods of the Present Invention**

The combination of B lymphocytes and T lymphocytes establish the underlying operation of the humoral and cellular immune responses, respectively. The humoral and cellular immune responses each proceed by activation of their respective cell types in response to stimulation from an antigen and the consequent secretions of various cytokines. The presentation of antigenic peptide to naïve CD4+ T helper cells causes the cells to differentiate into two distinct subsets of helper cells (Th-1 and Th-2) which can be distinguished by their function and cytokine expression profiles.

Mosman et al., Annu. Rev. Immunol., 7:145-173 (1989); Paul et al., Cell, 76: 241-251 (1994); O'Garra, Immunity, 8: 275-283 (1998).

The specific patterns of cytokines secreted by the CD4+ Th cells steer the immune response to a predominantly cellular, type-1 response (including IFN-γ, IL-1, IL-2, IL-12, and TNF-α) or a mainly humoral, type-2 response (including IL-4, IL-5, IL-6, IL-9, IL-10 and IL-13). Glimcher and Murphy, Genes Dev., 14:1693-1711 (2000); Abbas et al., Nature, 383:787-793 (1996). The Th-1 subset promotes both cell-mediated immunity through activation of CTL and NK cells, as well as humoral immunity characterized by immunoglobulin class switching from IgM to IgG and IgA in humans, and to IgG2a in mice. Th-1 responses may also be associated with delayed-type hypersensitivity and autoimmune disease. The Th-2 subset induces primarily humoral immunity and induces class switching to IgG1 and IgE in humans. The antibody isotypes associated with Th-1 responses generally have good neutralizing and opsonizing capabilities whereas those associated with Th-2 responses are generally more associated with allergic responses.

As demonstrated herein, the subject immunostimulatory compositions are capable of stimulating a strong, Th-1 biased immune response to antigenic stimulation, e.g., microbial antigens and tumor-associated antigens, and can enhance both the cellular and the humoral components of the host immune response. Thus, the immunostimulatory compositions described herein find use as adjuvants in methods of inducing Th-1 biased immune responses in general, and in vaccines directed to specific antigen (s) of interest. Also provided herein are methods for improving the maturation of the humoral response as well as methods for increasing antibody isotype switching in response to antigenic stimulation.

These immune responses can be measured in many ways including but not limited to activation, proliferation or differentiation of cells of the immune system (e.g., B cells, T cells, APCs, such as dendritic cells or macrophages, NK cells, NKT cells etc.); up-regulated or down- regulated expression of markers; cytokine secretion; stimulation of or increase in IgA, IgM, or IgG titer; isotype class switching, and splenomegaly (including increased spleen cellularity). The presence of a Th-1 biased immune response in particular can be determined directly by the induction of Th-1 cytokines (e. g., IFN-y,IL-12) and antigen-specific CD8+ CTL. Thus, if Th-1 cytokines or CTL are induced, Th-1 biased immune responses are induced according to the invention. Similarly, enhanced humoral responses and improvements in the maturation of the humoral response are indicated by detecting the isotype of type-1 antigen-specific antibodies that are induced (e.g., IgG2a, IgG1 in mice, IgG and IgA in humans), and determining if isotype switching has occurred, e.g., IgM to IgG or IgA, as exemplified herein. If increased isotype switching has occurred in comparison with alternative adjuvants, enhanced humoral immune responses are induced according to the invention.

In a preferred embodiment, the present invention comprises the use of an effective amount of an immunostimulatory composition comprising an LNA formulation including a methylated nucleic acid in the manufacture of a medicament for stimulating a Th1-baised immune response to antigenic stimulation. Vaccines are also provided comprising such LNA formulations in combination with one or more epitopes of one or more antigens of interest. Preferably the antigen is associated with the LNA particle, and most preferably a plurality of antigens are employed. In one embodiment, the ODN comprises a PS or other modified, non-phosphodiester backbone. Alternative adjuvants that induceTh1 responses include but are not limited to MPL, MDP, ISCOMS, IL-12, IFN-y, and SB-AS2.

In a further embodiment, the compositions and methods of the present invention can be used to modulate the level of a cytokine. "Modulate" as used herein with reference to a cytokine may refer to the suppression of expression of a particular cytokine when lower levels are desired, or augmentation of the expression of a particular cytokine when higher levels are desired. Modulation of a particular cytokine can occur locally or systemically. In a preferred embodiment, the compositions and methods of the present invention can be used to activate macrophages and dendritic cells to secrete cytokines. In general, Th1-type cytokines can be induced, and thus the immunostimulatory compositions of the present invention can promote a Th1 type antigen-specific immune response including cytotoxic T-cells.

**Indications, Administration and Dosages**

The compositions and methods of the present invention are indicated for use in any patient or organism having a need for immune system stimulation. Such a need encompasses, but is not limited to, most medical fields, such as oncology, inflammation, arthritis & rheumatology, immuno-deficiency disorders. One skilled in the art can select appropriate indications to test for efficacy based on the disclosure herein. In a preferred embodiment, the compositions and methods of the invention are used to treat a neoplasia (any neoplastic cell growth which is pathological or potentially pathological) such as the neoplasia described in the Examples below.

Administration of the compositions of the invention to a subject may be by any method including *in vivo* or ex vivo methods. *In vivo* methods can include local, regional or systemic applications. In a preferred embodiment, the compositions are administered intravenously such that particles are accessible to B cells, macrophages or a splenocytes in a patient, and/or the particle can stimulate lymphocyte proliferation, resulting in secretion of IL-6, IL-12, IFNg and/or IgM in said patient.

Vaccine compositions of the present invention may be administered by any known route of administration. In one embodiment, the compositions of the present invention are administered via intravenous injection. In another embodiment, intramuscular or subcutaneous injection is employed and in this manner larger-sized (150-300 nm) lipid particles can be used. Consequently, the need for costly extrusion steps can be reduced or eliminated, and since the particles do not need to circulate, the selection of lipid components can be biased in favor of less expensive materials. For example, the amount of Chol can be reduced, DSPC can be replaced with something less rigid (e.g., POPC or DMPC), and PEG-lipids can be replaced with less expensive PEG-acyl chains. In a still further embodiment, the compositions of the present invention are administered via the respiratory tract, e.g., by intratracheal instillation or intranasal inhalation.

One skilled in the art would know how to identify possible toxicities of formulations, for example, complement activation, coagulation, renal toxicities, liver enzyme assays, etc. Such toxicities may differ between organisms.

Pharmaceutical preparations of compositions usually employ additional carriers to improve or assist the delivery modality. Typically, compositions of the invention will be administered in a physiologically-acceptable carrier such as normal saline or phosphate buffer selected in accordance with standard pharmaceutical practice. Other suitable carriers include water, 0.9% saline, 0.3% glycine, and the like, including glycoproteins for enhanced stability, such as albumin, lipoprotein, globulin, etc.

Dosages of lipid-nucleic acid formulations depend on the desired lipid dosage, the desired nucleic acid dosage, and the drug:lipid ratio of the composition. One skilled in the art can select proper dosages based on the information provided herein.

Immunotherapy or vaccination protocols for priming, boosting, and maintenance of immunity are well known in the art and further described below. In particular, one skilled in the art would know how to calculate dosage amounts for a subject, particularly a mammal, and more particularly a human, based on the dosage amounts described herein. Specific conversion factors for converting dosage amounts from one animal to another (e.g., from mouse to human) are well known in the art and are fully described, *e.g.*, on the Food and Drug Administration Web site at: www.fda.gov/cder/cancer/animalframe.htm (in the oncology tools section). As compared to known immunostimulatory compositions having free nucleic acids, the immunostimulatory compositions and methods of the present invention may utilize reduced amounts of nucleic acids to stimulate enhanced immune responses *in vivo.*

The amount of nucleic acids in the formulations of the present invention will generally vary between about 0.001-60 mg/kg (mg nucleic acids per kg body weight of a mouse per dose). In preferred embodiments for intravenous (i.v.) administration, the compositions and methods of the present invention utilize about 1-50 mg/kg, more preferably about 5-20 mg/kg. In preferred embodiments for subcutaneous (s.c.) administration, the compositions and methods of the present invention utilize about 1-10 mg/kg, and more preferably about 1-5 mg/kg, usually about about 3-5 mg/kg. The amount of antigen associated with the lipid particles of the present invention is preferably about 0.04-40 mg/kg, and more preferably about 0.04-4 mg/kg. As described above, one skilled in the art could readily determine suitable dosage amounts for other mammals given the dosage amounts described herein, based on the well-known conversion factors identified above and further empirical testing.

The formulations of the invention may be administered in pharmaceutically acceptable solutions, which may routinely contain pharmaceutically acceptable concentrations of salt, buffering agents, preservatives, compatible carriers, adjuvants, and optionally other therapeutic ingredients.

For use in therapy, an effective amount of the immunostimulatory compositions of the present invention can be administered to a subject by any mode allowing uptake by the appropriate target cells. "Administering" the immunostimulatory composition of the present invention may be accomplished by any means known to the skilled artisan. Preferred routes of administration include but are not limited to parenteral injection (subcutaneous, intradermal, intravenous, parenteral, intraperitoneal, intrathecal, etc.), as well as mucosal, intranasal, intratracheal, inhalation, and intrarectal, intravaginal; or oral, transdermal (e.g., via a patch). An injection may be in a bolus or a continuous infusion.

For example, the immunostimulatory compositions of the present invention can be administered by intramuscular or intradermal injection, or other parenteral means, or by biolistic "gene-gun" application to the epidermis. The immunostimulatory compositions of the present invention may also be administered, for example, by inhalation, topically, intravenously, orally, implantation, rectally, or vaginally. Suitable liquid or solid pharmaceutical preparation forms are, for example, aqueous or saline solutions for injection or inhalation, encochleated, coated onto microscopic gold particles, and nebulized. For a brief review of present methods for drug delivery, see Langer, Science 249:1527-1533, 1990.

The pharmaceutical compositions are preferably prepared and administered in dose units. Liquid dose units are vials or ampoules for injection or other parenteral administration. Solid dose units are tablets, capsules and suppositories. For treatment of a patient, depending on activity of the compound, manner of administration, purpose of the immunization (i.e., prophylactic or therapeutic), nature and severity of the disorder, age and body weight of the patient, different doses may be necessary. The administration of a given dose can be carried out both by single administration in the form of an individual dose unit or else several smaller dose units. Multiple administration of doses at specific intervals of weeks or months apart is usual for boosting the antigen-specific responses.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium

chloride (0.003-0.03% w/v); chlorobutanol (0.3-0.9% w/v); parabens (0.01-0.25% w/v) and thimerosal (0.004-0.02% w/v).

In preferred embodiments, the immunostimulatory compositions of the present invention contain an effective amount of a combination of adjuvants and antigens optionally included in a pharmaceutically-acceptable carrier. "Pharmaceutically-acceptable carrier" as used herein refers to one or more compatible solid or liquid filler, dilutants or encapsulating substances which are suitable for administration to a human or other mammal. "Carrier" as used herein refers to an organic or inorganic ingredient, natural or synthetic, with which the active ingredient is combined to facilitate the application. The components of the immunostimulatory compositions of the present invention also are capable of being comingled with the compounds of the present invention, and with each other, in a manner such that there is no interaction which would substantially impair the desired pharmaceutical efficiency.

Compositions suitable for parenteral administration conveniently comprise sterile aqueous preparations, which can be isotonic with the blood of the recipient. Among the acceptable vehicles and solvents are water, Ringer's solution, phosphate buffered saline and isotonic sodium chloride solution. In addition, sterile, fixed oils are conventionally employed as a solvent or suspending medium. For this purpose any bland fixed mineral or non-mineral oil may be employed including synthetic mono-ordiglycerides. In addition, fatty acids such as oleic acid find use in the preparation of injectables. Carrier formulations suitable for subcutaneous, intramuscular, intraperitoneal, intravenous, etc. administrations may be found in Remington's Pharmaceutical Sciences, Mack Publishing Company, Easton, Pa.

The adjuvants or antigens useful in the invention may be delivered in mixtures of more than two adjuvants or antigens. A mixture may consist of several adjuvants in addition to the LNA formulations described herein.

A variety of administration routes are available. The particular mode selected will depend, of course, upon the particular adjuvants or antigen selected, the age and general health status of the subject, the particular condition being treated and the dosage required for therapeutic efficacy. Any mode of administration that is medically acceptable may be used, meaning any mode that produces effective levels of an immune response without causing clinically unacceptable adverse effects. Preferred modes of administration are discussed above.

The compositions may conveniently be presented in unit dosage form and may be prepared by any of the methods well known in the art of pharmacy. All methods include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product.

Other delivery systems can include time-release, delayed release or sustained release delivery systems. Such systems can avoid repeated administrations of the compounds, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109. Delivery systems also include non-polymer systems that are: lipids including sterols such as cholesterol, cholesterol esters and fatty acids or neutral fats such as mono-di-and triglycerides; hydrogel release systems; sylastic systems; peptide based systems; wax coatings; compressed tablets using conventional binders and excipients; partially fused implants; and the like. Specific examples include, but are not limited to: (a) erosional systems in which an agent of the invention is contained in a form within amatrix such as those described in U.S. Pat. Nos. 4,452,775, 4,675,189, and 5,736,152, and (b) diffusional systems in which an active component permeates at a controlled rate from a polymer such as described in U.S. Pat. Nos. 3,854,480, 5,133,974 and 5,407,686. In addition, pump-based hardware delivery systems can be used, some of which are adapted for implantation.

Turning now to certain particular aspects of the present invention, one aspect arises from the recognition that *in vitro* data showing effects of oligonucleotides on stimulation of various leukocyte markers, including activation markers, are not determinative of whether a free oligonucleotide is, in-fact, immunostimulatory *in vivo* or will have therapeutic efficacy.

## EXPERIMENTAL

### Experimental Details

Mice. Female, Balb/c or C57/BL6 ("B6") mice (6-8 weeks) were purchased from Harlan-Sprague Dawley (Indianapolis, IN). All animals were quarantined for one week prior to use. All studies were conducted in accordance with the guidelines established by the Canadian Council on Animal Care (CCAC) and the Institutional Animal Care and User Committee (IACUC).

Peptides. Peptides were obtained from Commonwealth Biotechnologies and were >95% pure as determined by HPLC. QC analyses were obtained with each peptide.

Oligonucleotide formulations. Oligonucleotides were obtained from Avecia or Proligo or Trilink. All ODN were rehydrated in sterile water and diluted to the appropriate concentrations in sterile DPBS, pH 7.2.

LNA formulations. LNA formulations were prepared as follows. Initial ODN:lipid ratios were 0.25, w/w. For phosphodiester formulations, 20 mM citrate buffer was used in place of 300 mM citrate buffer to dissolve the ODN. Failure to do this results in considerably diminished encapsulation efficiency (i.e. - 3% final).

Vaccinations. B6 mice were vaccinated subcutaneously (SC) with 100 ml per injection. Dosing schedules were either q7dx2 or q4dx4 and are indicated in the individual figure legends. CFA mixtures were very viscous and had to be given with a larger gauge syringe. For humoral studies, a prime (day 0) and boost (day 14) strategy was used and blood samples were collected at various times by tail nicking.

Efficacy studies. E.G7-OVA or EL-4 thymoma cell lines were used throughout the study. These cells were cultured in vitro according to established methods. For tumor studies, 2.5 x 106 E.G7-OVA cells were injected subcutaneously in 50 ml of PBS containing 1% FCS. Tumor measurements were made by repeated perpendicular measurements of tumor dimensions and using the formula:

$$\text{Volume (mm3)} = (L \times W \times H)p/6$$

TRP-2 and gp100 studies were conducted using the B16/BL6 murine melanoma model. B16 cells (1.0x105) were injected IV in a volume of 200 ml. Typically, animals were vaccinated weekly for 2-3 injections and B16 cells were then administered 1-2 days after the final vaccination. Animals were terminated between days 14-18 post-B16 injection, lungs were removed, and metastases were counted using a stereomicroscope.

Flow cytometry analysis. Antigen specific T cells were determined in vaccinated mice at various times using either MHC tetramers (Beckman Coulter) or the Dimer X reagent (Pharmingen). Antibodies against CD8a, B220, and CD4 were used to identify cell populations of interest and gate out unwanted populations. Single cell suspensions of spleen and lymph nodes were prepared according to protocols outlined in Current Protocols in Immunology. Antibody stains were done on 1 x 106 cells in 96 well plates kept at 4˚C. Generally, 0.2 mg of each antibody was used per 1 x 106 cells.

## Example 1

### In vitro vs. in vivo Activation of Leukocytes in Whole Blood Cells by Exposure to Free or Encapsulated Oligonucleotides

In order to demonstrate the effectiveness of an in vitro assay for predicting immune stimulation in vivo a comparison of CD69 expression is shown in Figures 1 and 2. CD69 is a cell activation marker, which quantifies the activation of NK cells, B cells and monocytes. Expression of CD69 on NK cells indicates cell activation and production of IFN-g, which is important to inducing a Th-1 immune response. Free and encapsulated ODN1 and 2 were tested in vitro and in vivo for their ability to induce CD69 expression. A dose of 0.1 mg/ml of ODN2 and 10mg/ml ODN1 were used in vitro and 10mg/kg of ODN2 and 20mg/kg of ODN1 in vivo. Each oligonucleotide was encapsulated in a lipid particle composed of POPC:CHOL:DODMA:PEGDMG in a ratio of 20:45:25:10.

Figure 1 illustrates the in vitro stimulation of leukocytes bearing the activation marker CD69 from treating mouse whole blood with free oligonucleotides and encapsulated oligonucleoties, specifically ODN1 and 2. When mouse whole blood was treated in vitro with free oligonucleotides there was a dose responsive increase in the amount of CD69 positive B-cells, monocytes and to some extent, NK cells, according to the amount of free oligonucleotide used 15 hours after treatment. In this in vitro assay, free ODN2 caused much greater stimulation of CD69 than free ODN1. However, when these same oligonucleotides were encapsulated in a lipid vesicle, the in vitro stimulation of CD69 production on these same cell types was reduced or abolished altogether.

When the same oligonucleotides were tested in vivo, however, surprising results were obtained. Figure 2 illustrates that in vivo treatment of ICR mice by injection with encapsulated or free oligonucleotides produces results that are contrary to those obtained in vitro. This figure clearly demonstrates that in vivo, the lipid encapsulated oligonucleotides were more effective than the free oligonucleotides in stimulating the CD69 marker on the same cell types at 16 and 24 hours after injection. The results show that in vitro data is not sufficient for determining whether an oligonucleotide will be immunostimulatory in vivo. Moreover, Figures 1 and 2 suggest that lipid encapsulation is an important factor in determining whether an oligonucleotide would be effective in vivo. The in vivo results show that encapsulated ODN1 and ODN2 were both able to stimulate production of CD69 on NK cells, whereas the in vitro results indicated lipid encapsulation of ODN1 and 2 actually reduced the stimulation of CD69 on NK cells below the control level.

The foregoing in vivo results show that free oligonucleotides are not necessarily immunostimulatory unless they are encapsulated in a lipid vesicle as measured by stimulation of CD69 bearing cells in vivo. This is true even though stimulation of CD69 was observed in vitro by free ODN. Further, the results indicate that an encapsulated

oligonucleotide may be effective in vivo even though it is not shown to be effective in vitro.

**Example 2**

**In vivo Dendritic Cell Activation with Methylated Oligonucleotides**

As discussed in the Background section above, the prior art teaches that methylated CpG oligonucleotides are generally not effective, or less effective in comparison to unmethylated CpG oligonucleotides in stimulating immune responses whether measured in vitro or in vivo. United States Patent No. 6,429,199 discloses that methylated oligonucleotides did not enhance the expression of CD40 on NK cells or human B cells, nor did they show any improved survival of dendritic cells, which are the major antigen presenting cells involved in humoral and cellular immunity in a Th-1 response. Further, the methylated CpG oligonucleotides disclosed were inactive in improving survival, differentiation, activation or maturation of dendritic cells in vitro. Similarly, the in vitro PBMC results disclosed in WO 02/069369 did not demonstrate any activity of methylated oligonucleotides on dendritic cells.

In contrast, the present invention shows that methylated oligonucleotides are at least as effective and typically more effective at inducing proliferation of dendritic cells than unmethylated oligonucleotides. The counterpart of unmethylated ODN1 was made where the cytosine residue of the single CpG dinucleotide sequence was methylated and is referred to as ODN1m.

In order to demonstrate that methylated oligonucleotides are capable of stimulating dendritic cells each of ODN1 and ODN1m were encapsulated in a lipid vesicle comprising POPC:CHOL:DODMA:PEGDMG in a ratio of 20: 45:25:10. PBS was used as a control. The results of this experiment are shown in Figure 3.

Figure 3 clearly illustrates the ability of encapsulated ODN1 m to activate dendritic cells. Furthermore, when encapsulated in a lipid vesicle, methylated ODN1m was more active than its unmethylated counterpart ODN1 in stimulating activation of dendritic cells in vivo. Dendritic cell activation was measured by the percentage of IFN-g secreting cells. These cells were labeled with an antibody to indicate the cell type and only those having the dendritic cell marker were included in this measurement.

In order to demonstrate the expansion of dendritic cells resulting from the administration of lipid-encapsulated unmethylated and methylated CpG oligonucleotides, cells from the blood, spleen and lymph nodes were analyzed for activation and expansion of dendritic cell populations. ICR mice were immunized with a single intravenous injection of encapsulated oligonucleotides at a dose of 20 mg/kg and the control ICR mouse was injected with PBS. Cells for each of the spleen, blood and lymph nodes were isolated at various time points, as shown in Figures 4 and 5, and the amount of dendritic cell expansion and activation was measured through the use of dendritic cell markers CD11c and DEC205. Each of these markers are specific to dendritic cells though they may represent different cell sub-populations. In each of Figures 4 and 5 the control was plotted as equivalent to 100% and the effect of ODN1m for each backbone configuration was plotted as a percentage of that control. Figure 4A illustrates in each of the panels that the methylated ODN1m stimulated the expansion of CD11c positive dendritic cells in spleen cells and whole blood cells but not in lymphoid tissue as measured against the control. Both the PO and PS backbones for ODN1m showed dendritic cell expansion. Figure 4B shows in each of the panels that methylated ODN1m also stimulated the expansion of DEC205 positive dendritic cells in spleen cells and lymphoid tissue but not in whole blood cells as measured against the control.

Figure 5 also demonstrates the activation of dendritic cells. On collection of the samples the cells were first analyzed by flow cytometry for the co-expression of CD86, which indicates cell activation, and the dendritic cell phenotype markers CD11c and DEC205. The percentage of activated dendritic cells was plotted against a PBS control equivalent to 100%. Figure 5, each of the panels show that the methylated ODN1m induced CD86 expression on CD11c positive dendritic cells when the oligonucleotide was lipid encapsulated. Similar results are shown when measuring DEC205 positive dendritic cells in Figure 5 panels.

The data in Figures 4 and 5 therefore refutes the statements in United States Patent No. 6,429,199 which teaches that methylated CpG oligonucleotides are inactive in improving survival, differentiation, activation or maturation of dendritic cells.

**Example 3**

**The Effect of PS and PO Backbone Configurations on Plasma Cytokine Levels**

As noted above, with non-lipid encapsulated oligonucleotides the backbone is traditionally modified so as to reduce molecular degradation by nucleases. However, on encapsulation, such a modification is no longer required to prevent degradation. To establish the effect of a different phosphate backbone on cytokine stimulation induced by lipid encapsulation, mice were injected with oligonucleotides having both PO and PS backbones, and cytokine stimulation was measured over a series of points in time (as shown in Figure 6 and 7B) and over a sliding dosage scale as shown

in Figure 7A. In this experiment ICR mice were injected i.v. with a 20mg ODN/kg dose of free PO ODN1, encapsulated PO-ODN1 and encapsulated PS-ODN1. Cytokines common to both Th-1 and Th-2 (IL-12, IL-6 and IFN-g) and MCP-1 (a macrophage chemokine) were measured over a 24-hour time course following administration. Cytokine stimulation is generally indicative of a cellular immune response as is a chemokine response in MCP-1. Oligonucleotides were encapsulated in a lipid particle composed of DSPC:Chol:DODAP:PEG-CER14 in a ratio of 20:45:25:10.

Figure 6 shows that in vivo administration of free PO-ODN1 had no effect on stimulation of IL-6, IL-12 IFN-g or MCP-1 indicating that the oligonucleotide was likely degraded by nucleases. It is well known in the art that a PS backbone is required when administering free oligonucleotide in order to avoid nuclease degradation. In contrast, in vivo administration of lipid encapsulated PO and PS ODN1 stimulated production of each of these cytokines and chemokine. However, Figure 6 indicates that the PO-ODN is more effective at inducing cytokine and chemokine production.

Figure 7A illustrates increased IL-12 induction by treatment of ICR mice with either encapsulated PO or PS ODN14 in comparison to free ODN14 measured over a sliding dosage scale. This figure supports the conclusions drawn from Figure 6 indicating that lipid encapsulation increases the effectiveness of cytokine stimulation as evidenced by an increase in IL-12 induction and that a PO-ODN is more effective at inducing a cytokine response than a PS-ODN. In fact, when administered at the same dose but in encapsulated form a 2.5 fold increase in peak plasma IL-12 is observed, along with even more dramatic increases in other cytokines such as IL-6 (1000-fold) and IFN-$\gamma$ (20-fold). Figure 7A also demonstrates that a lower dose of oligonucleotides is required to facilitate a cytokine response when encapsulated in comparison with free olignonucleotides administered in the absence of the lipid particles.

In order to further elucidate the difference in PO-ODN and PS-ODN Figure 7B illustrates differences in IFN-g cytokine stimulation over time specific to PS and PO backbone configurations. Treatment with encapsulated PO ODN14 stimulates a strong early induction of IFN-gamma while treatment with encapsulated PS ODN14 stimulates a smaller but still effective induction of IFN-gamma. Moreover, Figure 7B shows that over a period of days, a second large IFN-g peak occurred when stimulating with the PS-ODN which may indicate that the immune system was primed by treatment with the encapsulated PS-ODN to respond more effectively to IL-12 production, possibly through expansion of NK cells after treatment.

Figure 22 similarly demonstrates the late IFN-g peak seen in Figure 7B for the PS-ODN in comparison with the PO version of the same oligonucleotides, as discussed further in Example 4.

An important feature of lipid encapsulation according to the present invention is the finding that oligonucleotides having natural PO backbones can be used to stimulate an immune response whereas in the prior art, PS backbones are required for effective in vivo activity. As shown in Example 7 below, encapsulated PO oligonucleotides may be more effective than PS oligonucleotides when evaluating anti-tumor efficacy, especially where the oligonucleotide is methylated.

**Example 4**

**Evaluation of immunostimulatory properties of CpG ODN having PS and PO backbones**

When differentiating the levels of response particularly associated with ODN's having PO and PS backbones, a further aspect is the analysis of the type of response being evaluated, more specifically, whether the response is a humoral response or a cellular response. In order to assess the effect of the backbones, an experiment was conducted to look at the ability of PS-ODN and PO-ODN to initiate and induce maturation (i.e. facilitate isotype switching) of a humoral immune response. The magnitude and kinetics of a humoral immune response elicited by administration of encapsulated PO-ODN and PS-ODN was compared. Each of the PO-ODN and PS-ODN were administered subcutaneously at a dose of 100 mg/dose in a q14 x 2 prime-boost setting on Days 0 and 14 and assessed at 6 weeks on Day 35. The control mice were immunized at the same dose using OVA-PBS and OVA-Alum. Oligonucleotides were encapsulated in a lipid particle composed of DSPC:Chol:DODMA:PEG-DMG in a ratio of 20:45:25:10.

It can be concluded that although both PO and PS ODNs are able to induce a humoral immune response, the nature of the response is different. Figures 8 and 9 illustrate the magnitude of the IgM and IgG response after 6 weeks for the various oligonucleotides and control in terms of absorbance. As is clearly demonstrated in the Figures, each of PS-ODN1 and PS-ODN2 produced a weak IgM response whereas PO-ODN2 produced a strong IgM response. Conversely, the IgG response produced was consistently better for each of the PS-ODN tested in comparison with the same oligonucleotides having a PO backbone. This suggests that a PS-ODN produces a superior IgG response. These data indicate that while both PO and PS ODN are able to initiate a humoral immune response, the PO response does not mature as indicated by a lack of isotype switching and a preponderance of the IgM isotype. On the other hand, PS-ODN are able to initiate a humoral immune response as well as induce maturation of the response as indicated by isotype switching to a dominance of IgG isotype antibodies.

This phenomenon may be related to the cytokine profiles induced by PO vs. PS ODN. Figure 7(b) and Figure 22 illustrate that encapsulated PS oligonucleotides ODN1 and ODN2 produced a strong IFN-g peak 6 days after treatment

that is not produced by encapsulated PO oligonucleotides. It has been reported that cytokines such as IFN-g result in preferential isotype switching to various IgG isotypes. Therefore, the large PS-ODN-induced late IFN-g peak may induce isotype switching from IgM to IgG isotypes while the lack of such a peak in PO-ODN-treated mice may result in no isotype switching. The basis for this reduced late IFN-g peak with PO-ODN is not clear, but results may suggest that treatment with encapsulated PO oligonucleotides but not PS oligonucleotides causes a prior induction of type I interferons that inhibit the expression of IL-12, which is needed to promote IFN-g expression in NK or T cells.

Similarly, Figure 6 not only shows that encapsulation of the oligonucleotides is important for stimulating the production of cytokines that lead to a Th-1 response as previously discussed, but also shows that more cytokines are produced using encapsulated PO oligonucleotides than PS oligonucleotides. This contrasts with administration of free oligonucleotides as taught in the prior art, which generally shows that a PS backbone is preferred over PO oligonucleotides to prevent degradation of the oligonucleotide in vivo.

### Example 5

### In vivo Immunological Responses to Treatment with Oligonucleotides as measured by Cytokine Induction, Tetramer Analysis and Cytotoxicity Assay (CTL)

In vivo Immunological Responses to Treatment with Oligonucleotides as measured by Cytokine Induction, Tetramer Analysis and Cytotoxicity Assay (CTL)

To monitor immunological response to subcutaneous immunization, antigen specific cellular immune responses were monitored using MHC Class I-tetramer analyses, cytotoxicity assays and cytokine release assays while humoral immune responses were monitored by measuring plasma antibody levels. Cellular and humoral responses were assessed in C57Bl/6 and Balb/C mice respectively (5 animals per group). For analysis of the cellular response, mice were immunized subcutaneously with 3 injections on a q7d x 3 dosing regimen on Days 0, 7 and 14 at a dose of 100 mg oligonucleotide in combination with 20mg of antigen. The spleen, liver, lymph node and blood tissues were collected on Day 21. Solid tissues were mechanically dissociated and cells were processed to collect mononuclear cells. For analysis of the humoral response, animals were immunized twice on a q14d x 2 on Days 0 and 14 and blood was collected on Day 35 for analysis of plasma for immunoglobulin levels. In this series of experiments oligonucleotides were encapsulated either in lipid particles composed of DSPC:Chol:DODMA:PEG-DMG or POPC:Chol:DODMA:PEG-DMG at a ratio of 20:45:25:10. All comparisons were done with like lipid particles.

MHC-tetramer analysis is designed to detect CD8+ve, cytotoxic T-lymphocytes that possess the appropriate T-cell receptor to allow recognition and lysis of target cells bearing the target antigen in the context of a MHC Class I complex. Isolated splenocytes from immunized animals were stained with PE-labeled MHC Class I tetramers (H2Kb) complexed with the immunodominant OVA SIINFEKL peptide as well as FITC-labeled anti-CD8 and Cy-Chrome-labeled anti-TCR antibodies and subjected to flow cytometric analysis. CD8 +ve, TCR+ve T-lymphocytes were assessed for the number of cells possessing T-cell receptors capable of specifically recognizing and binding to OVA in the context of MHC Class I molecules.

For the cytotoxicity assay, the ability of splenocytes from immunized animals to specifically recognize and lyse target cells in an antigen specific manner was assessed using a 4 hour 51 Chromium-release assay. Target cells were labeled with 51 Chromium and the amount of cytotoxicity was determined by the amount of radionuclide released into the supernatant from targets lysed by immune effector cells. Isolated splenocytes from immunized animals were tested immediately or after 5 days of in vitro restimulation with OVA-pulsed, syngeneic antigen presenting cells, for their ability to specifically lyse EG.7, OVA expressing target cells compared to EL4, non OVA-expressing cells.

The aim of the cytokine release assay is to detect antigen-specific immune effector cells that are activated to produce and secrete cytokines, specifically IFN-g, in response to stimulation with a specific antigen. Cells were isolated from the spleen, liver, blood and lymph nodes of immunized animals and analyzed using the Cytokine Secretion Assay (Miltenyi Biotec). Cells were stimulated overnight with OVA-pulsed, autologous antigen presenting cells and labeled with a catch reagent (a bispecific antibody recognizing the CD45 epitope on the surface of immune cells and IFN-g). Any cells capable of recognizing and responding to the antigen stimulation, synthesized and secreted cytokines which were then captured by the cell-bound catch reagent, resulting in IFN-g bound markers on their surface. Cells were then labeled with fluorescently labeled antibodies against IFN-g and various phenotype markers and analyzed by flow cytometry to allow detection of specific cell types that were activated to secrete IFN-g.

Analysis of humoral response was designed to determine the level of antigen-specific IgG in the plasma of immunized mice. Blood was collected by cardiac puncture and centrifuged to collect plasma. Antigen specific immunoglobulin production was measured using the End-point dilution ELISA method to measure titers of total IgM, IgG and the IgG1, IgG2a, subclasses. Samples of pooled plasma were serially diluted and plated into OVA coated plates to capture OVA specific antibodies in the diluted samples. OVA specific antibodies were then detected with horseradish peroxidase-conjugated rabbit anti-mouse IgM, IgG, IgG1, or IgG2a antibodies and TMB substrate. The absorbance of

the colorimetric reaction was measured at 450nm on ELISA plate reader and end-point dilution titers were defined as highest dilution of plasma that resulted in absorbance value two times greater then that of naïve animals, with a cut-off value of 0.05. This was used to evaluate seroconversion and magnitude of response as well as to evaluate the Th type of response.

Each of Figures 10 and 11 illustrate the normalization of ODN1m to that of its unmethylated counterpart ODN1. Each of the bars on these figures represents a direct comparison of one animal group (5 animals per group) treated with a methylated ODN and a second group treated with the unmethylated counterpart wherein each oligonucleotide is lipid encapsulated in identical lipid particles. The results for the unmethylated population were set equivalent to 100% for each group and the methylated group was measured against this 100% standard. On bars showing an equivalence to 200%, this was the cut off value and in actuality the 200% line represents a value of 200% or greater.

Figure 10 shows the results of the cytokine release assay described above. This figure illustrates that over a series of screenings, although both the methylated and unmethylated lipid encapsulated oligonucleotides each exhibited an immune response, on comparison of the methylated ODN to the unmethylated ODN, the methylated oligonucleotide was as good as, and often better than, the unmethylated ODN in stimulating proliferation of dendritic cells, NK cells, and CD8+ T-cells as indicated by cytokine secretion in Figure 10A, B, and C respectively.

The results of the tetramer and CTL analyses are shown in Figures 11A - C. These figures again illustrates the ability of both methylated and unmethylated ODN to stimulate an immune response. However, Figures 11A and B further demonstrate that over a series of screenings of animals treated with methylated or unmethylated encapsulated ODN, in each of the tetramer and CTL analyses respectively, the methylated oligonucleotide were consistently better in stimulating proliferation of cytotoxic T lymphocytes and tetrameric lymphocytes cells than the unmethylated ODN. In addition, Figure 11C illustrates data from a representative tetramer study, wherein overall averages are shown in Figure 11B. Each of ODN5, ODN5m, ODN7 and ODN7m were tested as per the protocol described above. It is clearly shown in Figure 11C that lipid encapsulated ODN5m and ODN7m induce a higher number of antigen specific CD8 T-cells on comparison to their lipid encapsulated unmethylated counterparts.

From each of Figures 10 and 11 it is shown that immune stimulation resulting from immunization with methylated ODN1m is consistently at least equivalent to, and often better than, the same treatment with its unmethylated oligonucleotide counterpart. This is further demonstrated in the following example.

### EXAMPLE 6

### Prophylactic Anti-Tumor Efficacy Comparison of Methylated and Unmethylated Oligonucleotides In an EG7-OVA Tumor Model

The cancer vaccines provided herein include lipid-nucleic acid formulations in conjunction with a tumor-associated antigen to stimulate an immune response to the antigen and the tumor *in vivo.* Hen egg albumin (ovalbumin; OVA) is a widely studied model antigen system. The antigenic determinants have been mapped and reagents and models exist to monitor both humoral and cell-mediated immune responses. In addition, cell lines containing the OVA gene have been established and characterized and have been used routinely to evaluate anti-tumor immune responses following vaccination. Specifically, E.G7-OVA is a murine thymoma cell line engineered to express the OVA protein as a xenogeneic tumor-associated antigen and is an accepted model for investigating the factors required to induce a host's immune system to specifically attack malignant cells in vivo. A vigorous Th1 cytokine response and the induction of antigen-specific CD8+ T lymphocytes are considered essential for mounting an effective anti-tumor immune reaction.

Anti-tumor efficacy induced by subcutaneous immunization was assessed in C57Bl/6 (5 animals per group) in a prophylactic immunization model. Mice were immunized subcutaneously with 3 injections on a q7d x 3 dosing regimen on Days 0, 7 and 14 at a dose of 100μg oligonucleotide and 20μg of OVA antigen dose. Animals were then challenged with a subcutaneous injection of 2.5 x $10^6$ EG.7 Ova expressing tumor cells on Day 21. Mice were monitored 3 times weekly to assess tumor growth and weight gain. Control mice were injected with one of PBS or HBS and 20μg of OVA antigen on the same schedule described above. Oligonucleotides were encapsulated in a lipid particle having a lipid composition of one of POPC: CHOL: DODAP:PEGCer14 or DSPC: CHOL: DODAP:PEGCer14 each in a ratio of 25: 45:20:10. All comparisons of methylated and unmethylated oligonucleotides were done using like lipid particles. Results from these efficacy experiments are detailed in Figures 12 - 15, 18 - 21, and 25 - 28. Day 0 on each of the Figures is the day each animal was challenged with the tumor.

Figure 12 illustrates the efficacy trend when animals are immunized with free ODN. The results shown are consistent with the prior art, namely that when an animal is administered free oligonucleotides, the methylated oligonucleotides have less therapeutic efficacy than the unmethylated oligonucleotides in reducing tumor growth. Specifically, free unmethylated ODN1 and ODN2, having PS backbones so as to avoid nuclease degradation, showed a greater reduction in tumor growth than their methylated counterparts, ODN1m and ODN2m. This was most especially true about 25 days after inoculation with the tumor when the tumor growth rate of the methylated oligonucleotides approached the

rate of the control animal treated only with a PBS buffer.

Figures 13-15 illustrate that encapsulation of oligonucleotides provides equivalent or better therapeutic efficacy of methylated over unmethylated oligonucleotides particularly when the oligonucleotides contain a natural phosphodiester (PO) backbone. Figure 13 shows that after implantation with a tumor, treatment with the methylated encapsulated ODN1m having a PS backbone was equal in therapeutic efficacy in comparison to the unmethylated ODN1. In contrast, Figure 14 shows the effect with the corresponding encapsulated methylated ODN1 m and unmethylated ODN1 oligonucleotides having a PO backbone, where therapeutic efficacy was greatest with the methylated version 32 days after transplantation while the unmethylated version lost its efficacy. Figure 15 shows that unmethylated ODN2 and its methylated counterpart ODN2m had virtually identical efficacy in reducing tumor growth. Accordingly, in certain embodiments the methylated oligonucleotide is at least as efficacious as an unmethylated counterpart when configured with a PS backbone.

Each of Figures 18, 19, 20 and 21 further elaborate on the above efficacy data. Figure 18 shows that lipid encapsulation of methylated PS-ODN1m provided a therapeutic benefit that was more effective than encapsulation of the PS-ODN1 in reducing tumor growth over a prolonged period of time. This effectiveness was further borne out by the superior survival rates of mice treated with encapsulated PS-ODN1 m in comparison to treatment with the PS-ODN1 in two different studies depicted in Figure 19. Figure 19A illustrates the percentage of animals that are tumor free at a series of time points and 19B, the number of animals remaining in the study at these same time points. As is clearly shown in Figure 19B, the number of animals remaining in the study treated with ODN1 and ODN1m was essentially identical throughout the study. However, Figure 19A clearly illustrates a greater percentage of tumor free animals when treated with the methylated ODN 1 m compared to those treated with unmethylated ODN1.

Similarly, Figure 20 illustrates the tumor volume in mice treated with the two oligonucleotides over time and Figure 21 the percentage of animals surviving over time. Figure 20 shows improved efficacy when animals were treated with the encapsulated methylated ODN1m in comparison to the encapsulated unmethylated counterpart, ODN1. Correspondingly, Figure 21 shows an increase in the survival rate of mice treated with the methylated ODN1m relative to treatment with unmethylated ODN1.

A further study efficacy study was conducted on the same tumor model using a different immunization protocol. In this study anti-tumor efficacy induced by subcutaneous immunization was assessed in C57Bl/6 (5 animals per group) in a prophylactic immunization model. Mice were immunized subcutaneously with 2 injections on a q7d x 2 dosing regimen on Days 0 and 7 at a dose of 100$\mu$g oligonucleotide and 20$\mu$g of antigen. Animals were then challenged with a subcutaneous injection of 5 x $10^5$ EG.7 Ova expressing tumor cells on Day 21. Mice were monitored 3 times weekly to assess tumor growth and weight gain. Control mice were injected with PBS on the same schedule described above. Oligonucleotides in Figure 24(b) were encapsulated in a lipid particle having a lipid composition of DSPC: CHOL: DODAP: PEGCer14 each in a ratio of 25:45:20:10. All comparisons of methylated and unmethylated oligos were done using like lipid particles. Results from these efficacy experiments are detailed in Figure 24. Day 0 on the Figure is the day each animal was challenged with the tumor.

Figure 24 illustrates an example of treating the experimental tumor E-G7 using the lipid encapsulated PS-ODN1, PS-ODN2, each unmethylated, PS-ODN1m, methylated, in conjunction with an E-G7 OVA tumor antigen, which in this case was associated with the lipid particle by being attached to the surface thereof. Figure 24A shows that when the oligonucleotides were administered in the absence of the immunostimulatory lipid particle, the methylated PS-ODN1m had little effect on tumor growth. The corresponding unmethylated oligonucleotide PS-ODN1 was effective in reducing tumor volume while the unmethylated oligonucleotide PS-ODN2 was partially effective. Figure 24B shows that not only did encapsulation of the oligonucleotides in the lipid particle increase the effectiveness of the unmethylated PS-ODN2 to a level similar to ODN1, but also that the encapsulated methylated oligonucleotide PS-ODN1 m was more effective than either of the encapsulated unmethylated oligonucleotides.

Figures 25-28 further illustrate that encapsulation of oligonucleotides provides equivalent or greater therapeutic efficacy for encapsulated methylated over unmethylated oligonucleotides. Figure 25 illustrates that lipid encapsulation of methylated PS-ODN5m provided a more effective therapeutic benefit than encapsulation of the equivalent unmethylated PS-ODN5 in reducing tumor growth over time. The effectiveness was further borne out by the superior survival rate of mice treated with encapsulated methylated PS-ODN5m in comparison to treatment with the unmethylated PS-ODN5 as shown in Figure 26. Figure 27 illustrates that while free unmethylated PS-ODN7 provides some anti-tumor benefit, free unmethylated PS-ODN 7 and PO-ODN7 as well as free methylated PS-ODN7 and PO-ODN7 were relatively ineffective in reducing tumor growth. However, lipid encapsulation of methylated PO-ODN7m provided effective therapeutic benefit in reducing tumor growth. Similarly, these trends were also illustrated in Figure 28 in the survival rate of mice treated with these same ODN.

**EXAMPLE 7**

**Therapeutic Anti-Tumor Efficacy Comparison of Methylated and Unmethylated Oligonucleotides in a B-16 Melanoma Tumor Model**

Anti-tumor efficacy induced by intravenous tail immunization was assessed in C57Bl/6 (8 animals per group) in a therapeutic immunization model. Animals were challenged with a subcutaneous injection of 3.0 x $10^5$ EG.7 B16/BL6 murine melanoma expressing tumor cells on Day-0. Mice were then treated intravenously every other day starting on day 4 for 14 days at a dose of 20mg/kg ODN. Mice were monitored every other day to assess tumor growth and weight gain. Control mice were injected with HBS on the same schedule described above. Oligonucleotides were encapsulated in a lipid particle having a lipid composition of DSPC: CHOL: DODAP:PEGCer14 each in a ratio of 25:45:20:10. Results from this efficacy experiments are detailed in Figures 16 and 17. Day 0 on each of the Figures is the day each animal was challenged with the tumor.

Figure 16 illustrates therapeutic efficacy of administering the methylated PS-ODN1m to an animal inoculated with a B16 melanoma tumor in comparison to its unmethylated counterpart PS-ODN1. Encapsulation of PS-ODN1m in a lipid particle increased its efficacy in reducing tumor volume to at least that of the encapsulated unmethylated PS-ODN1.

Figure 17 illustrates the average weight of the tumors in each mouse on Day 22. The average tumor size in mice treated with free methylated PS-ODN1 m was nearly the same as in mice treated with a buffer control, while mice treated with free unmethylated PS-ODN1 showed reduced tumor growth. In contrast, when mice were treated with the methylated PS-ODN1 encapsulated in a lipid particle, the amount of tumor reduction was near equivalent to that obtained with the lipid encapsulated unmethylated PS-ODN1. Accordingly, lipid encapsulation of methylated oligonucleotides can yield efficacy in treating a tumor *in vivo* even though the free methylated oligonucleotide has little or no efficacy.

**EXAMPLE 8**

**Blood Clearance Levels when Treated with Encapsulated Oligonucleotides**

An important aspect in effective immune stimulation is the ability of the immune system to raise an antibody response against specific antigens. One of the first demonstrations of the capacity of antigen associated with lipid encapsulated oligonucleotides to initiate such a response is illustrated by the data shown in Figure 23.

Each of ODN1 and ODN1m were encapsulated in two different lipid particles; Lipid one (L1) being a DSPC: CHOL:DODAP:PEGCer20 and the Lipid 2 (L2) being the same but having a PEGCer14 in the place of the PEGCer20. The half-life of the PEGCer 20 within the liposome is known to be much longer than that of the PEGCer14 and thus the PEGCer20 remains with the lipid particle for a longer time period. Mice were given a series of 4 i.v. tail injections, starting on Day 0, and were dosed once a week for 3 weeks. Blood was collected 1-hour post injection each week and were analyzed for the presence of the encapsulated ODN.

Figure 23 illustrates the effect on clearance from the blood in mice for the different lipid compositions, L1 and L2 each with different PEG-ceramide steric coatings (PEG-ceramide-C-20 and PEG-ceramide C-14 respectively) in combination with either methylated or unmethylated oligonucleotides, ODN1m and ODN1 respectively. After injection 1 the results show extended circulation/slow clearance for both of the encapsulated ODNs from the blood sample regardless of composition. However, for each of injections 2, 3 and 4 the results show that L1 liposomes (L1-ODN1 and L1-ODN1m) containing the long-lived PEGCer20 had shorter circulation/rapid clearance while L2-ODN1 and L2-ODN1m containing the short-lived PEG-ceramide C-14 had longer circulation/slower clearance than those encapsulated with lipid particles containing PEGCer20 lipid.

The data depicted herein demonstrates two specific points: (1) the induction of antigen specific antibodies; and (2) the relative immunostimulatory capacity of unmethylated and methylated oligonucleotide. In terms of induction of antigen specific antibodies, the initial injection resulted in the induction of antibodies directed against the PEG moiety of the PEG-ceramide steric barrier lipid. The presence of these antibodies in the plasma of injected animals resulted in the opsonization and subsequent rapid clearance from the circulation of liposomes containing PEG after injections 2, 3 and 4 as seen with L1 liposomes with PEGCer20. However, animals injected with liposomes without PEG, such as L2 liposomes with PEGCer14 from which the PEG dissociated very rapidly in circulation, were not oposonized and thus had relatively extended circulation times. In terms of the relative immunostimulatory potency of unmethylated vs. methylated oligonucleotide, the clearance of the liposomes containing either the unmethylated oligonucleotide or the corresponding methylated form were cleared at similar rates, thus indicating that both are able to induce antigen specific antibodies.

## EXAMPLE 9

### Induction of CTL Response Using A Polytope Approach with Multiple Tumor-Associated Antigens

Single epitope-based approaches have the disadvantage that an MHC-restricted CTL response is raised to only one antigen. In addition many cancer antigens are non-mutated differentiation antigens, such as TRP-2 exemplified above, and thus self-reactive T cells in the host are predominantly deleted during thymic education. A polytope approach would allow multiple antigens to be simultaneously targeted and should increase the spectrum of anti-tumor CTL responses against such self-antigens. CTL responses specific for multiple antigens and restricted by multiple MHC alleles would clearly be desirable for broader immune reactivity, given the variable expression of tumor antigens and MHC alleles in different malignancies. Targeting multiple antigens associated with a particular malignancy would minimize the chances of tumor escape by antigen downregulation or epitope mutation.

Targeting multiple antigens and MHC alleles might be achieved by using multiple recombinant antigen mixtures of synthetic peptide epitopes. To improve the immune response against these multi-epitope antigens several adjuvants are under assay. This experiment employs encapsulated PS-ODN 1 m using POPC:Chol:DODMA:PEG-DMG in combination with two murine melanoma antigens TRP2 ($H_2K_b$, VYDFFVWL) and Gp100 ($H_2D_b$, EGSRNQDWL). C57BL/6 mice were injected 3 times with either antigen, or both, in the presence of encapsulated PS-ODN 1m. As positive control we used dendritic cells known to be potent in inducing CTL responses. B16 lysate containing multiple epitopes was also assayed either with ODN 1 m or DC for the ability to induce multi-epitope immunity. Immune response was assessed by the ability of CD8+ T cells to mediate cytotoxicity against tumor cells in an antigen-specific manner.
Results: When injected together with encapsulated ODN 1 m, a CTL response was raised against both antigens (Figure 29). PS-ODN 1 m was as good as DC in generating CTL response against both antigens delivered together (Figure 30). Injection of B16 lysate with PS-ODN 1 m was more potent in inducing CTL than injection of DC incubated with B16 lysate (Figure 31).

The Examples provided illustrate certain embodiments of the invention. In a more general sense, however, the invention encompasses compositions and methods for providing therapeutic benefits to mammalian subjects (including humans) utilizing such compositions. The compositions of the invention are in the form of a lipid membrane vesicle; and a nucleic acid fully encapsulated within said vesicle. Where stimulation of a response to a particular antigen is desired, the composition may also associate the antigen with the vesicle, for example via chemical coupling, hydrophobic bonding or ionic bonding to an external surface of the vesicle, or encapsulation within the vesicle.

Preferred compositions are those in which the nucleic acid comprises greater than 4% by weight of the composition.

The nucleic acid in the compositions of the invention may suitably be nucleic acids which are not complementary to the genome of the treated mammal, and which provide immunostimulation through a mechanism which does not depend on a complementary base-pairing interaction with nucleic acids of the mammal. Such nucleic acids will frequently contain an immunostimulating sequence, such as a CpG motif or an immune stimulating palindrome.

The nucleic acids used in the compositions of the invention may be nucleic acids which do not induce an immune response when administered in free form to a naïve mammal, or which suppress an immune response to an immune stimulating sequence of nucleotides when administered in free form to a naïve mammal.

The nucleic acids may have exclusively phosphodiester internucleotide linkages or may be modified in which a way that they a plurality of phosphodiester internucleotide linkages in combination with modified internucleotide linkages. The nucleic acids may also contain exclusively modified linkages, or a plurality of modified linkages. For example, the nucleic acid may contain exclusively phosphorothioate internucleotide linkages or a plurality of phosphorothioate internucleotide linkages.

The cationic lipid which is used in formulating the composition suitably is selected from DODAP, DODMA, DMDMA, DOTAP, DC-Chol, DDAB, DODAC, DMRIE, and DOSPA. In addition, the lipid formulation preferably includes an aggregation preventing compound, such as a PEG-lipid, a PAO-lipid or a ganglioside.

In addition to or instead of an antigen, the compositions of the invention can include a co-encapsulated cytotoxic agent such as doxorubicin. The lipid membrane vesicle fully encapsulates both the nucleic acid and the cytotoxic agent. Compositions of this type can be prepared by a method which is a further aspect if the invention. In this method, a therapeutic composition is prepared preparing lipid in ethanol; mixing lipid with oligonucleotide in aqueous buffer to form oligonucleotide loaded lipid vesicles; and exposing the oligonucleotide loaded lipid vesicles to a cytotoxic agent such that the cytotoxic agent actively accumulates in the interior space of said vesicle.

The compositions of the invention can be used in various methods to provide therapeutic benefits to mammals, including humans, through the use of a lipid-nucleic acid particle comprising a nucleic acid which is fully encapsulated in a lipid formulation comprising a cationic lipid in the manufacture of a medicament. Thus, the compositions can be used to induce an immune response in a mammal, to activate CTL or B cells in a mammal or to treat neoplasia in a mammal having a neoplasia by a method comprising the steps of preparing a lipid-nucleic acid particle comprising a

nucleic acid which is fully encapsulated in a lipid formulation, which lipid formulation comprises a cationic lipid; and administering the lipid-nucleic acid particle to the mammal.

When an antigen is included in the composition, the invention provides a method of inducing an immune response to the antigen comprising preparing a particle comprising a lipid membrane vesicle comprising a nucleic acid fully encapsulated within said vesicle and an antigen to which an immune response is desired associated with an external surface of said vesicle, and administering the particles to the mammalian subject to be treated.

As demonstrated in the examples above, the utilization of a lipid carrier in the compositions in accordance with the invention allows a substantial reduction in the amount of oligonucleotide needed to achieve the desired stimulation of the immune system. In some cases, this is reflected in the fact that an oligonucleotide which had no apparent activity in the free form is useful for stimulating an immune response when provided in lipid-encapsulated form. In other cases, this is reflected in the fact that the amount of ODN necessary to achieve the same level of response with a lower dosage of ODN. Thus, in practicing a method employing an effective amount of oligonucleotide to stimulate an immune response in a mammal, the present invention provides the improvement comprising fully-encapsulating the oligonucleotide in a lipid vesicle and administering less than 20% of said effective amount of oligonucleotide to a mammalian subject, thereby obtaining a desired immune response in said mammalian subject.

While the data depicted herein demonstrates immunostimulatory activity *in vivo* and therapeutic efficacy using certain exemplary embodiments of the invention, which are provided for completeness and consistency, it is understood that the invention is not limited to these exemplary embodiments. One of ordinary skill in the art will be readily able to make and use other specific embodiments of the invention consistent with the teachings provided herein.

## Claims

**1.** An adjuvant comprising a lipid-nucleic acid (LNA) formulation, wherein said LNA formulation comprises:

a) a lipid component comprising at least one cationic lipid; and
b) a nucleic acid component comprising at least one methylated oligonucleotide; wherein said at least one methylated oligonucleotide comprises at least one CpG dinucleotide having a methylated cytosine, wherein said adjuvant is capable of stimulating dendritic cells *in vivo* in response to antigenic stimulation.

**2.** The adjuvant according to Claim 1, wherein said adjuvant is capable of stimulating dendritic cell expansion *in vivo* **characterized by** an increase in the number of antigen-presenting cells expressing at least one of a CD11c and a DEC205 marker.

**3.** The adjuvant according to Claim 1, wherein said adjuvant is capable of stimulating dendritic cell activation *in vivo* **characterized by** an increase in the number of antigen-presenting cells co-expressing at least one of a CD11c and a DEC205 marker in conjunction with a CD86 marker.

**4.** The adjuvant according to Claim 1 wherein said at least one methylated oligonucleotide comprises a single CpG dinucleotide having a methylated cytosine.

**5.** The adjuvant according to Claim 4, wherein said at least one methylated oligonucleotide comprises the sequence 5' TAACGTTGAGGGGCAT 3'

6. The adjuvant according to Claim 1, wherein said at least one methylated oligonucleotide comprises two CpG dinucleotides, and wherein at least one of the cytosines in said CpG dinucleotides is methylated.

7. The adjuvant according to Claim 6, wherein said at least one methylated oligonucleotide comprises the sequence 5' TTCCATGACGTTCCTGACGTT 3'.

8. The adjuvant according to any one of Claims 1 to 7, wherein said oligonucleotide comprises a modified phosphate backbone.

9. The adjuvant according to Claim 8, wherein said modified phosphate backbone is phosphorothioate.

10. The adjuvant according to Claim 1, wherein said at least one methylated oligonucleotide comprises a plurality of CpG dinucleotides, and wherein at least one of said CpG dinucleotides comprises a methylated cytosine.

11. A vaccine comprising an adjuvant as claimed in any one of Claims 1-10 in combination with at least one target antigen, wherein said at least one target antigen is mixed with or associated with said LNA formulation, and wherein said vaccine is capable of stimulating dendritic cells *in vivo* in response to presentation of said at least one target antigen by said formulation to antigen-presenting cells.

12. The vaccine according to Claim 11, wherein said at least one target antigen comprises a microbial antigen.

13. The vaccine according to Claim 11, wherein said at least one target antigen comprises a plurality of epitopes from the same antigen.

14. The vaccine according to Claim 11, wherein said at least one target antigen comprises a plurality of epitopes from different antigens.

15. The use of an adjuvant as claimed in any one of Claims 1 to 10 or a vaccine as claimed in any one of Claims 11 to 14 in the manufacture of a medicament for stimulating an enhanced host immune response to antigenic stimulation.

16. An *in vitro* method for stimulating dendritic cells, comprising contacting at least one dendritic cell with a lipid-methylated nucleic acid formulation said formulation comprising a lipid component and a nucleic acid component comprising a methylated nucleic acid sequence, wherein said methylated nucleic acid sequence comprises at least one CpG dinucleotide having a methylated cytosine, and wherein said lipid-methylated nucleic acid formulation is capable of stimulating dendritic cells *in vivo* in response to antigenic stimulation.

17. The method according to Claim 16, wherein said methylated cytosine comprises a methyl or hydroxymethyl group attached to the carbon-4 position or carbon-5 position.

18. The use of a lipid-methylated nucleic acid formulation, said formulation comprising a lipid component and a nucleic acid component comprising a methylated nucleic acid sequence, wherein said methylated nucleic acid sequence comprises at least one CpG dinucleotide having a methylated cytosine, and wherein said lipid-methylated nucleic acid formulation is capable of stimulating dendritic cells *in vivo* in response to antigenic stimulation, in the manufacture of a medicament for stimulating host dendritic cells.

19. The use according to Claim 18, wherein said methylated cytosine comprises a methyl or hydroxymethyl group attached to the carbon-4 position or carbon-5 position.

20. The method according to Claims 16 or 17 or use according to Claims 18 or 19, wherein stimulating dendritic cells comprises dendritic cell expansion **characterized by** an increase in the number of antigen-presenting cells expressing at least one of a CD11c and a DEC205 marker.

21. The method according to Claims 16 or 17 or use according to Claims 18 or 19, wherein stimulating dendritic cells comprises dendritic cell activation **characterized by** an increase in the number of antigen-presenting cells co-expressing at least one of a CAD11c and a DEC205 marker in conjunction with a CD86 marker.

22. The use of a lipid-nucleic (LNA) formulation associated with a target antigen in the manufacture of a medicament for simultaneously delivering antigenic and adjuvant immune stimulation to antigen presenting cells, said LNA

formulation comprising:

a) a lipid component comprising at least one cationic lipid; and
b) a nucleic acid component comprising at least one methylated oligonucleotide comprising at least one CpG dinucleotide having a methylated cytosine; wherein said LNA formulation is capable of stimulating dendritic cells *in vivo.*

**23.** Products containing an adjuvant as claimed in any one of Claims 1-10 and at least one target antigen mixed or associated with said LNA formulation for simultaneous, sequential or separate use in stimulating an enhanced host immune response to antigen stimulation.

**Patentansprüche**

**1.** Ein Adjuvans umfassend eine Lipid-Nukleinsäure (LNA) Formulierung, wobei die LNA Formulierung

a) einen Lipid-Bestandteil, der mindestens ein kationisches Lipid umfasst; und
b) einen Nukleinsäure-Bestandteil, der mindestens ein methyliertes Oligonukleotid umfasst,

umfasst; wobei das mindestens eine methylierte Oligonukleotid mindestens ein CpG Dinukleotid, das ein methyliertes Cytosin besitzt, umfasst, und wobei das Adjuvans in der Lage ist dendritische Zellen *in vivo* als Antwort auf Antigen-Stimulation zu stimulieren.

**2.** Das Adjuvans gemäß Anspruch 1, wobei das Adjuvans in der Lage ist, eine Expansion von dendritischen Zellen, die sich durch einen Anstieg der Zahl antigen-präsentierender Zellen, die mindestens einen von einem CD11c und einem DEC205 Marker exprimieren, auszeichnet, *in vivo* zu stimulieren.

**3.** Das Adjuvans gemäß Anspruch 1, wobei das Adjuvans in der Lage ist, eine Aktivierung von dendritischen Zellen, die sich durch einen Anstieg der Zahl antigen-präsentierender Zellen, die mindestens einen von einem CD11c und einem DEC205 Marker zusammen mit einem CD86 Marker koexprimieren, auszeichnet, *in vivo* zu stimulieren.

**4.** Das Adjuvans gemäß Anspruch 1, wobei das mindestens eine methylierte Oligonukleotid ein einzelnes CpG Dinukleotid, das ein methyliertes Cytosin besitzt, umfasst.

**5.** Das Adjuvans gemäß Anspruch 4, wobei das mindestens eine methylierte Oligonukleotid die Sequenz 5' TAACGTTGAGGGGCAT 3' umfasst.

**6.** Das Adjuvans gemäß Anspruch 1, wobei das mindestens eine methylierte Oligonukleotid zwei CpG Dinukleotide umfasst und wobei mindestens eins der Cytosine in den CpG Dinukleotiden methyliert ist.

**7.** Das Adjuvans gemäß Anspruch 6, wobei das mindestens eine methylierte Oligonukleotid die Sequenz 5' TTCCATGACGTTCCTGACGTT 3' umfasst.

**8.** Das Adjuvans gemäß einem der Ansprüche 1 bis 7, wobei das Oligonukleotid ein modifiziertes Phosphatrückgrat umfasst.

**9.** Das Adjuvans gemäß Anspruch 8, wobei das modifizierte Phosphatrückgrat Phosphorothioat ist.

**10.** Das Adjuvans gemäß Anspruch 1, wobei das mindestens eine methylierte Oligonukleotid eine Vielzahl von CpG Dinukleotiden umfasst und wobei mindestens eins der CpG Dinukleotide ein methyliertes Cytosin umfasst.

**11.** Ein Vakzin, das ein Adjuvans wie in einem der Ansprüche 1-10 beansprucht in Kombination mit mindestens einem Ziel-Antigen umfasst, wobei das mindestens eine Ziel-Antigen mit der LNA Formulierung gemischt oder damit assoziiert ist, und wobei das Vakzin in der Lage ist, dendritische Zellen *in vivo* als Antwort auf die Präsentation des mindestens einen Ziel-Antigens gegenüber antigen-pcäsentierenden Zellen durch die Formulierung zu stimulieren.

**12.** Das Vakzin gemäß Anspruch 11, wobei das mindestens eine Ziel-Antigen ein mikrobielles Antigen umfasst.

**13.** Das Vakzin gemäß Anspruch 11, wobei das mindestens eine Ziel-Antigen eine Vielzahl von Epitopen desselben Antigens umfasst.

**14.** Das Vakzin gemäß Anspruch 11, wobei das mindestens eine Ziel-Antigen eine Vielzahl von Epitopen von unterschiedlichen Antigenen umfasst

**15.** Verwendung eines Adjuvans wie in einem der Anspruche 1 bis 10 beansprucht oder eines Vakzins wie in einem der Ansprüche 11 bis 14 beansprucht bei der Herstellung eines Medikaments für die Stimulation einer verstärkten Immunantwort eines Wirts auf eine Antigen-Stimulation.

**16.** Ein *in vitro* Verfahren zum Stimulieren dendritischer Zellen, umfassend das Inkontaktbringen mindestens einer dendritischen Zelle mit einer Lipid-methylierten Nukleinsäure Formulierung, wobei die Formulierung einen Lipid-Bestandteil und einen Nukleinsäure-Bestandteil, der eine methylierte Nukleinsäuresequenz umfasst, umfasst, wobei die methylierte Nukleinsäuresequenz mindestens ein CpG, Dinukleotid, das ein methyliertes Cytosin besitzt, umfasst, und wobei die Lipid-methylierte Nukleinsäure Formulierung in der Lage ist, dendritische Zellen *in vivo* als Antwort auf Antigen-Stimulation zu stimulieren.

**17.** Das Verfahren gemäß Anspruch 16, wobei das methylierte Cytosin eine Methyl- oder Hydroxymethyl-Gruppe umfasst, die an die Kohlenstoff-4 Position oder Kohlenstoff-5 Position gebunden ist.

**18.** Verwendung einer Lipid-methylierte Nukleinsäure Formulierung, wobei die Formulierung einen Lipid-Bestandteil und einen Nukleinsäure-Bestandteil, der eine methylierte Nukleinsäuresequenz umfasst, umfasst, wobei die methylierte Nukleinsäuresequenz mindestens ein CpG Dinukleotid, das ein methyliertes Cytosin besitzt, umfasst, und wobei die Lipid-methylierte Nukleinsäure Formulierung in der Lage ist, dendritische Zellen *in vivo* als Antwort auf Antigen-Stimulation zu stimulieren, bei der Herstellung eines Medikaments zur Stimulation der dendritischen Zellen eines Wirts.

**19.** Die Verwendung gemäß Anspruch 18, wobei das methylierte Cytosin eine Methyl- oder Hydroxymethyl-Gruppe umfasst, die an die Kohlenstoff-4 Position oder Kohlenstoff-5 Position gebunden ist.

**20.** Das Verfahren gemäß Anspruch 16 oder 17 oder die Verwendung gemäß Anspruch 18 oder 19, wobei die Stimulation der dendritischen Zellen eine Expansion der dendritischen Zellen, die sich durch den Anstieg der Anzahl der antigenpräsentierenden Zellen, die mindestens einen von einem CD11c und einem DC205 Marker exprimieren, auszeichnet, umfasst.

**21.** Das Verfahren gemäß Anspruch 16 oder 17 oder die Verwendung gemäß Anspruch 18 oder 19, wobei die Stimulation der dendritischen Zellen eine Aktivierung der dendritischen Zellen, die sich durch den Anstieg der Anzahl der antigenpräsentierenden Zellen, die mindestens einen von einem CD11c und einem DEC205 Marker zusammen mit einem CD86 Marker koexprimieren, auszeichnet, umfasst.

**22.** Verwendung einer Lipid-Nukleinsäure (LNA) Formulierung, die mit einem Ziel-Antigen assoziiert ist, bei der Herstellung eines Medikaments zur gleichzeitigen Abgabe einer Antigen- und Adjuvans-Immunstimulation an antigenpräsentierende Zellen, wobei die LNA Formulierung:

a) einen Lipid-Bestandteil, der mindestens ein kationisches Lipid umfasst; und
b) einen Nukleinsäire-Bestandteil, der mindestens ein methyliertes Oligonukleotid, das mindestens ein CpG Dinukleotid, das ein methyliertes Cytosin besitzt, umfasst,

umfasst; wobei die LNA Formulierung in der Lage ist, dendritische Zellen *in vivo* zu stimulieren.

**23.** Produkte, die ein Adjuvans wie in einem der Ansprüche 1 bis 10 beansprucht und mindestens ein Ziel-Antigen, das mit der LMA Formulierung gemischt oder damit assoziiert ist, enthalten, für die gleichzeitige, aufeinander folgende oder getrennte Verwendung bei der Stimulation einer verstärkten Immunantwort eines Wirts auf eine Antigen-Stimulation.

**Revendications**

1. Un adjuvant comportant une formulation lipide - acide nucléique (LNA), dans lequel ladite formulation LNA comprend :

    a) un composant lipidique comprenant au moins un lipide cationique ; et
    b) un composant acide nucléique comprenant au moins un oligonucléotide méthylé ; dans lequel ledit au moins un oligonucléotide méthylé comporte au moins un dinucléotide CpG ayant une cytosine méthylée, dans lequel ledit adjuvant a la propriété de stimuler les cellules dendritiques *in vivo* en réponse à une stimulation antigénique.

2. L'adjuvant selon la revendication 1, dans lequel ledit adjuvant a la propriété de stimuler la multiplication des cellules dendritiques *in vivo* **caractérisées par** l'augmentation du nombre de cellules présentatrices de l'antigène exprimant au moins un des marqueurs CD11c et DEC205.

3. L'adjuvant selon la revendication 1, dans lequel ledit adjuvant a la propriété de stimuler l'activation des cellules dendritiques *in vivo* **caractérisées par** une augmentation du nombre de cellules présentatrices de l'antigène co-exprimant au moins un des marqueurs CD11c et DEC205 en conjonction avec le marqueur CD86.

4. L'adjuvant selon la revendication 1 dans lequel ledit au moins un oligonucléotide comprend un unique dinucléotide CpG ayant une cytosine méthylée.

5. L'adjuvant selon la revendication 4, dans lequel ledit au moins un oligonucléotide comprend la séquence 5' TAACGTTGAGGGGCAT 3'.

6. L'adjuvant selon la revendication 1 dans lequel ledit au moins un oligonucléotide comprend deux dinucléotides CpG, et dans lequel au moins une des cytosines de ces dinucléotides CpG est méthylée.

7. L'adjuvant selon la revendication 6, dans lequel ledit au moins un oligonucléotide méthylé comprend la séquence 5' TTCCATGACGTTCCTGACGTT 3'.

8. L'adjuvant selon l'une quelconque des revendications de 1 à 7, dans lequel ledit oligonucléotide comporte un squelette phosphate modifié.

9. L'adjuvant selon la revendication 8, dans lequel ledit squelette phosphate modifié est un phosphorothioate.

10. L'adjuvant selon la revendication 1, dans lequel ledit au moins un oligonucléotide méthylé comporte une pluralité de dinucléotides CpG, et dans lequel au moins un de ces dinucléotides CpG comporte une cytosine méthylée.

11. Un vaccin comportant un adjuvant revendiqué selon l'une quelconque des revendications 1-10 en combinaison avec au moins un antigène cible, dans lequel ledit au moins un antigène cible est mélangé avec ou associé avec ladite formulation LNA, et dans lequel ledit vaccin a la propriété de stimuler les cellules dendritiques *in vivo* en réponse à la présentation dudit au moins un antigène cible par ladite formulation à des cellules présentatrices de l'antigène.

12. Le vaccin selon la revendication 11, dans lequel ledit au moins un antigène cible comporte un antigène microbien.

13. Le vaccin selon la revendication 11, dans lequel ledit au moins un antigène cible comporte une pluralité d'épitopes à partir d'un même antigène.

14. Le vaccin selon la revendication 11, dans lequel ledit au moins un antigène cible comporte une pluralité d'épitopes à partir d'antigènes différents.

15. L'utilisation d'un adjuvant revendiqué selon l'une quelconque des revendications 1 à 10 ou un vaccin revendiqué selon l'une quelconque des revendications de 11 à 14 dans la fabrication d'un médicament pour la stimulation de la réponse immune induite de l'hôte par une stimulation antigénique.

16. Une méthode *in vitro* pour la stimulation de cellules dendritiques, comprenant la mise en contact d'au moins une cellule dendritique avec une formulation lipide - acide nucléique méthylée, ladite formulation comportant un composant lipide et un composant acide nucléique comportant une séquence acide nucléique méthylée, dans laquelle ladite séquence acide nucléique méthylée comporte au moins un dinucléotide CpG ayant une cytosine méthylée, et dans laquelle ladite formulation lipide - acide nucléique méthylé a la propriété de stimuler les cellules dendritiques *in vivo* en réponse à la stimulation antigénique.

**17.** La méthode selon la revendication 16, dans laquelle ladite cytosine méthylée comporte un groupement méthyle ou hydroxyméthyle attaché au carbone en position 4 ou au carbone en position 5.

**18.** L'utilisation d'une formulation lipide - acide nucléique méthylé, ladite formulation comprenant un composant lipide et un composant acide nucléique comportant une séquence acide nucléique méthylée, dans laquelle ladite séquence acide nucléique méthylée comprend au moins un dinucléotide CpG ayant une cytosine méthylée, et dans laquelle la formulation lipide - acide nucléique méthylée a la propriété de stimuler les cellules dendritiques *in vivo* en réponse à la stimulation antigénique, dans la fabrication d'un médicament pour la stimulation de cellules dendritiques hôtes.

**19.** L'utilisation selon la revendication 18, dans laquelle ladite cytosine méthylée comporte un groupement méthyle ou hydroxyméthyle attachée au carbone en position 4 ou au carbone en position 5.

**20.** La méthode selon les revendications 16 ou 17 ou l'utilisation selon les revendications 18 ou 19, dans laquelle la stimulation des cellules dendritiques comporte la multiplication de la cellule dendritique **caractérisé par** une augmentation du nombre de cellules présentatrices de l'antigène exprimant au moins un des marqueurs CD11 c et DEC205.

**21.** La méthode selon les revendications 16 ou 17 ou l'utilisation selon les revendications 18 ou 19, dans laquelle la stimulation des cellules dendritiques comporte l'activation d'une cellule dendritique **caractérisée par** une augmentation du nombre des cellules présentatrices d'antigène co-exprimant au moins un des marqueurs CD11c et DEC205 en conjonction avec le marqueur CD86.

**22.** L'utilisation d'une formulation lipide - acide nucléique (LNA) associée avec un antigène cible dans la fabrication d'un médicament pour délivrer de façon simultanée une stimulation antigénique et un adjuvant à des cellules présentatrices, ladite formulation LNA comportant :

a) un composant lipide comportant au moins un lipide cationique ; et
b) un composant acide nucléique comportant au moins un oligonucléotide comportant au moins un dinucléotide CpG ayant une cytosine méthylée ; dans lequel ladite formulation LNA a la propriété de stimuler ces cellules dendritique *in vivo.*

**23.** Les produits contenant un adjuvant revendiqué selon l'une quelconque des revendications 1 - 10 et au moins un antigène cible mixé ou associé avec ladite formulation LNA pour une utilisation simultanée, séquentielle ou séparée dans la stimulation d'une réponse immune induite chez l'hôte par une stimulation antigénique.

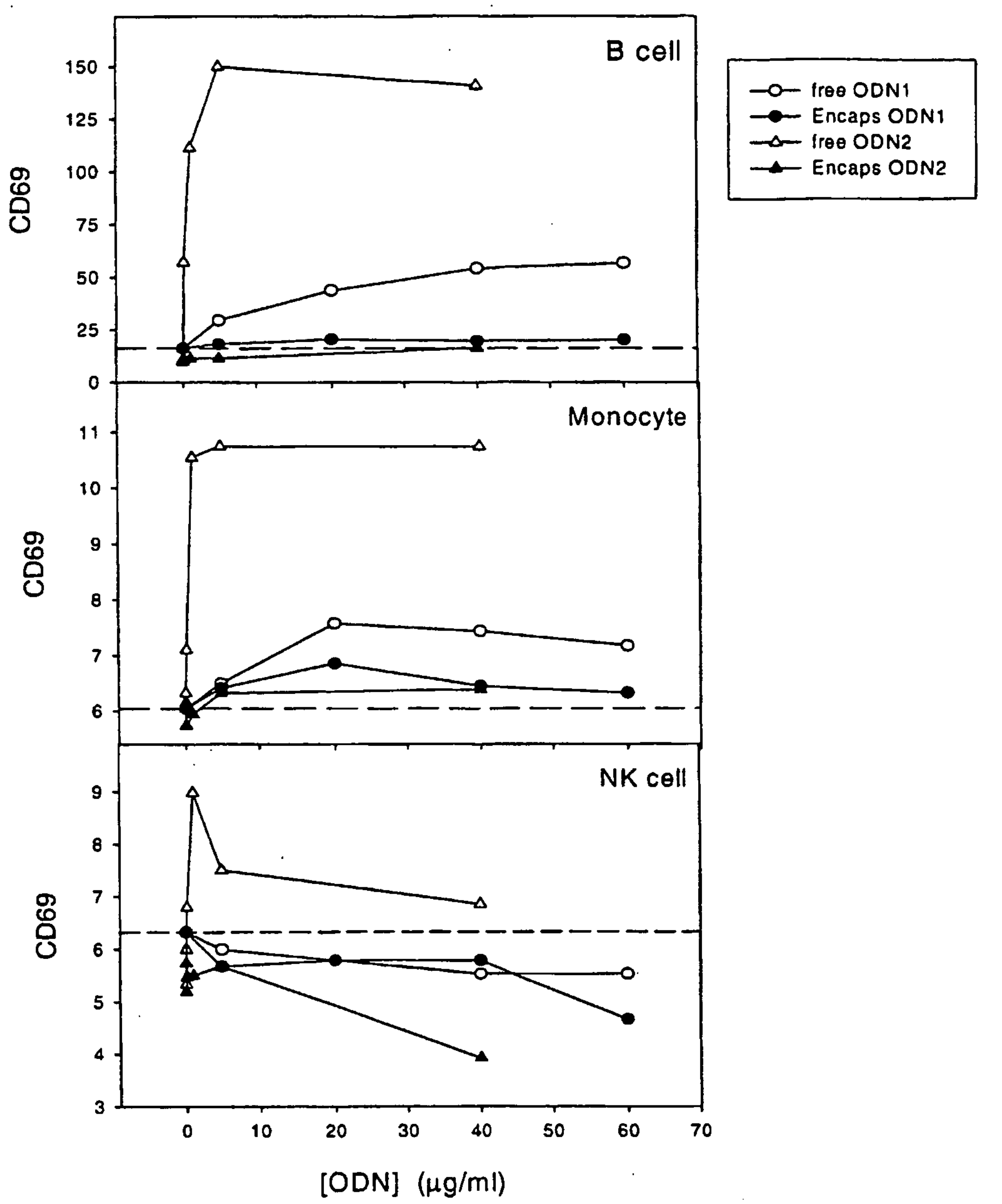
B cell
Monocyte
NK cell
CD69
CD69
CD69
150
125
100
75
50
25
0
11
10
9
8
7
6
9
8
7
6
5
4
3
0
10
20
30
40
50
60
70
[ODN] (µg/ml)
free ODN1
Encaps ODN1
free ODN2
Encaps ODN2

Figure 1

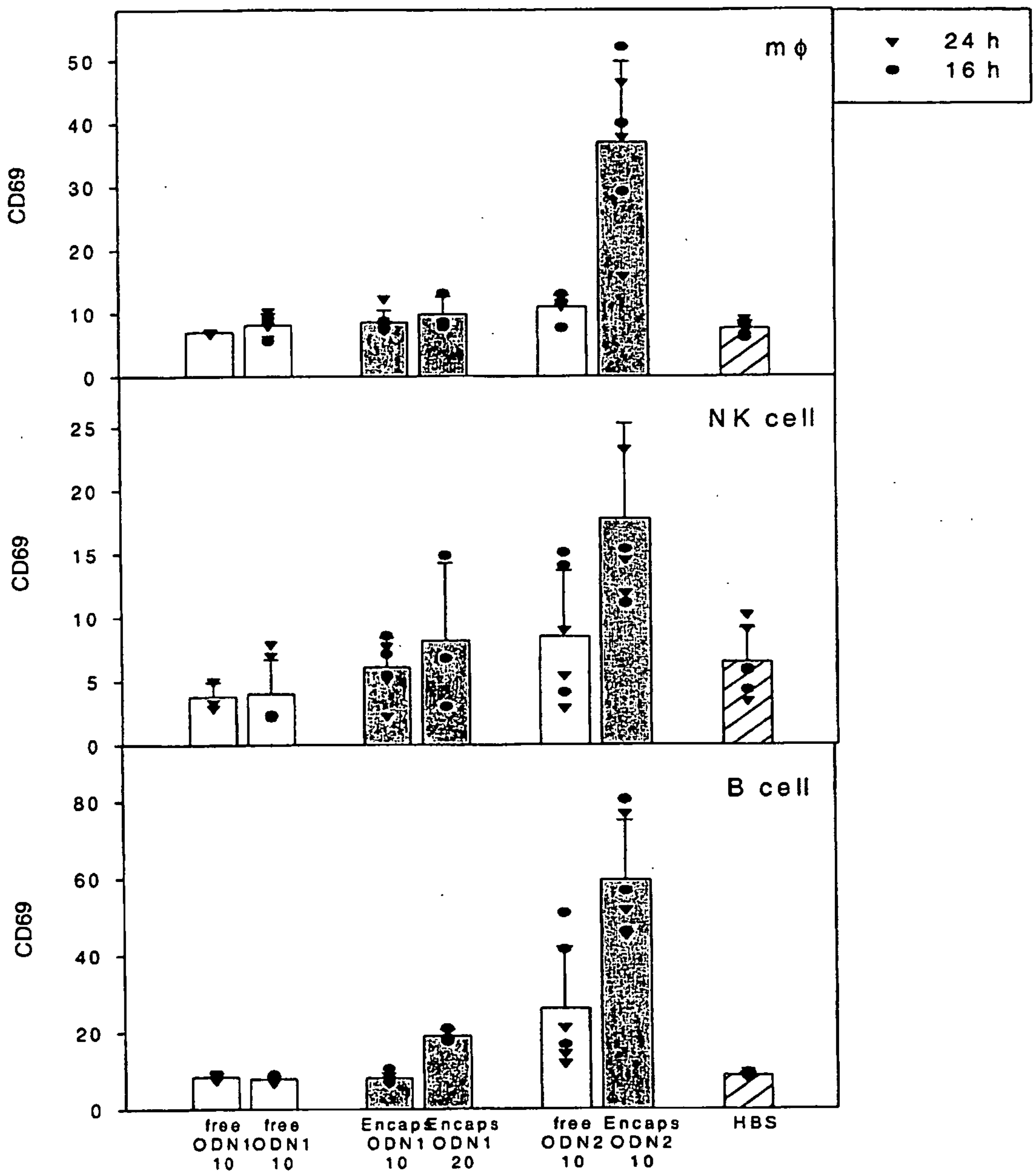
m ϕ
NK cell
B cell
24 h
16 h
CD69
CD69
CD69
0
10
20
30
40
50
0
5
10
15
20
25
0
20
40
60
80
free ODN1 10
free ODN1 10
Encaps ODN1 10
Encaps ODN1 20
free ODN2 10
Encaps ODN2 10
HBS

Figure 2

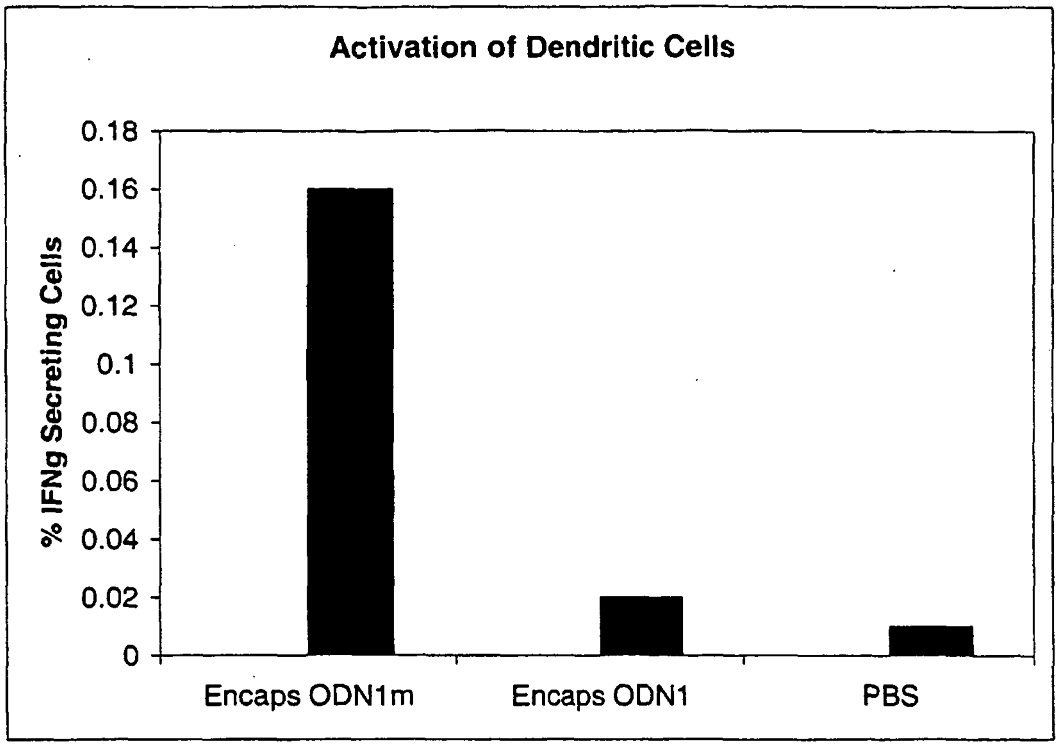
Activation of Dendritic Cells
% IFNg Secreting Cells
0.18
0.16
0.14
0.12
0.1
0.08
0.06
0.04
0.02
0
Encaps ODN1m
Encaps ODN1
PBS

Figure 3

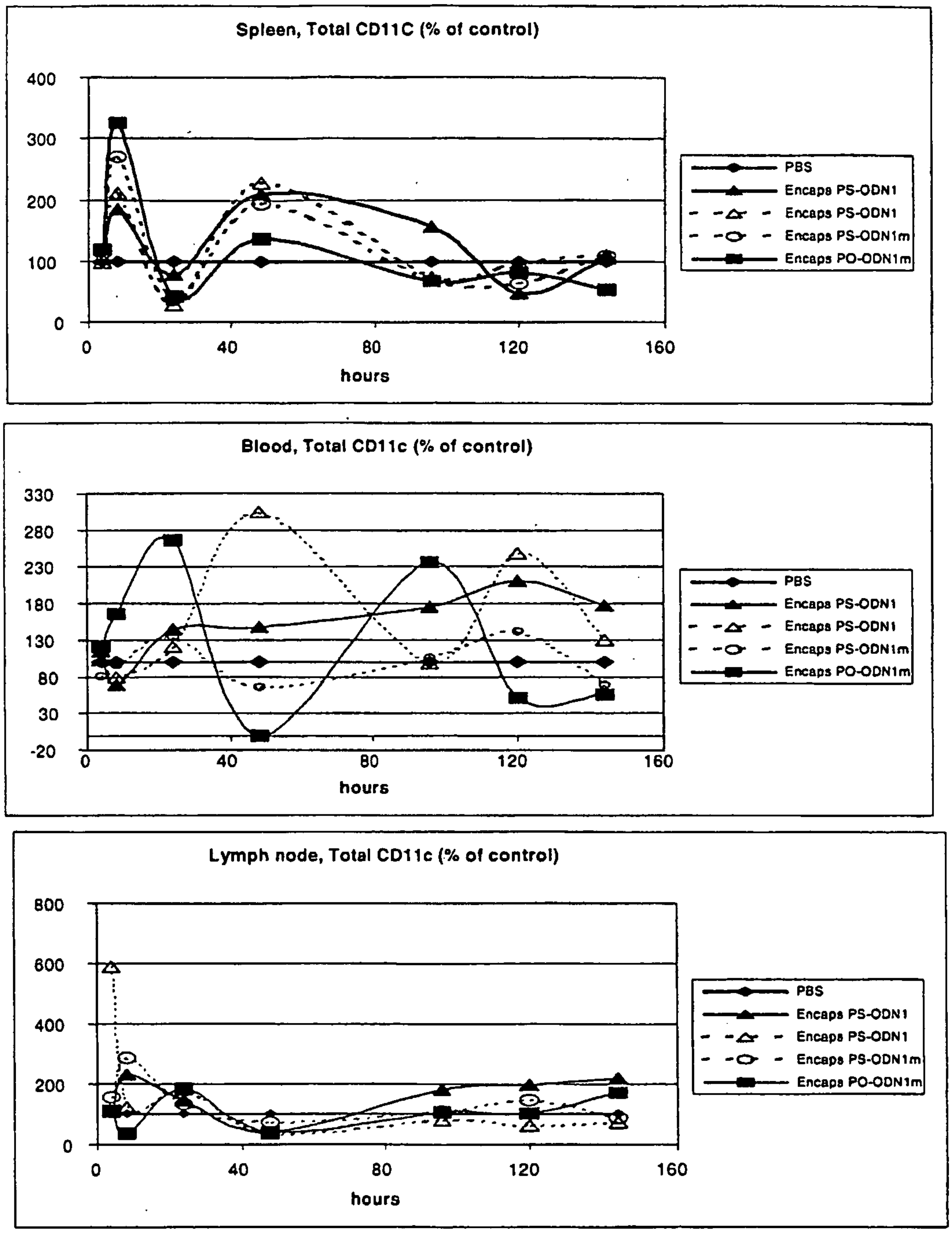
Spleen, Total CD11C (% of control)
400
300
200
100
0
0
40
80
120
160
hours
PBS
Encaps PS-ODN1
Encaps PS-ODN1
Encaps PS-ODN1m
Encaps PO-ODN1m
Blood, Total CD11c (% of control)
330
280
230
180
130
80
30
-20
0
40
80
120
160
hours
PBS
Encaps PS-ODN1
Encaps PS-ODN1
Encaps PS-ODN1m
Encaps PO-ODN1m
Lymph node, Total CD11c (% of control)
800
600
400
200
0
0
40
80
120
160
hours
PBS
Encaps PS-ODN1
Encaps PS-ODN1
Encaps PS-ODN1m
Encaps PO-ODN1m

Figure 4A

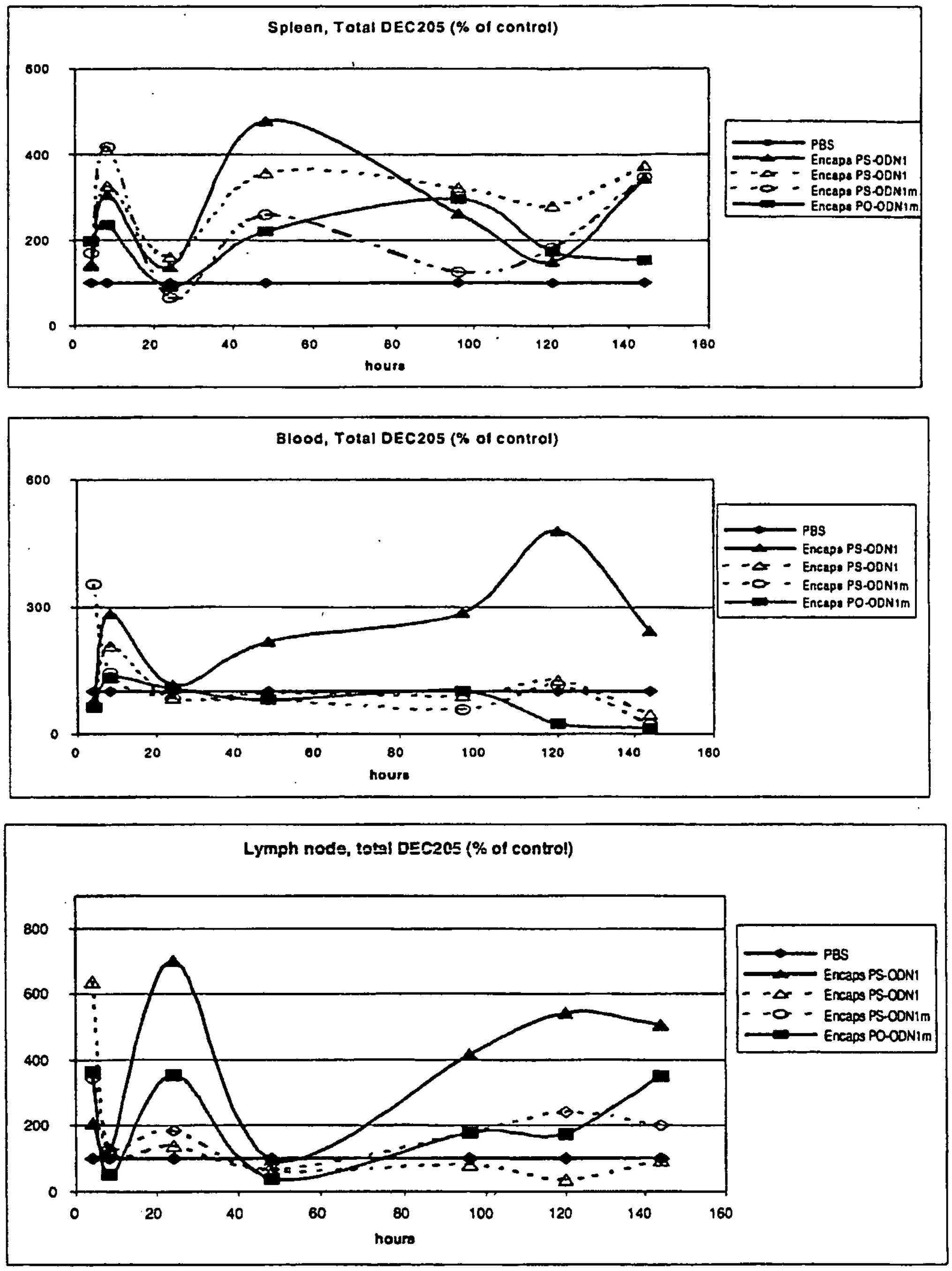
Spleen, Total DEC205 (% of control)
600
400
200
0
0 20 40 60 80 100 120 140 160
hours
PBS
Encaps PS-ODN1
Encaps PS-ODN1
Encaps PS-ODN1m
Encaps PO-ODN1m
Blood, Total DEC205 (% of control)
600
300
0
0 20 40 60 80 100 120 140 160
hours
PBS
Encaps PS-ODN1
Encaps PS-ODN1
Encaps PS-ODN1m
Encaps PO-ODN1m
Lymph node, total DEC205 (% of control)
800
600
400
200
0
0 20 40 60 80 100 120 140 160
hours
PBS
Encaps PS-ODN1
Encaps PS-ODN1
Encaps PS-ODN1m
Encaps PO-ODN1m

Figure 4B

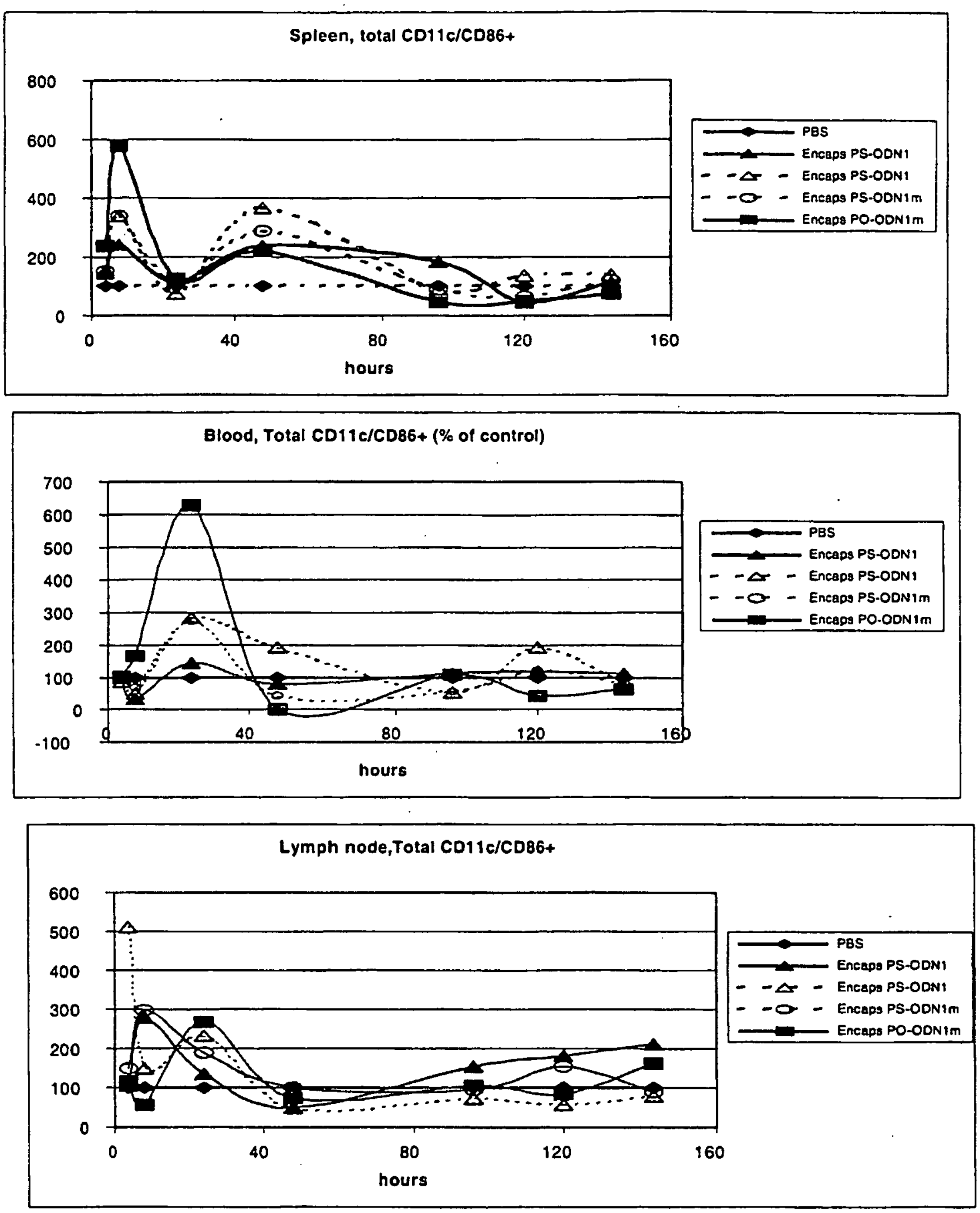
Spleen, total CD11c/CD86+
800
600
400
200
0
0
40
80
120
160
hours
PBS
Encaps PS-ODN1
Encaps PS-ODN1
Encaps PS-ODN1m
Encaps PO-ODN1m
Blood, Total CD11c/CD86+ (% of control)
700
600
500
400
300
200
100
0
-100
0
40
80
120
160
hours
PBS
Encaps PS-ODN1
Encaps PS-ODN1
Encaps PS-ODN1m
Encaps PO-ODN1m
Lymph node,Total CD11c/CD86+
600
500
400
300
200
100
0
0
40
80
120
160
hours
PBS
Encaps PS-ODN1
Encaps PS-ODN1
Encaps PS-ODN1m
Encaps PO-ODN1m

Figure 5A

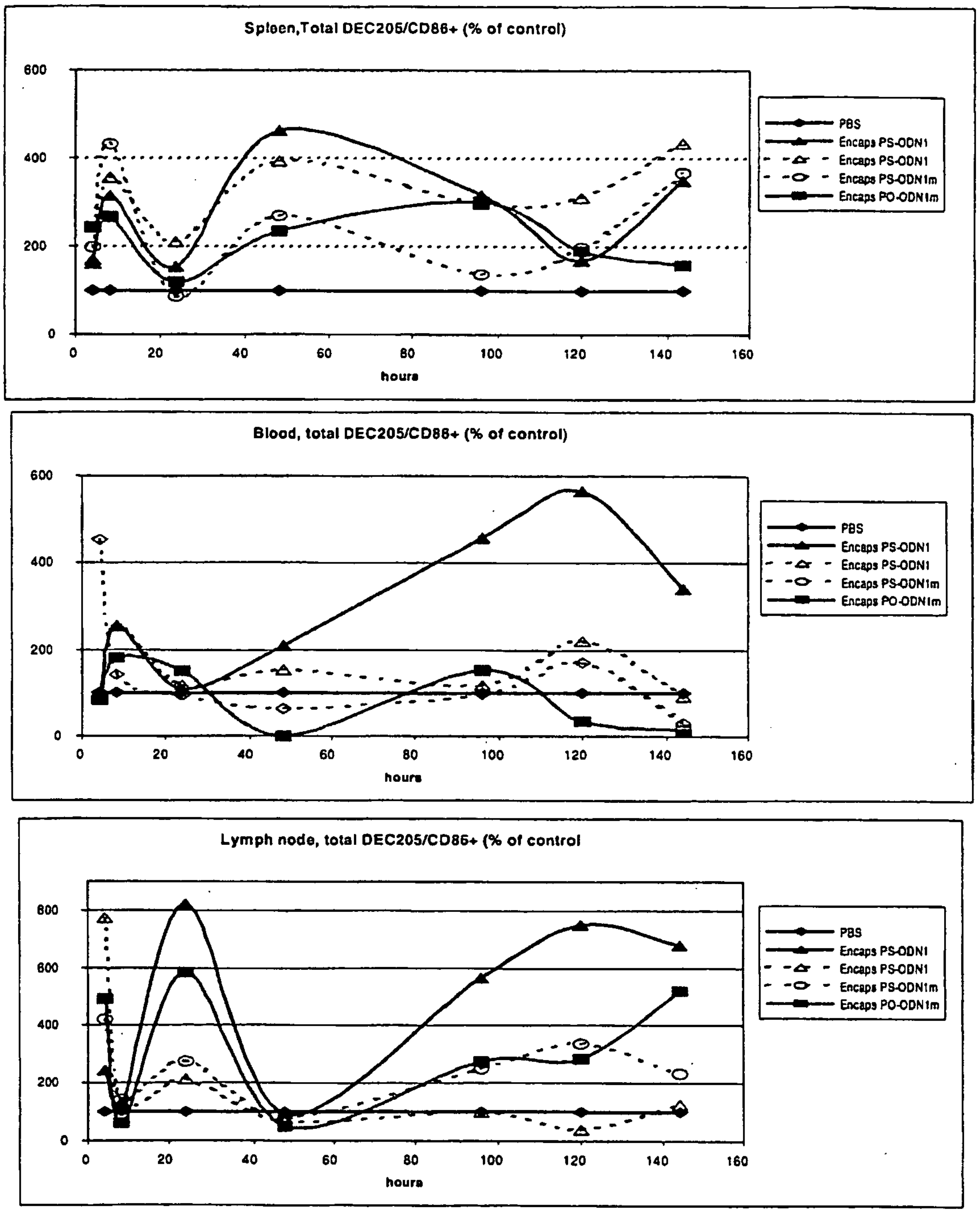
Spleen,Total DEC205/CD86+ (% of control)
600
400
200
0
0
20
40
60
80
100
120
140
160
hours
PBS
Encaps PS-ODN1
Encaps PS-ODN1
Encaps PS-ODN1m
Encaps PO-ODN1m
Blood, total DEC205/CD86+ (% of control)
600
400
200
0
0
20
40
60
80
100
120
140
160
hours
PBS
Encaps PS-ODN1
Encaps PS-ODN1
Encaps PS-ODN1m
Encaps PO-ODN1m
Lymph node, total DEC205/CD86+ (% of control
800
600
400
200
0
0
20
40
60
80
100
120
140
160
hours
PBS
Encaps PS-ODN1
Encaps PS-ODN1
Encaps PS-ODN1m
Encaps PO-ODN1m

Figure 5B

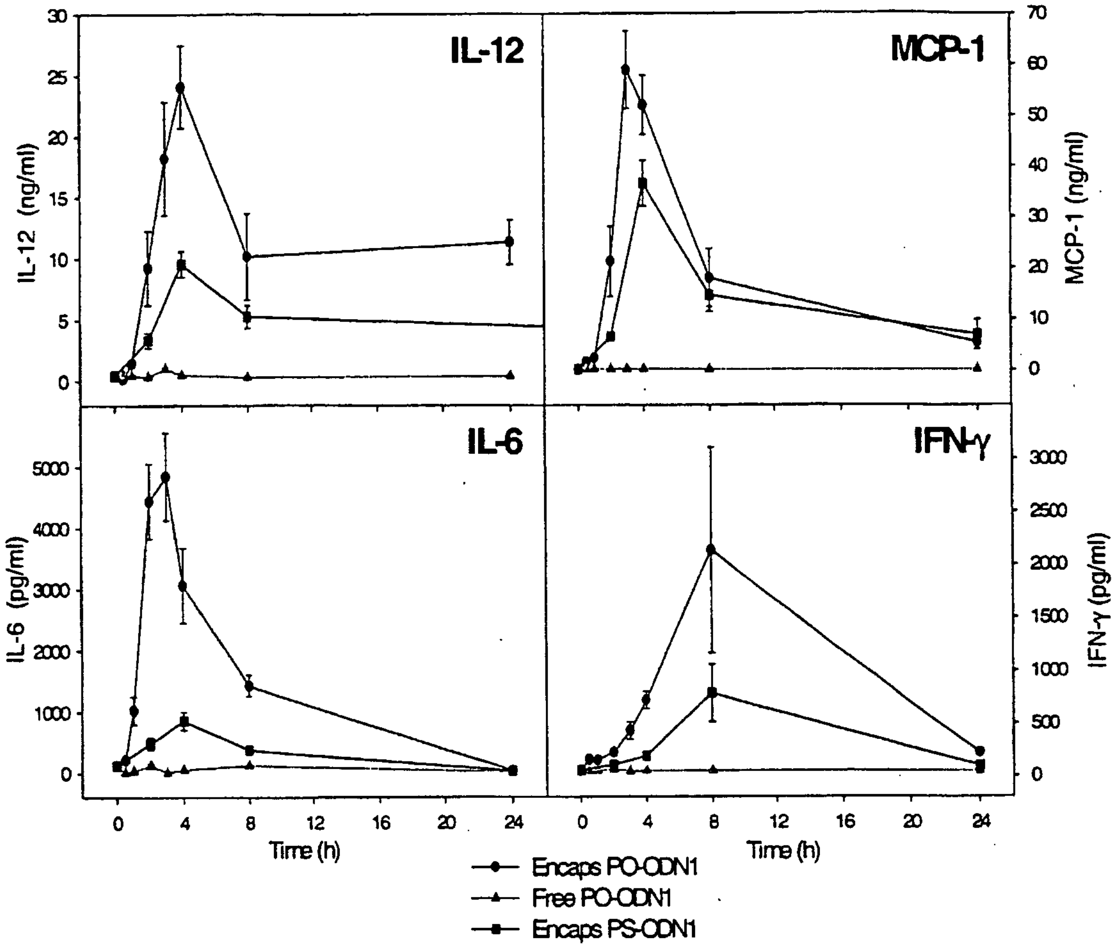
IL-12
MCP-1
IL-6
IFN-γ
IL-12 (ng/ml)
MCP-1 (ng/ml)
IL-6 (pg/ml)
IFN-γ (pg/ml)
Time (h)
Time (h)
Encaps PO-ODN1
Free PO-ODN1
Encaps PS-ODN1

Figure 6

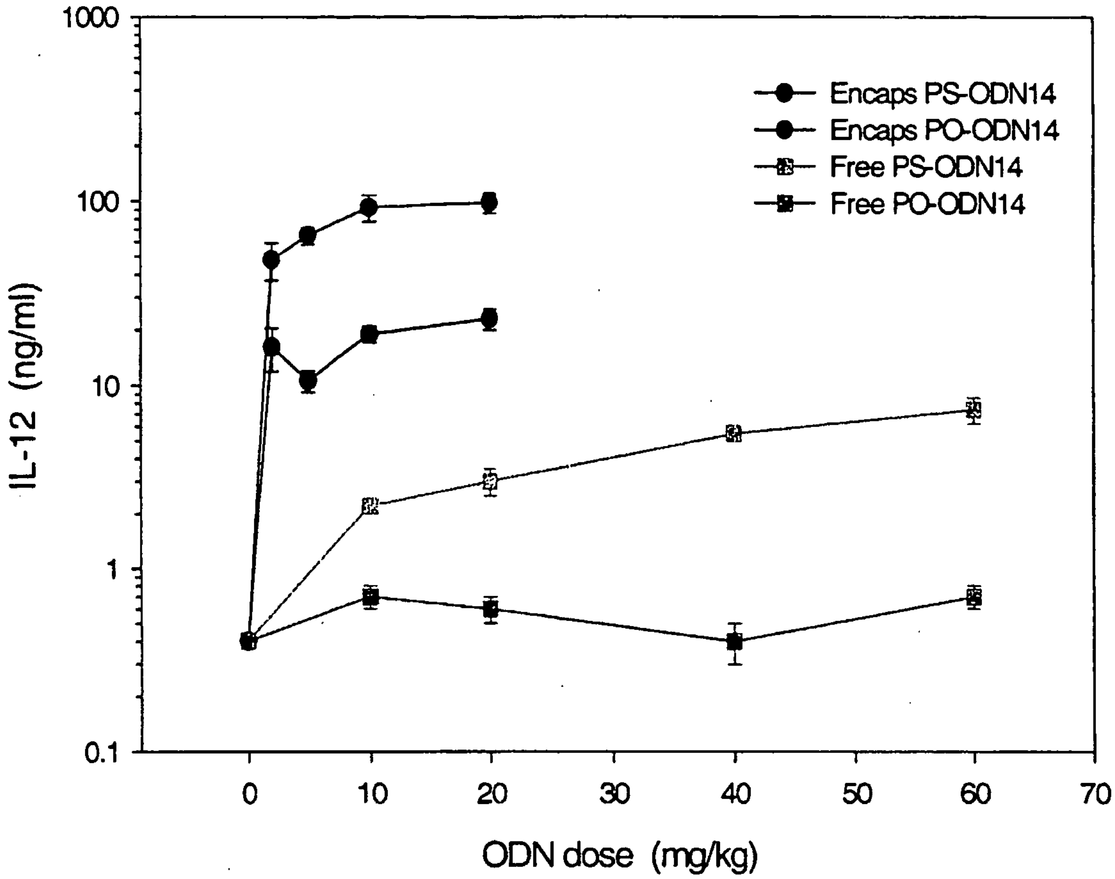
1000
100
10
1
0.1
IL-12 (ng/ml)
Encaps PS-ODN14
Encaps PO-ODN14
Free PS-ODN14
Free PO-ODN14
0
10
20
30
40
50
60
70
ODN dose (mg/kg)

Figure 7A

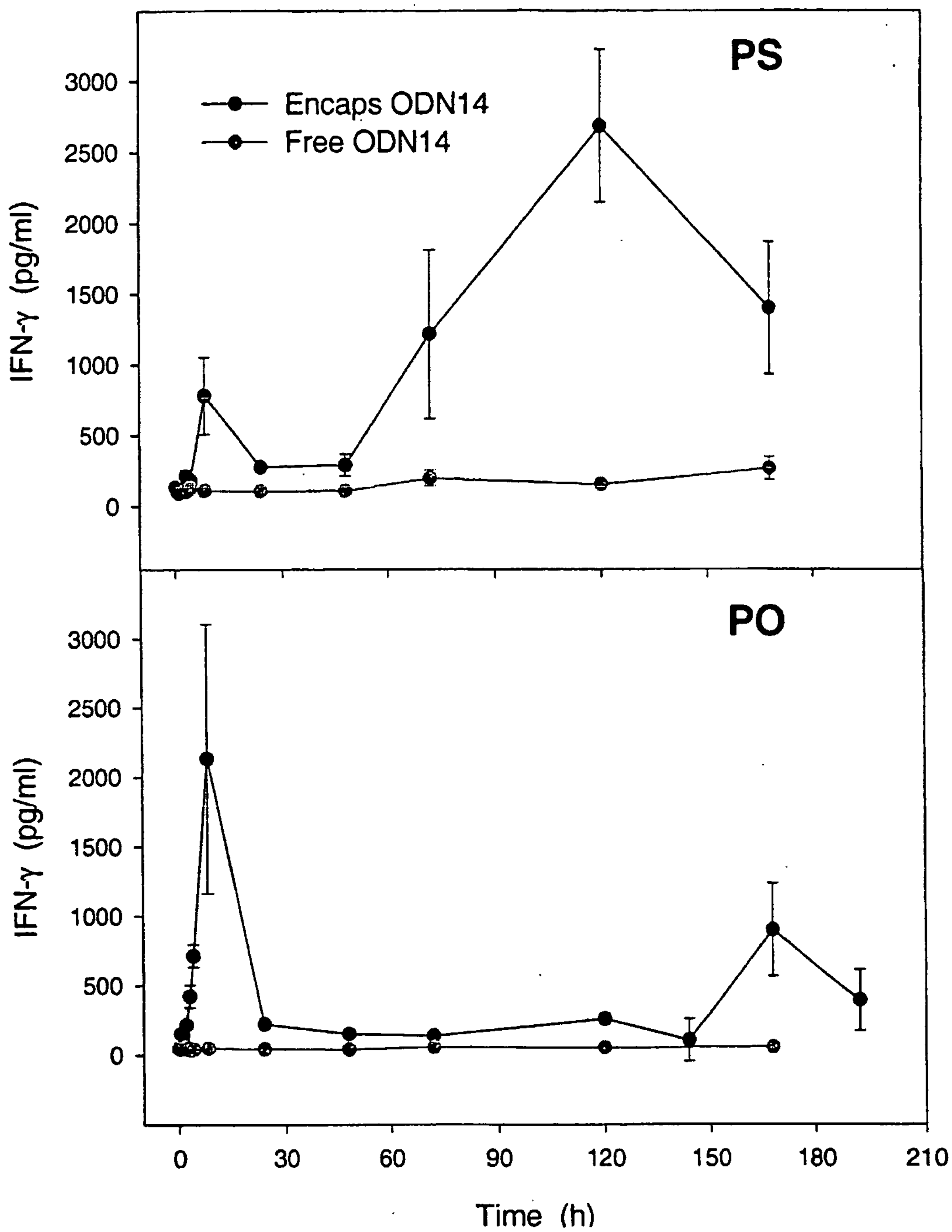
PS
Encaps ODN14
Free ODN14
3000
2500
2000
1500
1000
500
0
IFN-γ (pg/ml)
PO
3000
2500
2000
1500
1000
500
0
IFN-γ (pg/ml)
0
30
60
90
120
150
180
210
Time (h)

Figure 7B

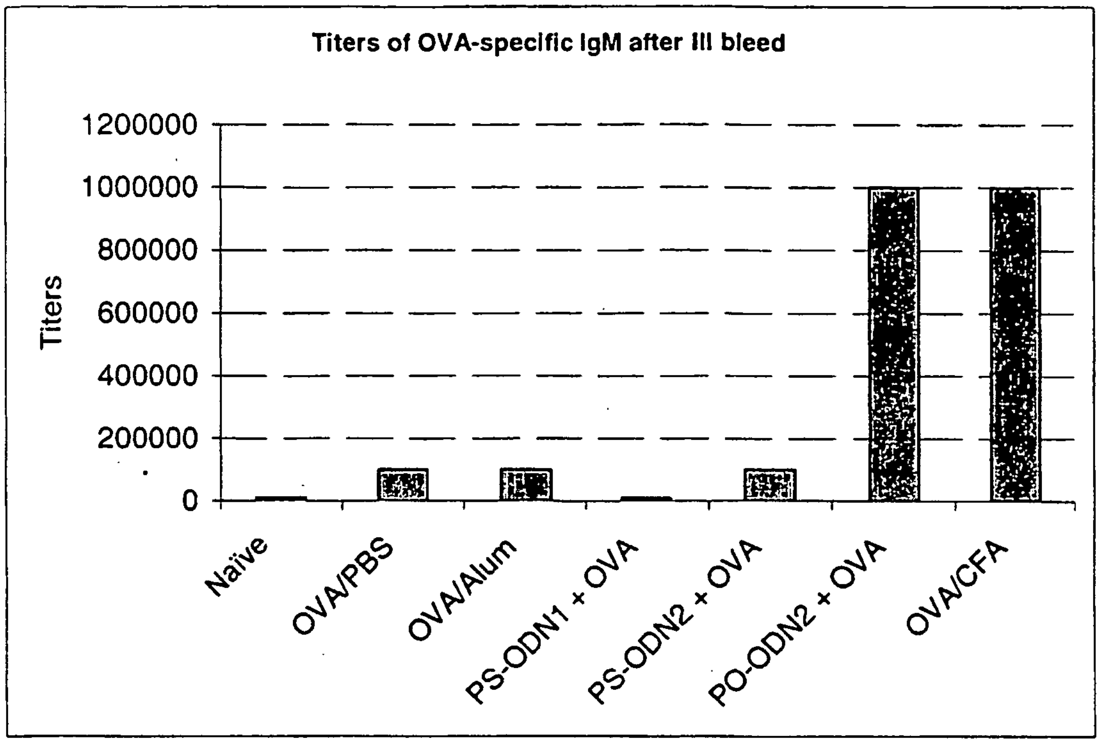
Titers of OVA-specific IgM after III bleed
Titers
1200000
1000000
800000
600000
400000
200000
0
Naïve
OVA/PBS
OVA/Alum
PS-ODN1 + OVA
PS-ODN2 + OVA
PO-ODN2 + OVA
OVA/CFA

Figure 8

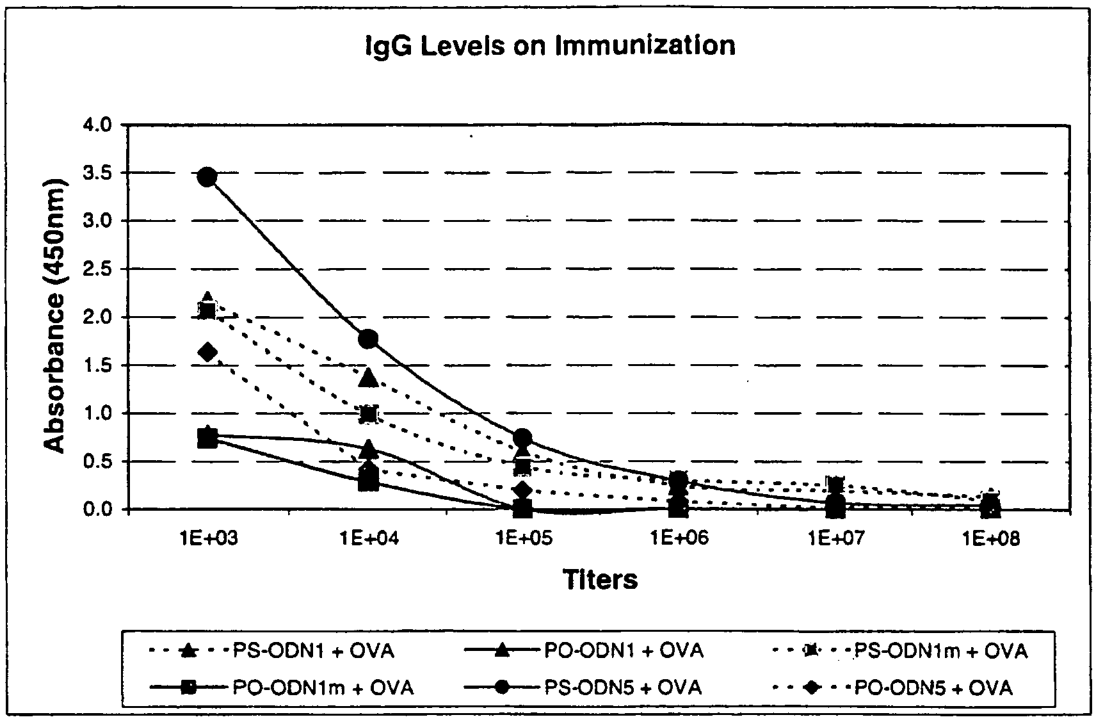
IgG Levels on Immunization
Absorbance (450nm)
4.0
3.5
3.0
2.5
2.0
1.5
1.0
0.5
0.0
1E+03
1E+04
1E+05
1E+06
1E+07
1E+08
Titers
PS-ODN1 + OVA
PO-ODN1 + OVA
PS-ODN1m + OVA
PO-ODN1m + OVA
PS-ODN5 + OVA
PO-ODN5 + OVA

Figure 9

(a)

(b)

(c)

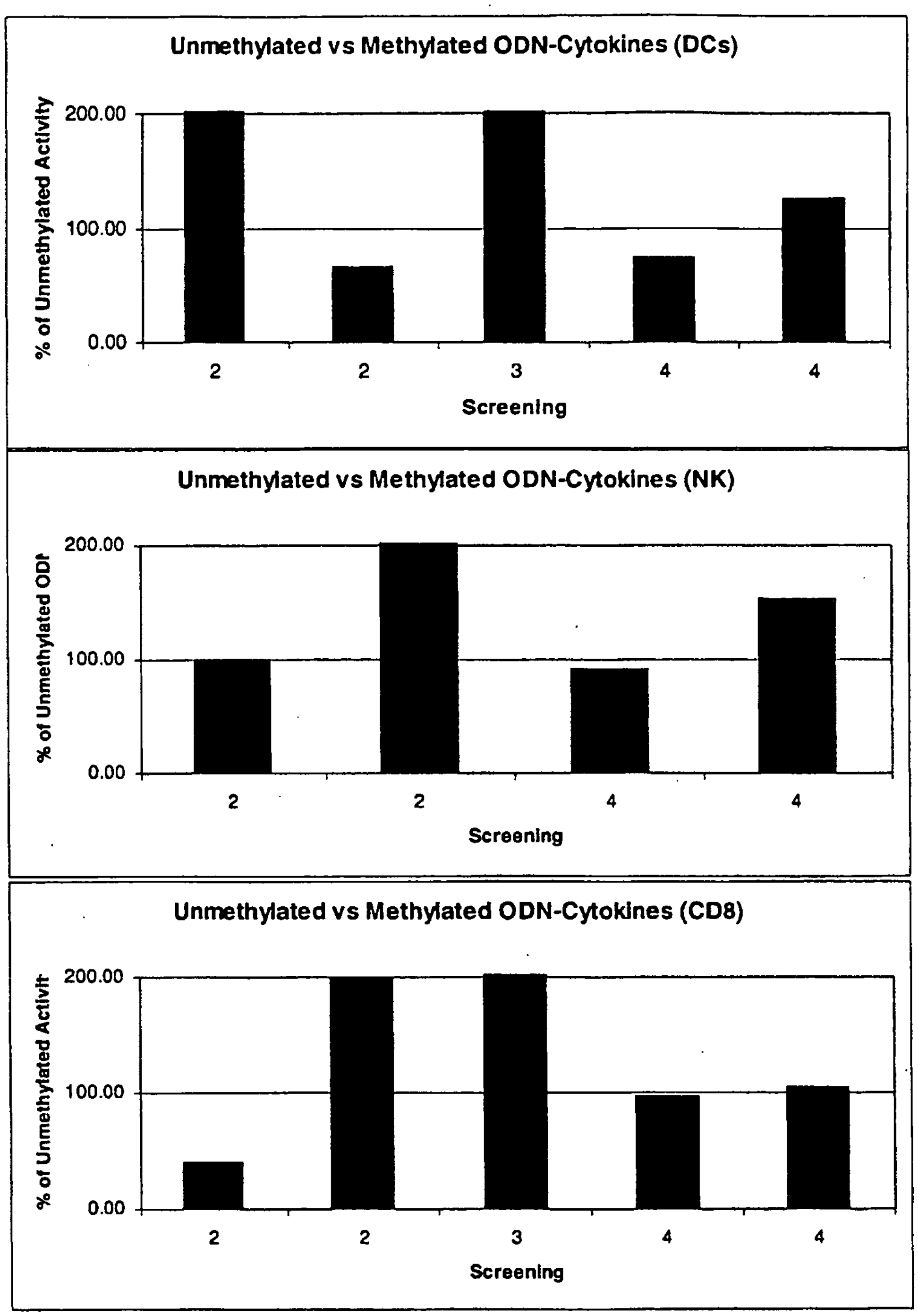


Figure 10

(a)

(b)

(c)

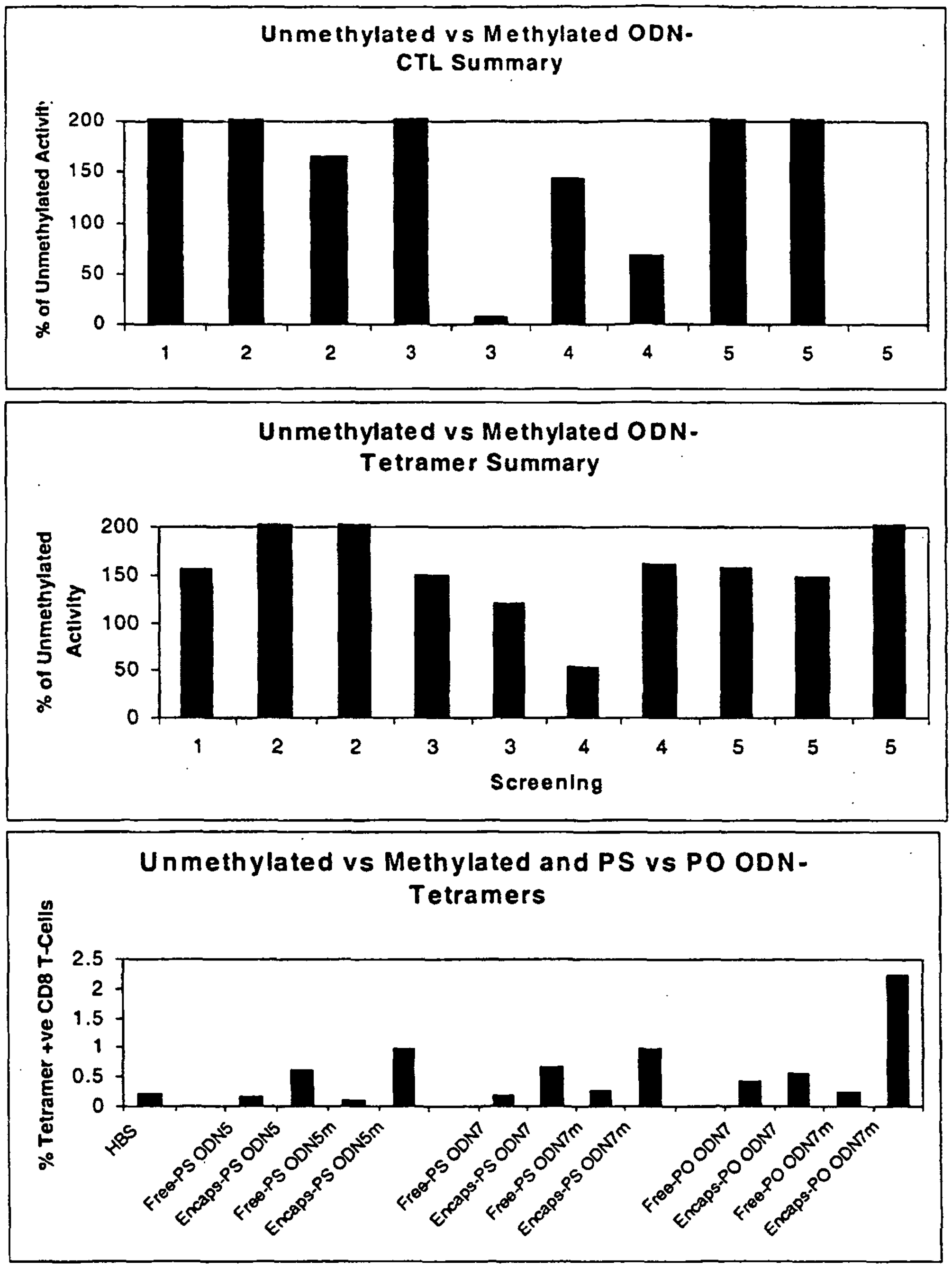


Figure 11

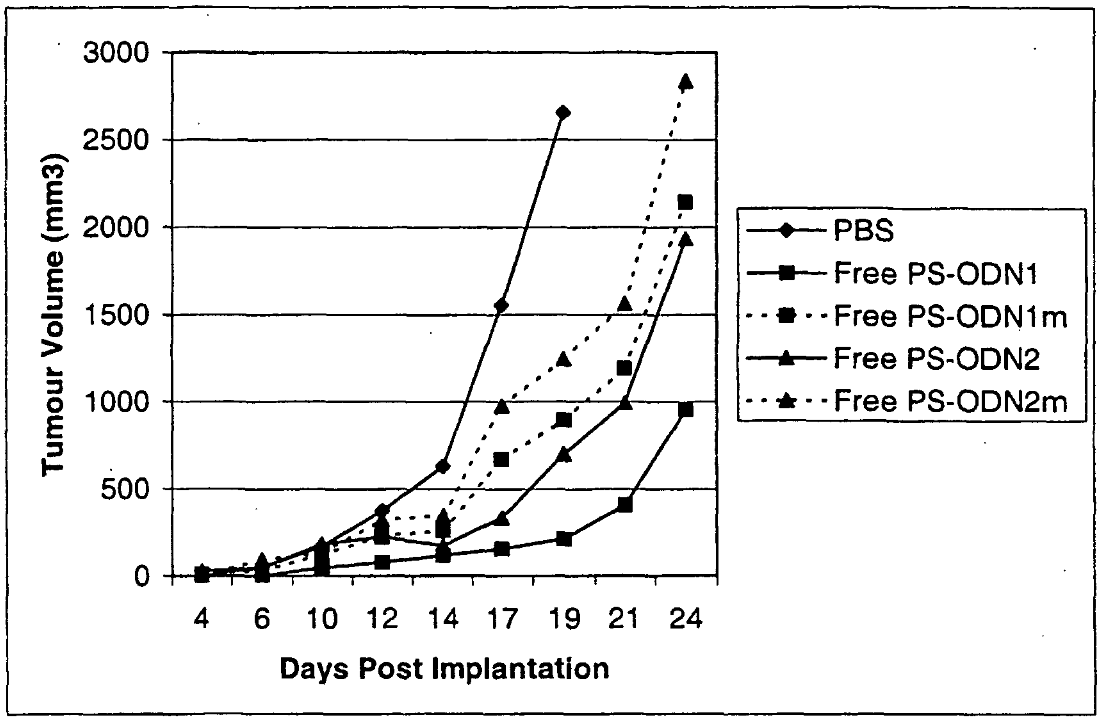
3000
2500
2000
1500
1000
500
0
Tumour Volume (mm3)
4
6
10
12
14
17
19
21
24
Days Post Implantation
PBS
Free PS-ODN1
Free PS-ODN1m
Free PS-ODN2
Free PS-ODN2m

Figure 12

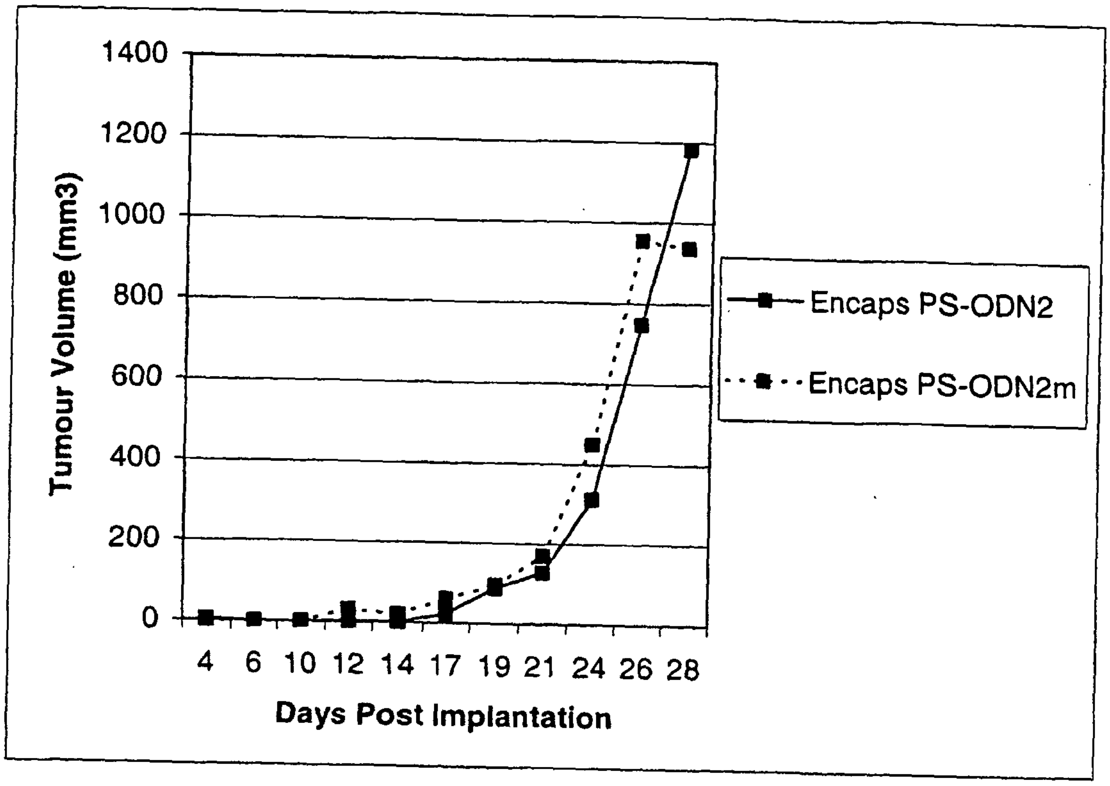
1400
1200
1000
800
600
400
200
0
Tumour Volume (mm3)
4 6 10 12 14 17 19 21 24 26 28
Days Post Implantation
Encaps PS-ODN2
Encaps PS-ODN2m

Figure 13

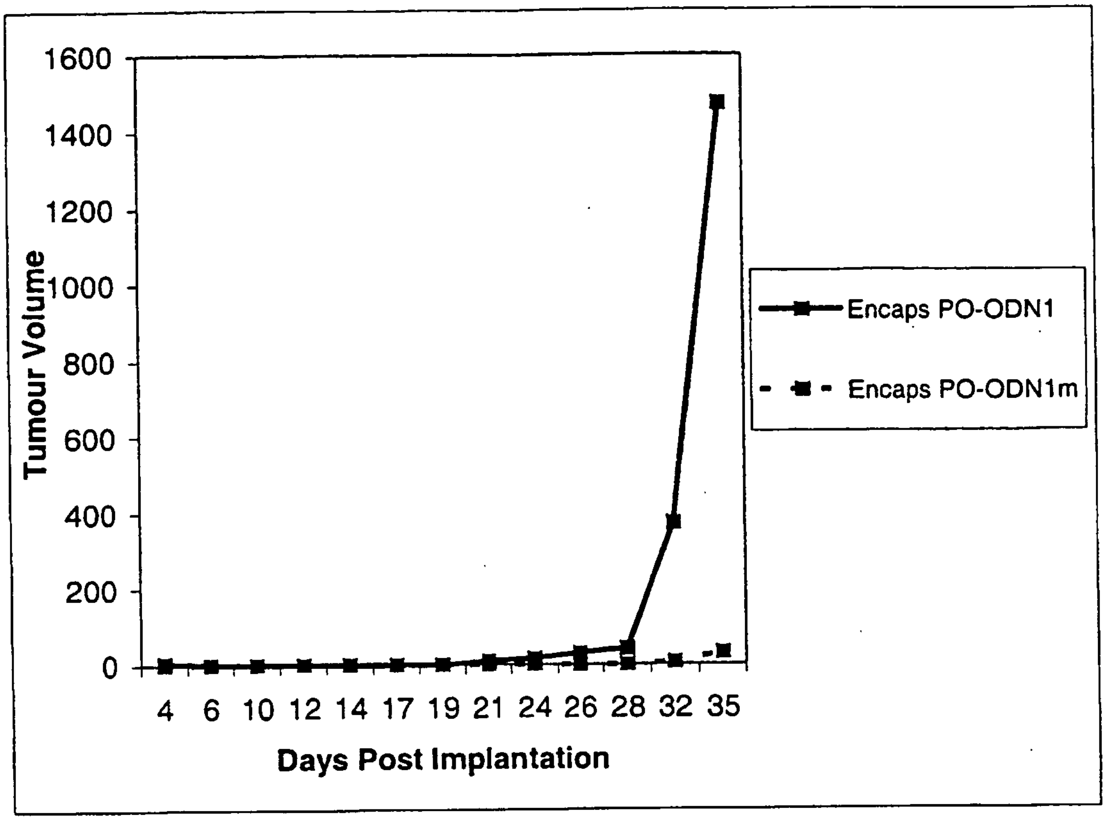
1600
1400
1200
1000
800
600
400
200
0
Tumour Volume
4
6
10
12
14
17
19
21
24
26
28
32
35
Days Post Implantation
Encaps PO-ODN1
Encaps PO-ODN1m

Figure 14

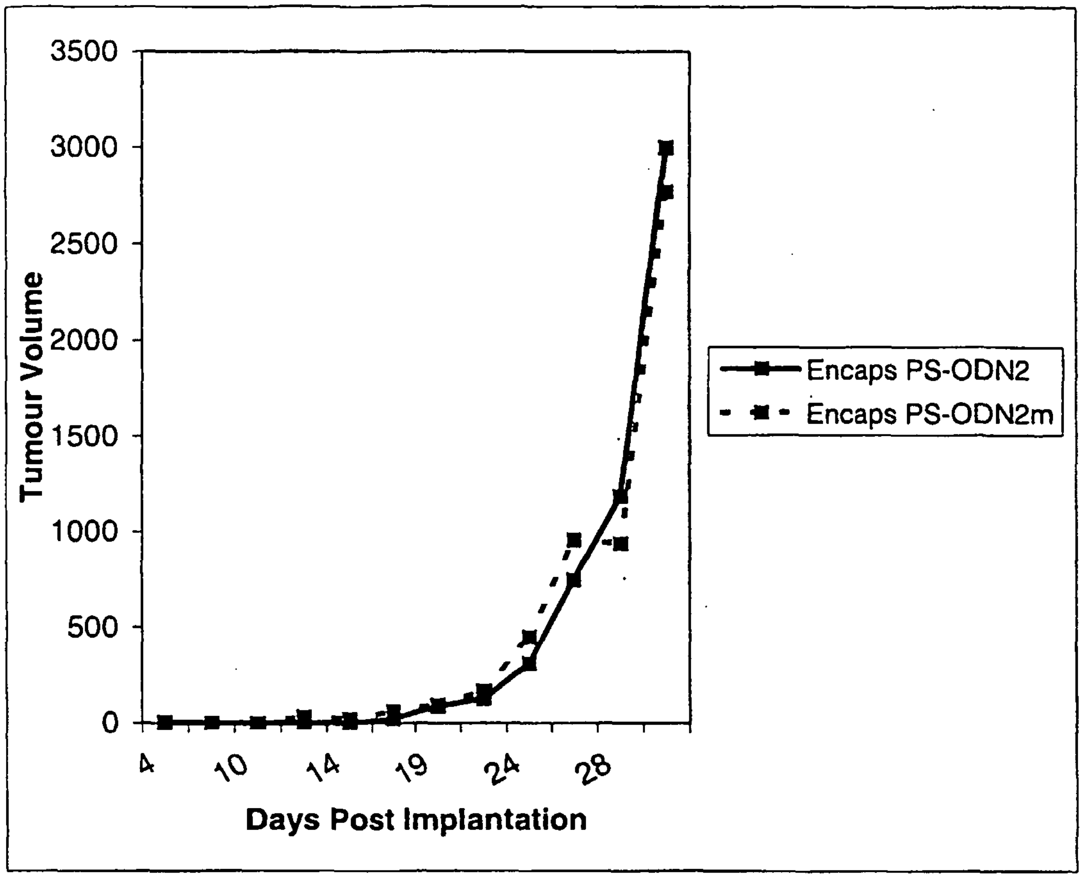
3500
3000
2500
2000
1500
1000
500
0
Tumour Volume
4
10
14
19
24
28
Days Post Implantation
Encaps PS-ODN2
Encaps PS-ODN2m

Figure 15

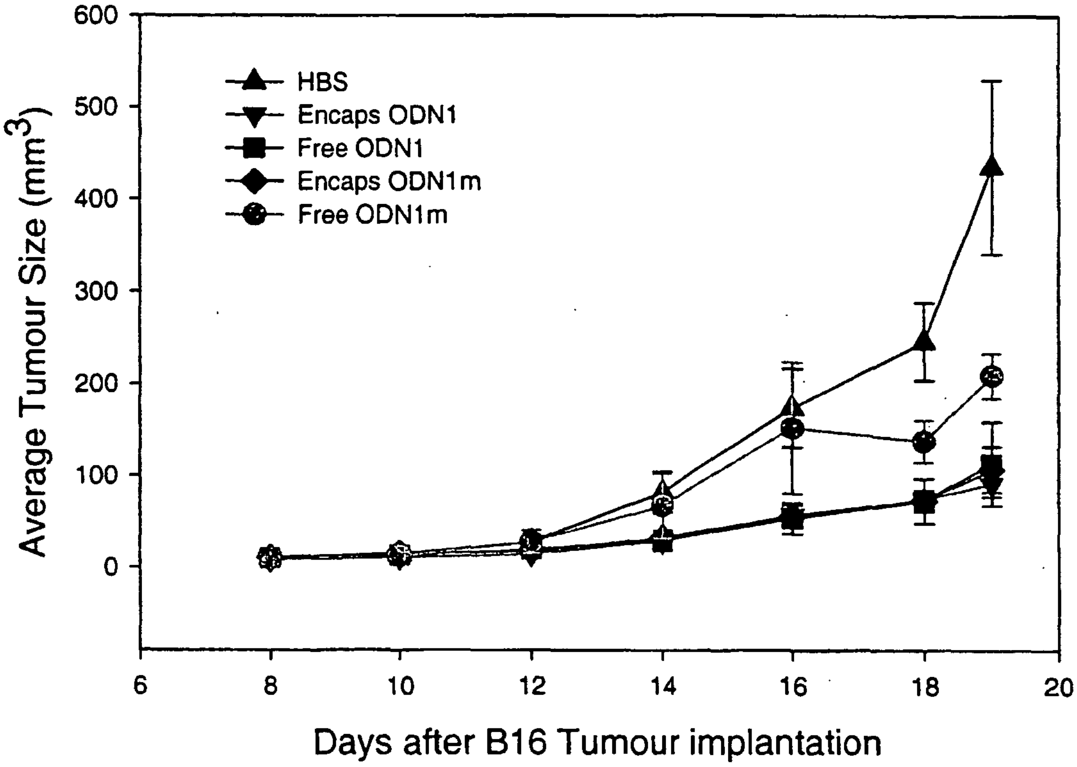
600
500
400
300
200
100
0
Average Tumour Size (mm3)
HBS
Encaps ODN1
Free ODN1
Encaps ODN1m
Free ODN1m
6
8
10
12
14
16
18
20
Days after B16 Tumour implantation

Figure 16

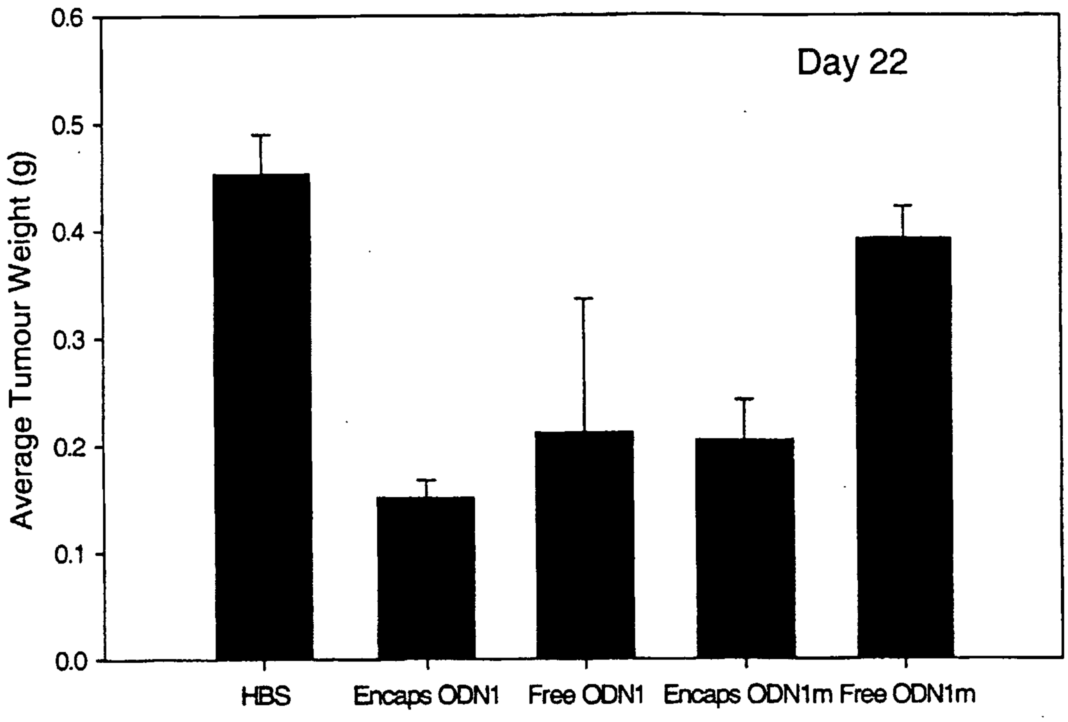
Day 22
Average Tumour Weight (g)
0.6
0.5
0.4
0.3
0.2
0.1
0.0
HBS
Encaps ODN1
Free ODN1
Encaps ODN1m
Free ODN1m

Figure 17

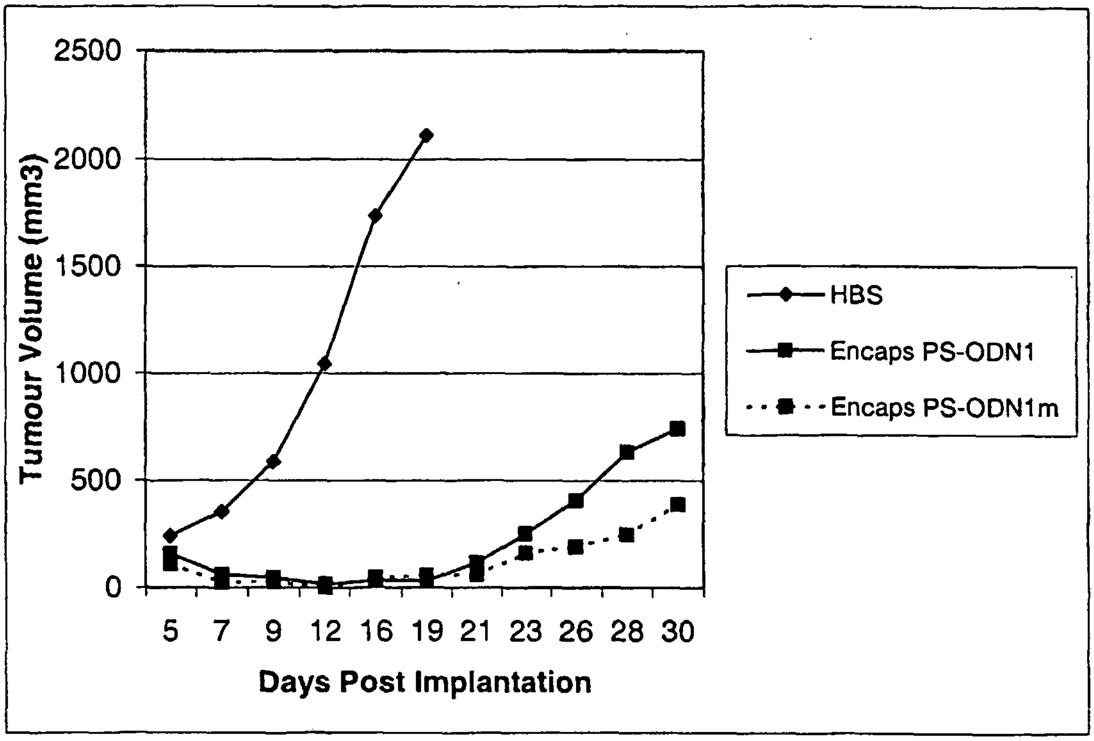
2500
2000
1500
1000
500
0
Tumour Volume (mm3)
5 7 9 12 16 19 21 23 26 28 30
Days Post Implantation
HBS
Encaps PS-ODN1
Encaps PS-ODN1m

Figure 18

(a)
(b)

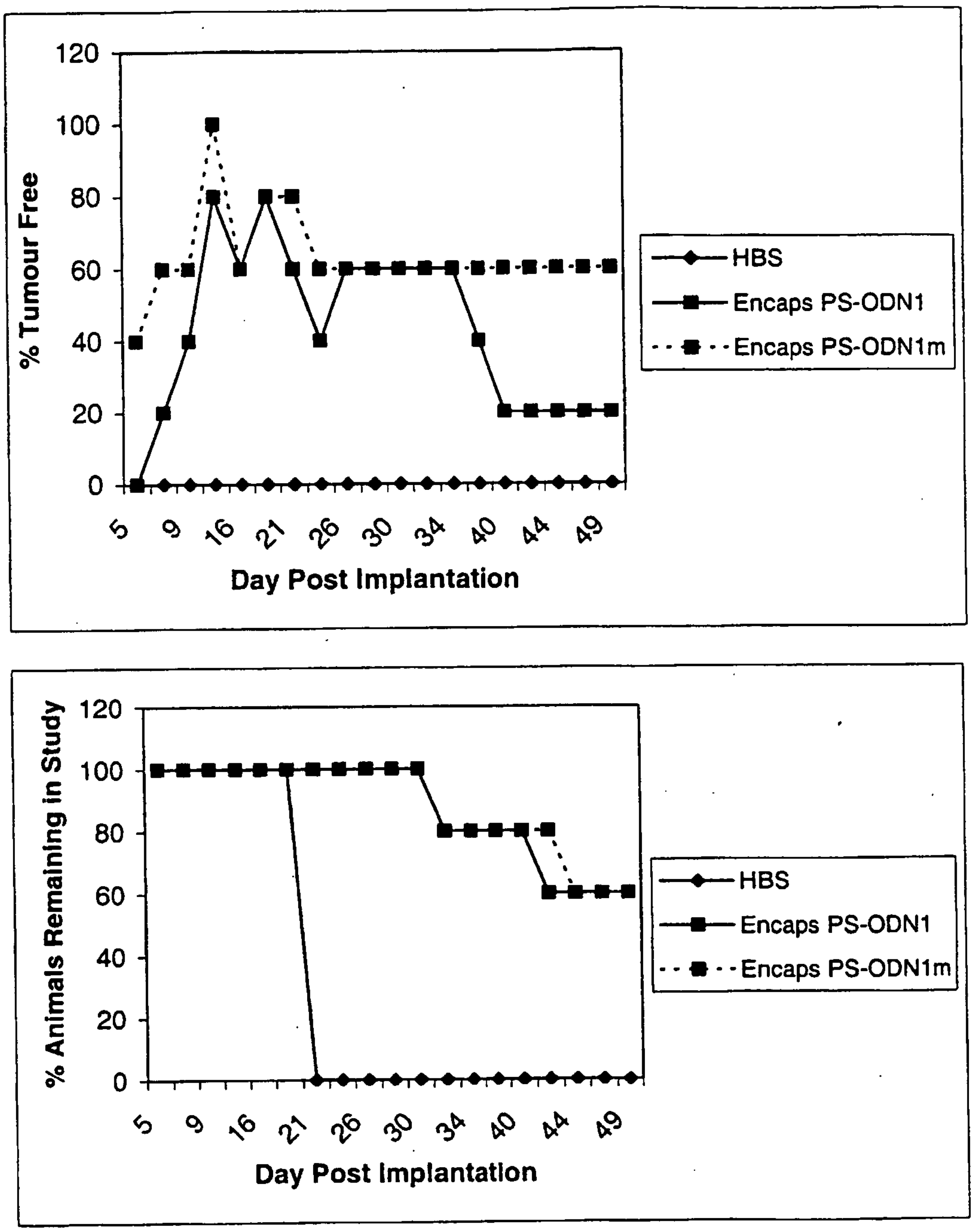

% Tumour Free
120
100
80
60
40
20
0
5
9
16
21
26
30
34
40
44
49
Day Post Implantation
HBS
Encaps PS-ODN1
Encaps PS-ODN1m
% Animals Remaining in Study
120
100
80
60
40
20
0
5
9
16
21
26
30
34
40
44
49
Day Post Implantation
HBS
Encaps PS-ODN1
Encaps PS-ODN1m


Figure 19

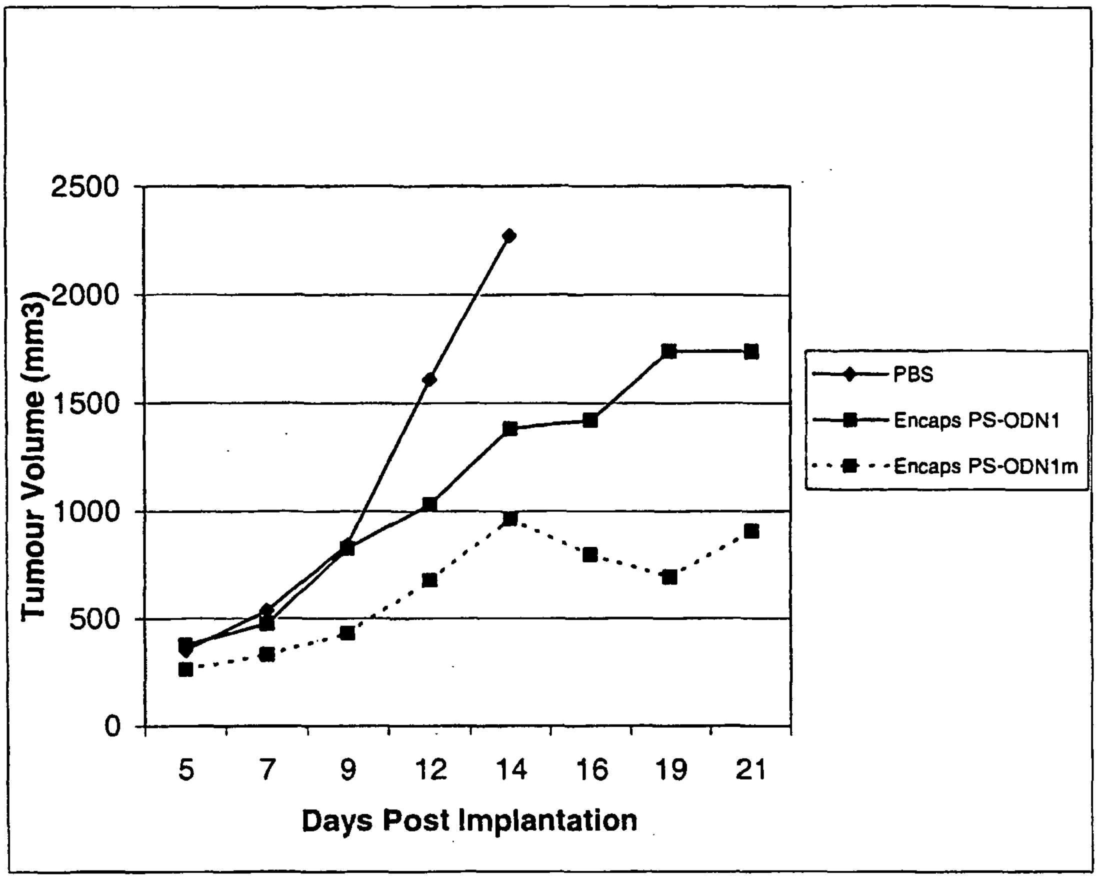
2500
2000
1500
1000
500
0
Tumour Volume (mm3)
5 7 9 12 14 16 19 21
Days Post Implantation
PBS
Encaps PS-ODN1
Encaps PS-ODN1m

Figure 20

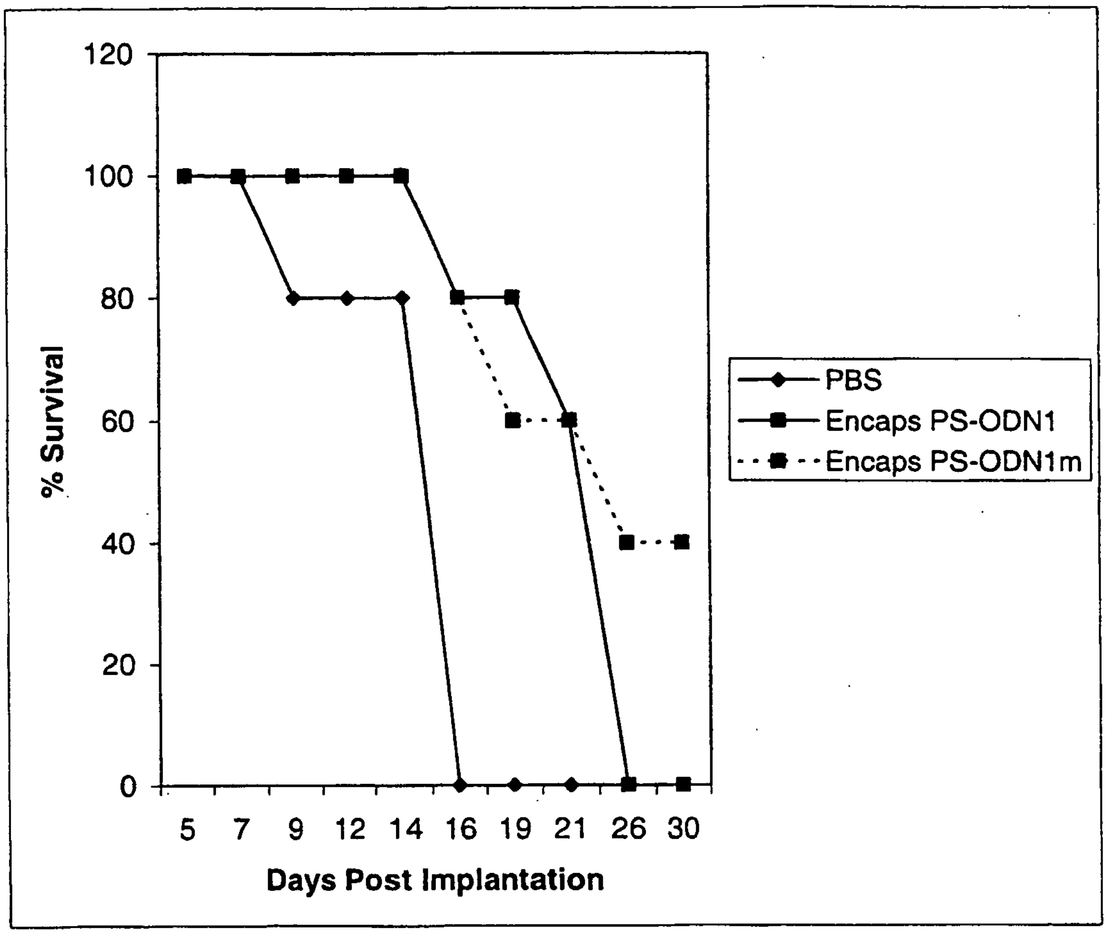
120
100
80
60
40
20
0
% Survival
5
7
9
12
14
16
19
21
26
30
Days Post Implantation
PBS
Encaps PS-ODN1
Encaps PS-ODN1m

Figure 21

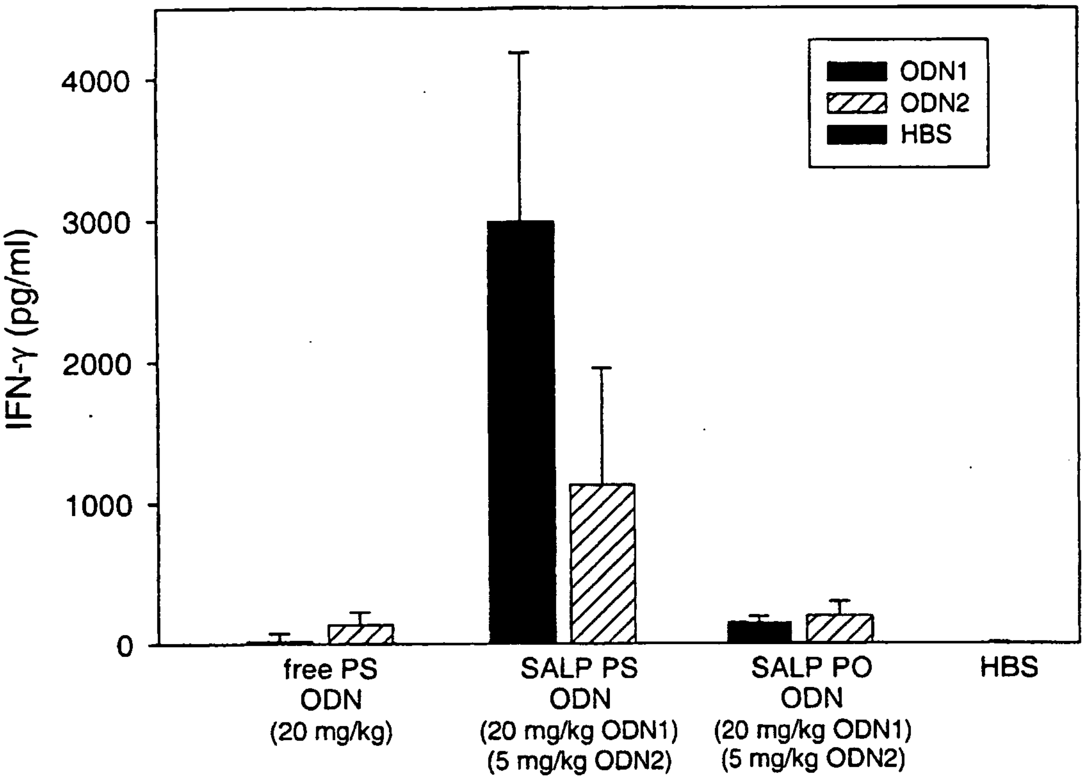
ODN1
ODN2
HBS
IFN-γ (pg/ml)
4000
3000
2000
1000
0
free PS ODN (20 mg/kg)
SALP PS ODN (20 mg/kg ODN1) (5 mg/kg ODN2)
SALP PO ODN (20 mg/kg ODN1) (5 mg/kg ODN2)
HBS

Figure 22

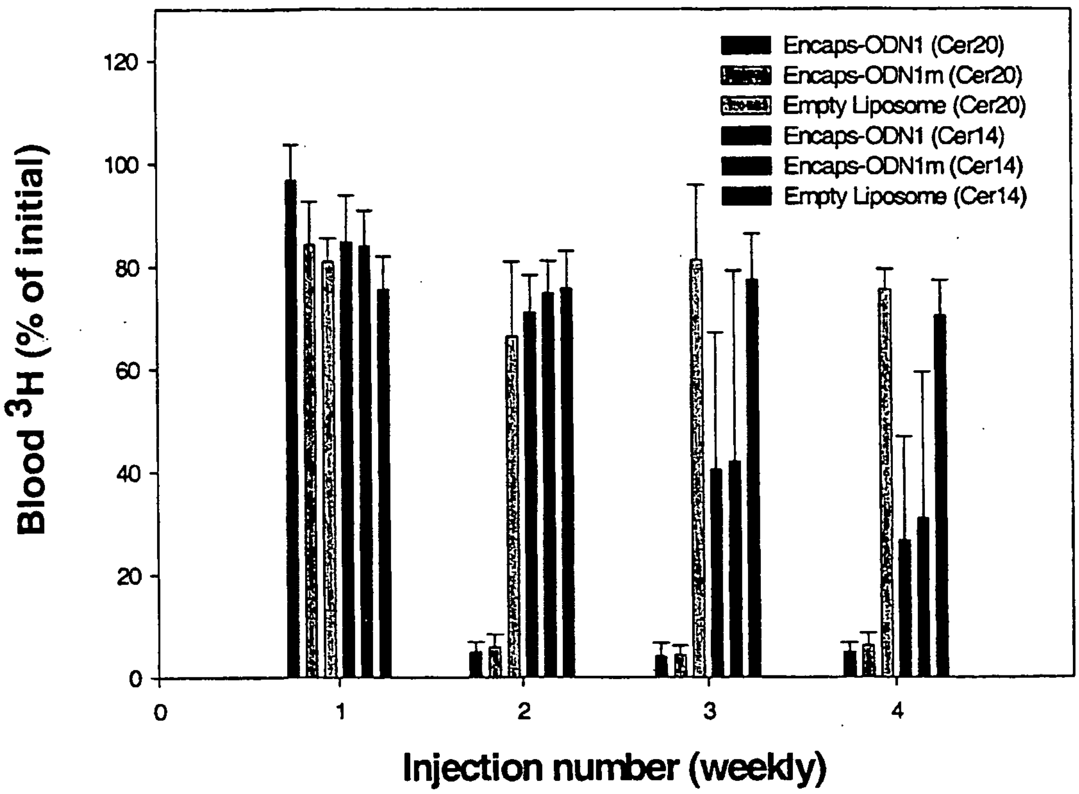
Encaps-ODN1 (Cer20)
Encaps-ODN1m (Cer20)
Empty Liposome (Cer20)
Encaps-ODN1 (Cer14)
Encaps-ODN1m (Cer14)
Empty Liposome (Cer14)
Blood $^3$H (% of initial)
Injection number (weekly)
0
20
40
60
80
100
120
1
2
3
4

Figure 23

(a)

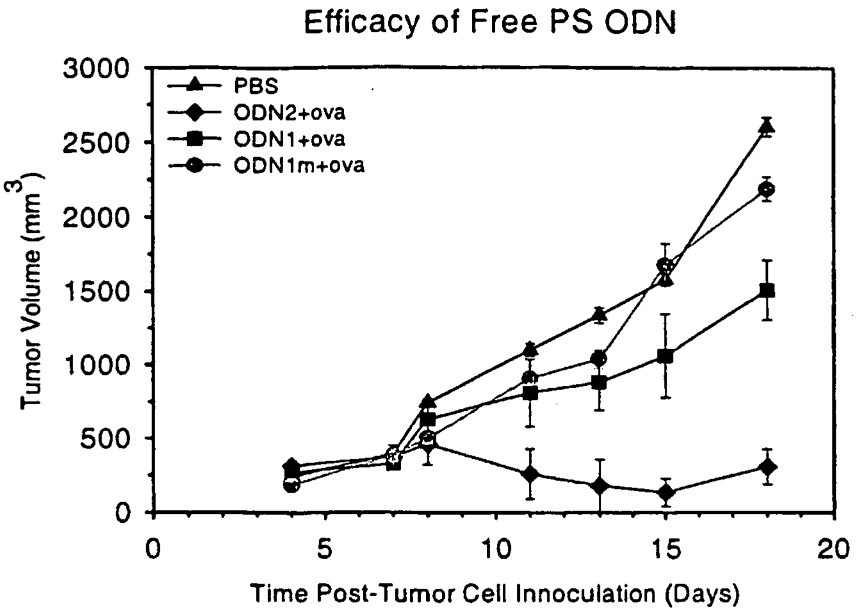
Efficacy of Free PS ODN
PBS
ODN2+ova
ODN1+ova
ODN1m+ova
Tumor Volume (mm3)
3000
2500
2000
1500
1000
500
0
0
5
10
15
20
Time Post-Tumor Cell Innoculation (Days)

(b)

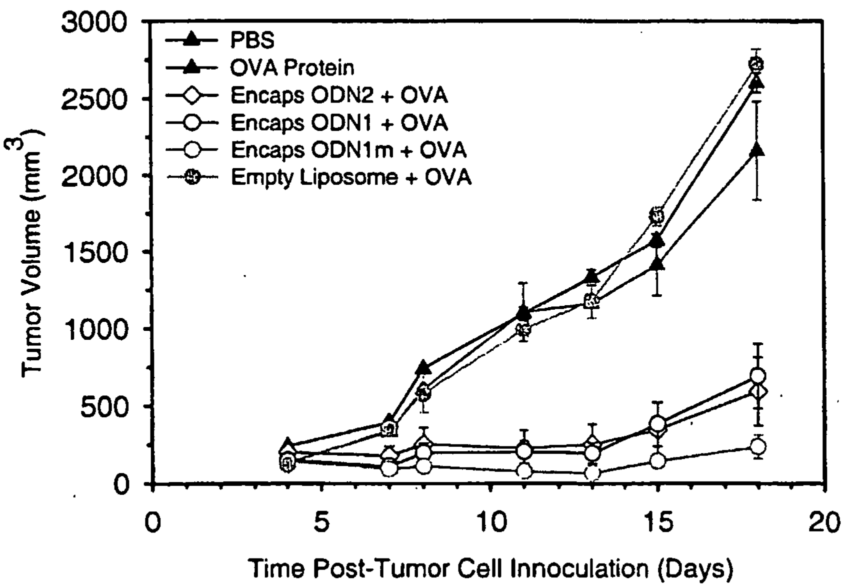
PBS
OVA Protein
Encaps ODN2 + OVA
Encaps ODN1 + OVA
Encaps ODN1m + OVA
Empty Liposome + OVA
Tumor Volume (mm3)
3000
2500
2000
1500
1000
500
0
0
5
10
15
20
Time Post-Tumor Cell Innoculation (Days)

Figure 24

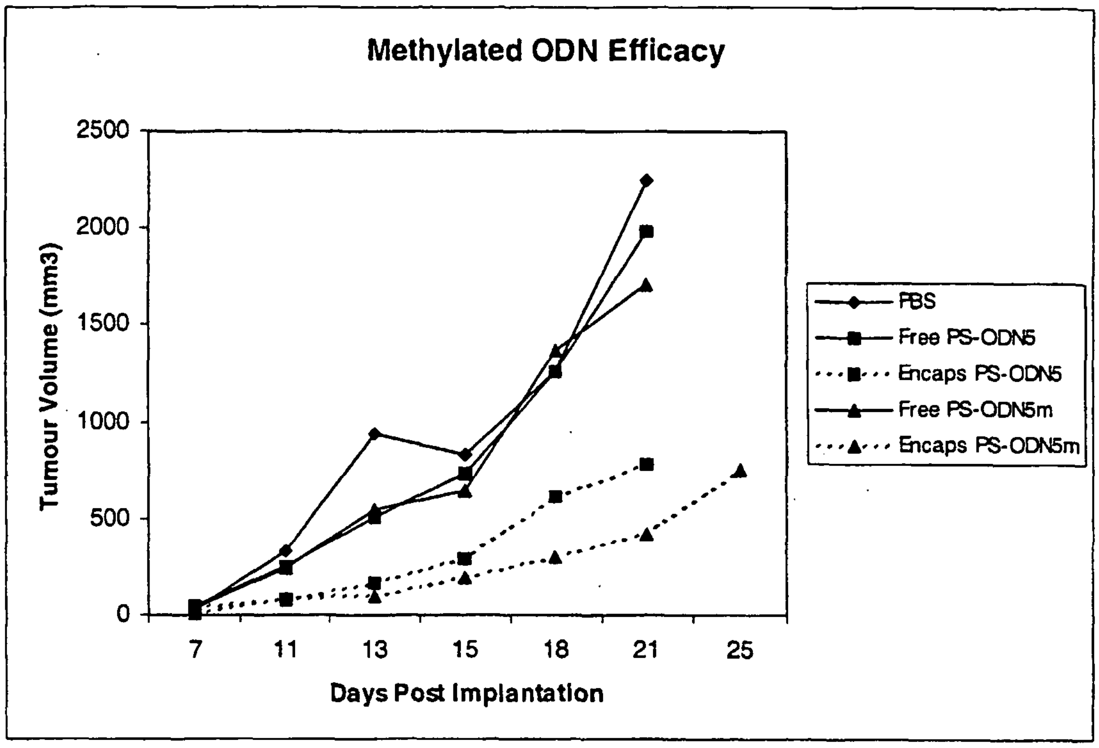
Methylated ODN Efficacy
Tumour Volume (mm3)
2500
2000
1500
1000
500
0
7
11
13
15
18
21
25
Days Post Implantation
PBS
Free PS-ODN5
Encaps PS-ODN5
Free PS-ODN5m
Encaps PS-ODN5m

Figure 25

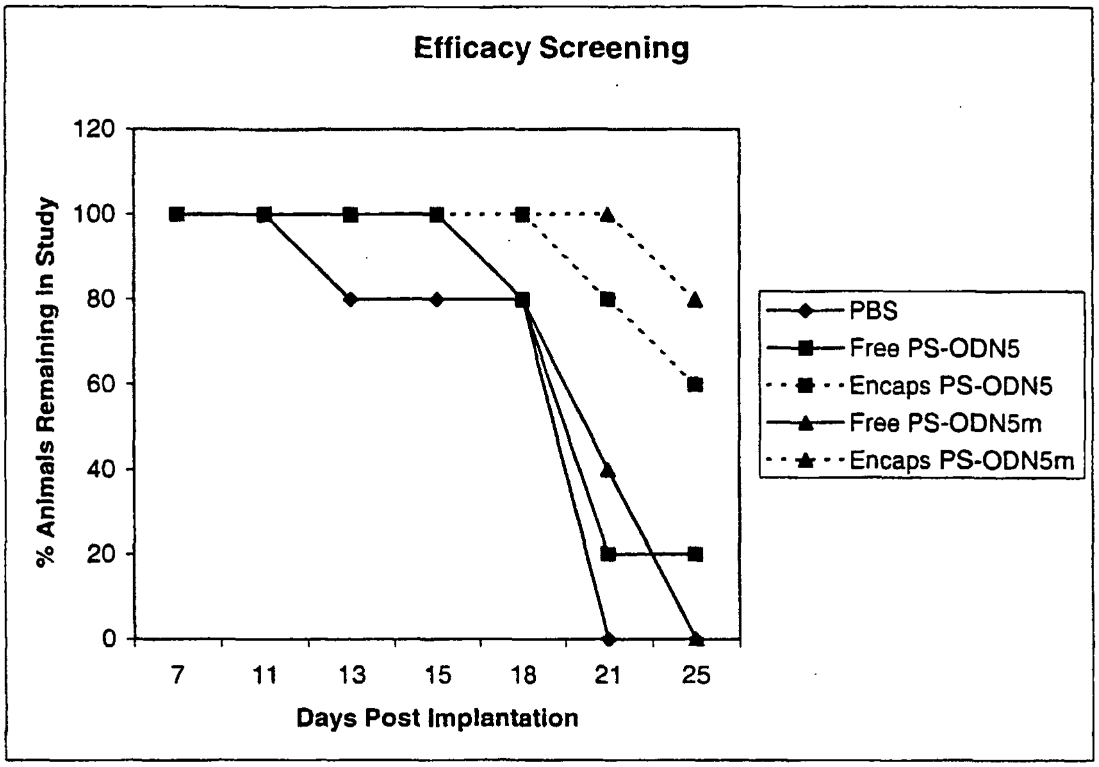
Efficacy Screening
120
100
80
60
40
20
0
% Animals Remaining in Study
7
11
13
15
18
21
25
Days Post Implantation
PBS
Free PS-ODN5
Encaps PS-ODN5
Free PS-ODN5m
Encaps PS-ODN5m

Figure 26

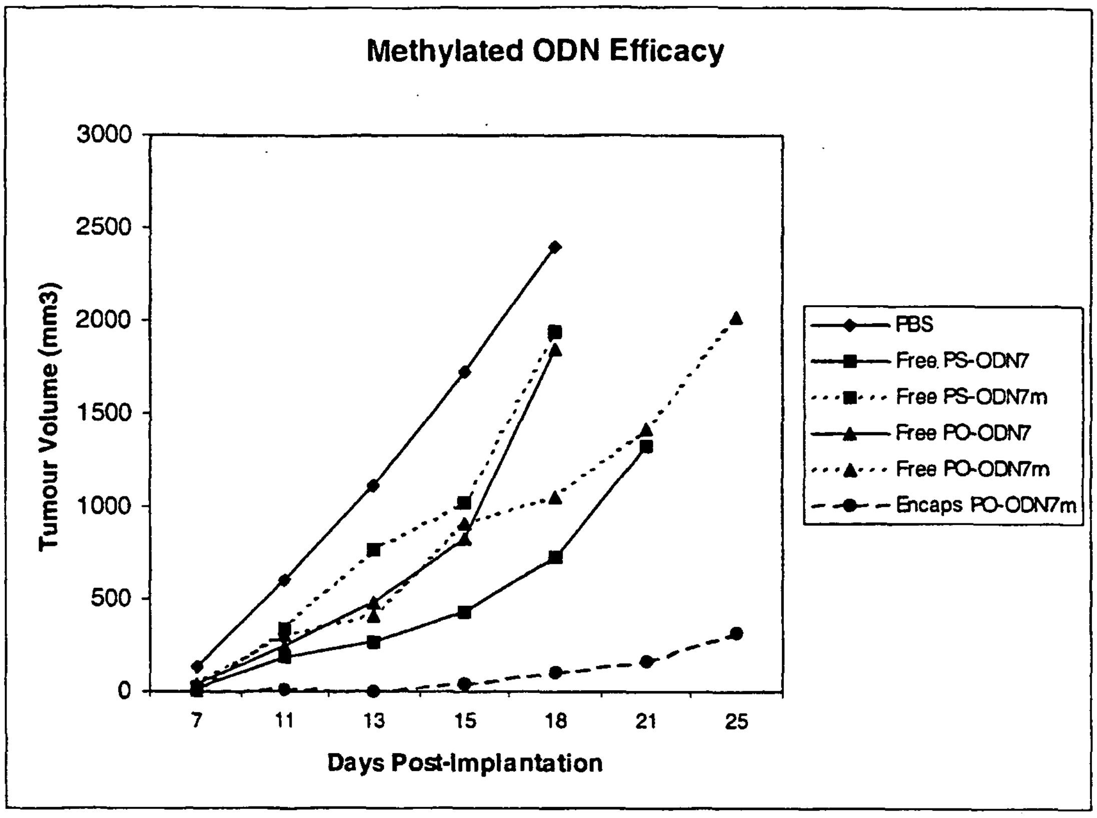
Methylated ODN Efficacy
3000
2500
2000
1500
1000
500
0
Tumour Volume (mm3)
7
11
13
15
18
21
25
Days Post-Implantation
PBS
Free PS-ODN7
Free PS-ODN7m
Free PO-ODN7
Free PO-ODN7m
Encaps PO-ODN7m

Figure 27

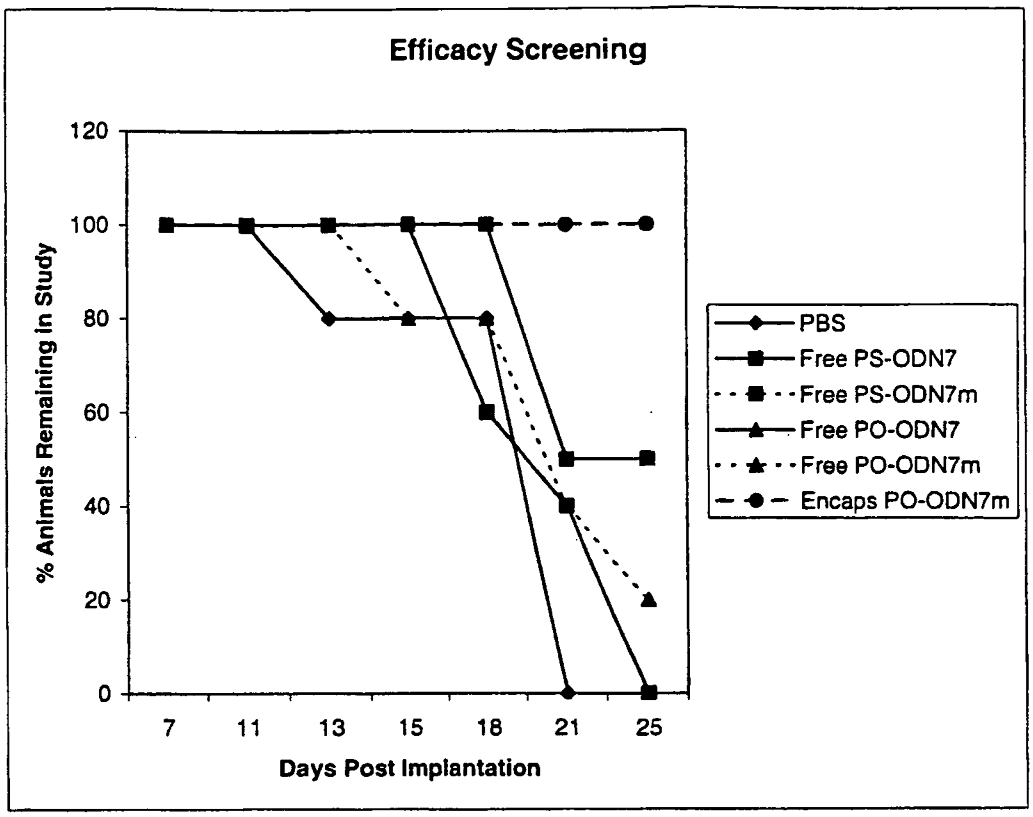
Efficacy Screening
120
100
80
60
40
20
0
% Animals Remaining in Study
7
11
13
15
18
21
25
Days Post Implantation
PBS
Free PS-ODN7
Free PS-ODN7m
Free PO-ODN7
Free PO-ODN7m
Encaps PO-ODN7m

Figure 28

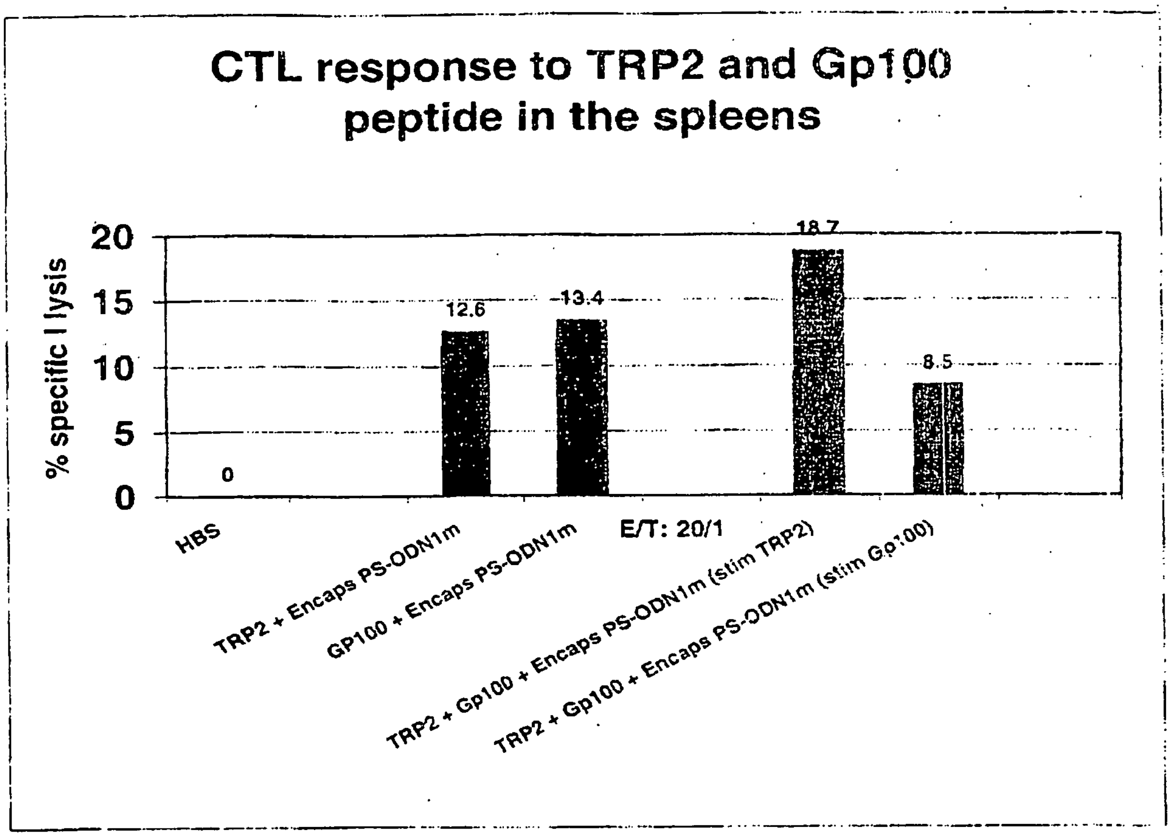
CTL response to TRP2 and Gp100 peptide in the spleens
% specific l lysis
20
15
10
5
0
0
12.6
13.4
18.7
8.5
HBS
TRP2 + Encaps PS-ODN1m
GP100 + Encaps PS-ODN1m
E/T: 20/1
TRP2 + Gp100 + Encaps PS-ODN1m (stim TRP2)
TRP2 + Gp100 + Encaps PS-ODN1m (stim Gp100)

Figure 29

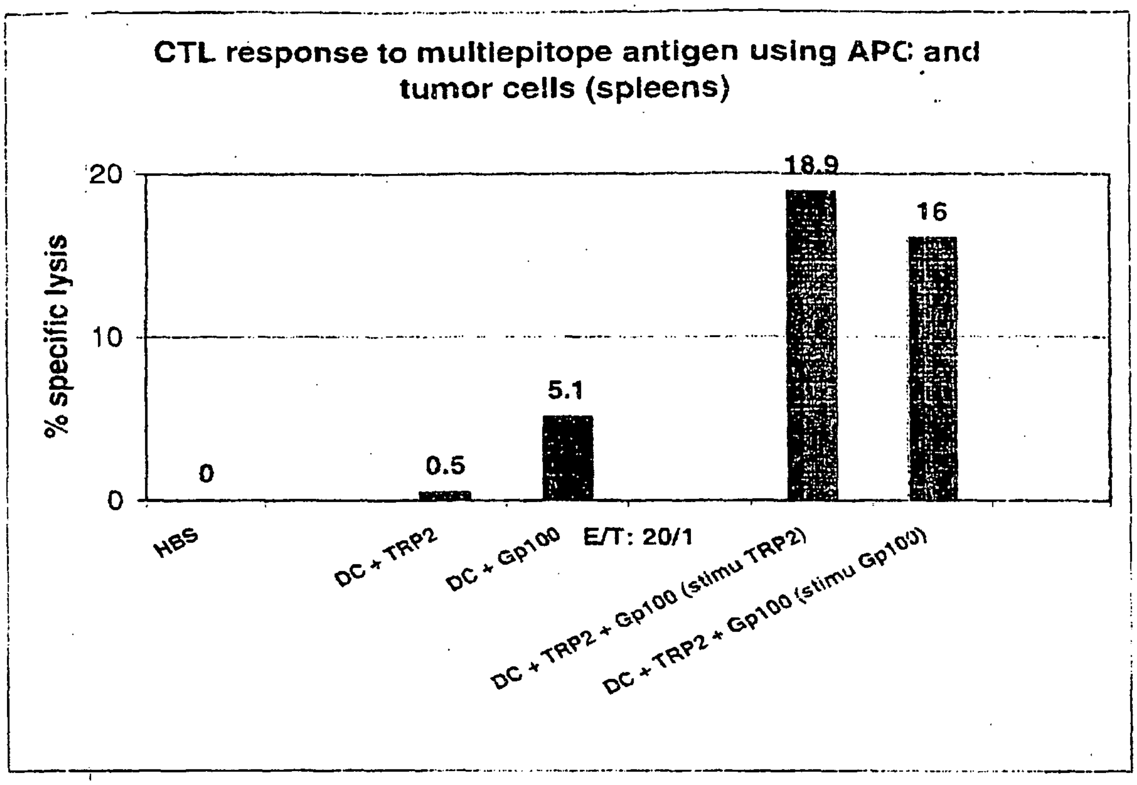
CTL response to multiepitope antigen using APC and tumor cells (spleens)
% specific lysis
20
10
0
18.9
16
5.1
0.5
0
HBS
DC + TRP2
DC + Gp100
E/T: 20/1
DC + TRP2 + Gp100 (stimu TRP2)
DC + TRP2 + Gp100 (stimu Gp100)

Figure 30

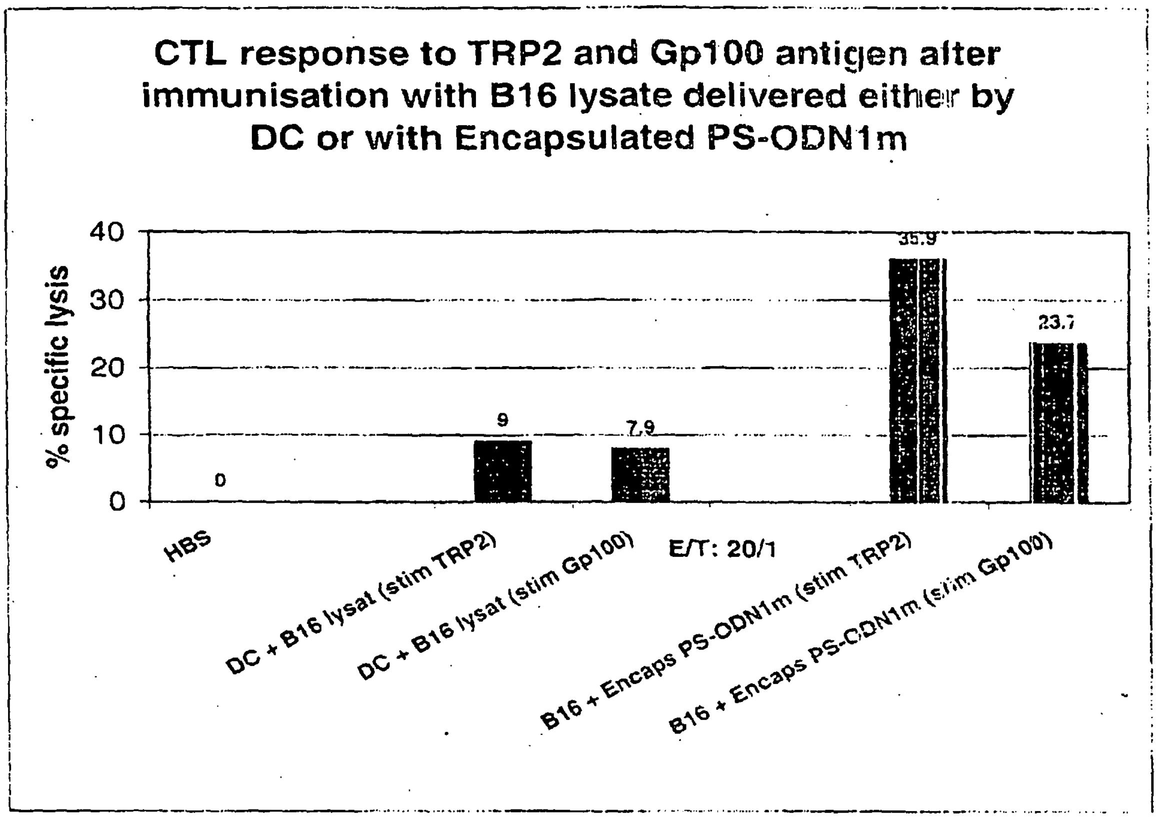
CTL response to TRP2 and Gp100 antigen after immunisation with B16 lysate delivered either by DC or with Encapsulated PS-ODN1m
% specific lysis
40
30
20
10
0
0
9
7.9
35.9
23.7
HBS
DC + B16 lysat (stim TRP2)
DC + B16 lysat (stim Gp100)
E/T: 20/1
B16 + Encaps PS-ODN1m (stim TRP2)
B16 + Encaps PS-ODN1m (stim Gp100)

Figure 31

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0115726 A
- WO 9602555 A
- US 6194388 B
- US 6207646 B
- US 6239116 B
- US 6406705 B
- US 6429199 B
- WO 02069369 A
- US 4469863 A
- US 5023243 A
- EP 092574 A
- WO 9611266 A
- US 379343 P
- US 64952 A
- US 6465439 B
- US 6379698 B
- US 6365611 B
- US 6093816 A
- US 5785992 A
- US 6287591 B
- US 6287591 B1
- US 649527 A
- US 4320121 A
- US 5820873 A
- US 5885613 A
- WO 9851278 A
- US 218988 A
- US 094540 A
- US 6320017 B
- US 6417326 B
- US 674191 A
- GB 2147243 A, Takahashi
- US 4235871 A
- US 4501728 A
- US 4837028 A
- US 4737323 A
- US 5705385 A
- US 5976567 A
- US 140476 A
- US 5981501 A
- WO 9640964 A
- US 5075109 A
- US 4452775 A
- US 4675189 A
- US 5736152 A
- US 3854480 A
- US 5133974 A
- US 5407686 A
- CA 0300678 W
- CA 60379343
- CA 60460646
- CA 10290545
- CA 10437275


### Non-patent literature cited in the description


- **COSTELLO ; PLASS.** *J. Med. Genet.,* 2001, vol. 38, 285
- **MESSINA et al.** *J. Immunol,* 1991, vol. 147, 1759
- **KRIEG et al.** *Nature,* 1995, vol. 374, 546-9
- **PARRONCHI P. et al.** *J. Immunol,* 1999, vol. 163, 5946-53
- **KREIG A.M.** *Biochim Biophys Acta,* 1999, vol. 1489, 107-16
- **SAMBROOK, J. et al.** Molecular Cloning,: A Laboratory Manual. Cold Spring Harbor Laboratory Press, 1989
- Directed Mutagenesis: A Practical Approach. IRL Press, 1991
- **JONES, J.** Amino Acid and Peptide Synthesis. Oxford Science Publications, 1992
- **AUSTEN, B. M. ; WESTWOOD, O.M.R.** Protein Targeting and Secretion. IRL Press, 1991
- **HENRY et al.** *J. Pharmacol. Exp. Ther.,* 2000, vol. 292, 468
- **ZHAO et al.** *Bioorg. Med. Chem Lett.,* 1999, vol. 9, 3453
- **ZHAO et al.** *Biorg Med. Chem Lett.,* 2000, vol. 10, 1051
- **J. F. COLLINS et al.** *Comput. Appl. Biosci.,* 1988, vol. 4, 67-72
- Molecular Sequence Comparison and Alignment. **J. F. COLLINS et al.** Practical Approach Series: Nucleic Acid and Protein Sequence Analysis XVIII. IRL Press, 1987, 417
- **E.G. SHPAER et al.** *Genomics,* 1996, vol. 38, 179-191
- **UHLMANN ; PEYMAN.** *Chem. Rev.,* 1990, vol. 90, 544
- **GOODCHILD.** *Bioconjugate Chem.,* 1990, vol. 1, 165

- **YAMAMOTO S. et al.** *J. Immunol.,* 1992, vol. 148, 4072-4076
- **BEAUCAGE, S. L. ; CARUTHERS, M. H.** *Tet. Let.,* 1981, vol. 22, 1859
- **GAREGG et al.** *Tet. Let.,* 1986, vol. 27, 4051-4054
- **FROEHLER et al.** *Nucl. Acid. Res.,* 1986, vol. 14, 5399-5407
- **GAREGG et al.** *Tet. Let.,* 1986, vol. 27, 4055-4058
- **GAFFNEY et al.** *Tet. Let.,* 1988, vol. 29, 2619-2622
- **SAMBROOK, T. et al.** Molecular Cloning: A Laboratory Manual. Cold Spring Harbor laboratory Press, 1989
- **RANEY et al.** *Journal of Pharmacology and Experimental Therapeutics,* 2001, vol. 298, 1185-1192
- **KLIBANOV et al.** *FEBS Letters,* 1990, vol. 268 (1), 235-237
- **MARCH.** Advanced Organic Chemistry. Wiley-Interscience, 1992
- **PARR et al.** *Biochim. Biophys. Acta,* 1994, vol. 1195, 21-30
- **SZOKA et al.** *Ann. Rev. Biophys. Bioeng.,* 1980, vol. 9, 467
- Liposomes. Marcel Dekker, Inc, 1983
- **HOPE et al.** *Chem. Phys. Lip.,* 1986, vol. 40, 89
- **COHEN et al.** *Cancer Res.,* 1994, vol. 54, 1055
- **C. G. A THOMAS.** *Medical Microbiology,* 1983
- **BENNETT, K.** Compendium of Veterinary Products. North American Compendiums, Inc, 1995
- **CREAVEN ; MIHICH.** *Semin. Oncol.,* 1977, vol. 4, 147
- **WHITE ; OLDEN.** *Cancer Commun.,* 1991, vol. 3, 83
- **NEWTON.** *Cancer Commun.,* 1989, vol. 1, 373
- **OLDEN, K.** *J. Natl. Cancer Inst.,* 1991, vol. 83, 1149
- **DENNIS, J.** *Cancer Res.,* 1990, vol. 50, 1867
- **OLDEN, K.** *Pharm. Ther.,* 1989, vol. 44, 85
- **WHITE ; OLDEN.** *Anticancer Res.,* 1990, vol. 10, 1515
- **MOSMAN et al.** *Annu. Rev. Immunol.,* 1989, vol. 7, 145-173
- **PAUL et al.** *Cell,* 1994, vol. 76, 241-251
- **O'GARRA.** *Immunity,* 1998, vol. 8, 275-283
- **GLIMCHER ; MURPHY.** *Genes Dev.,* 2000, vol. 14, 1693-1711
- **ABBAS et al.** *Nature,* 1996, vol. 383, 787-793
- **LANGER.** *Science,* 1990, vol. 249, 1527-1533